# EUROPÄISCHE PATENTSCHRIFT

(11) **EP 1 088 558 B1**
(45) Veröffentlichungstag und Bekanntmachung des Hinweises auf die Patenterteilung: **27.06.2007**
(21) Anmeldenummer: 00250323.3
(22) Anmeldetag: 28.09.2000
(51) Int. Cl.: A61K 49/00, A61K 49/18, A61K 49/06, A61P 7/00, A61P 9/00

(54) **Galenische Formulierungen die paramagnetische und diamagnetische Verbindungen enthalten**
Galenic formulations comprising paramagnetic and diamagnetic compounds
Formulations galéniques comprenant des composés paramagnétiques et diamagnétiques

(30) Priorität: 29.09.1999 DE 19948651; 08.10.1999 US 158307 P
(43) Veröffentlichungstag der Anmeldung: 04.04.2001
(73) Patentinhaber: Bayer Schering Pharma Aktiengesellschaft, 13353 Berlin (DE)
(72) Erfinder: Platzek, Johannes, Dr., 12621 Berlin (DE); Niedballa, Ulrich, Dr., 14195 Berlin (DE); Radüchel, Bernd, Dr., 13465 Berlin (DE); Mareski, Peter, Dr., 13359 Berlin (DE); Misselwitz, Bernd, Dr., 16548 Glienicke (DE); Frenzel, Thomas, Dr., 12247 Berlin (DE); Weinmann, Hanns-Joachim, Dr., 14129 Berlin (DE)

(56) Entgegenhaltungen:
- EP-A- 0 707 857
- EP-A- 1 088 559
- WO-A-02/14309
- WO-A-95/33494
- DE-A- 10 040 380
- US-A- 5 804 163

## Beschreibung

Die Erfindung liegt auf dem Gebiet der galenischen Formulierungen, welche insbesondere als Kontrastmittel zur Darstellung der Lymphknoten eingesetzt werden. Die Erfindung betrifft den in den Patentansprüchen gekennzeichneten Gegenstand, nämlich neue Formulierungen, welche paramagnetische und diamagnetische perfluoralkylhaltige Substanzen enthalten.

Maligne Tumoren metastasieren gehäuft in regionale Lymphknoten, wobei auch mehrere Lymphknotenstationen beteiligt sein können. So werden Lymphknotenmetastasen in etwa 50 - 69% aller Patienten mit malignen Tumoren gefunden (Elke, Lymphographie, in: Frommhold, Stender, Thurn (eds.), Radiologische Diagnostik in Klinik und Praxis, Band IV, Thieme Verlag Stuttgart, 7th ed., 434-496, 1984). Die Diagnose eines metastatischen Befalls von Lymphknoten ist im Hinblick auf die Therapie und Prognose maligner Erkrankungen von großer Bedeutung. Mit den modernen bildgebenden Methoden (CT, US und MRT) werden lymphogene Absiedlungen von malignen Tumoren nur unzureichend erkannt, da zumeist nur die Größe sowie die Form des Lymphknotens als Diagnosekriterium herangezogen werden kann. Damit sind kleine Metastasen in nicht-vergrößerten Lymphknoten (< 2 cm) nicht von Lymphknotenhyperplasien ohne malignen Befall zu unterscheiden (Steinkamp et al., Sonographie und Kernspintomographie: Differentialdiagnostik von reaktiver Lymphknotenvergrößerung und Lymphknotenmetastasen am Hals, Radiol.diagn. 33:158, 1992).

Es wäre wünschenswert, Lymphknoten mit metastatischen Befall und hyperplastische Lymphknoten mit Hilfe spezifischer Kontrastmittel zu unterscheiden. Dabei könnte das Kontrastmittel intravasal oder interstitiell/intrakutan appliziert werden (s.a. Siefert, H.M. et al., Lymphology 13, 150 - 157, 1980). Die Applikation interstitiell/intrakutan hat den Vorteil, daß die Substanz direkt vom streuenden Herd (z.B. Primärtumor) durch die entsprechenden Lymphwege in die potentiell betroffenen, regionalen Lymphknotenstationen transportiert wird. Gleichfalls kann mit einer geringen Dosis eine hohe Konzentration des Kontrastmittels in den Lymphknoten erreicht werden. Solche interstitiell zu verabreichenden Marker werden bisher vor allem in der nuklearmedizinischen Beurteilung verwendet (mittels radioaktiver Partikel wie z.B. ¹⁹⁸Au-Kolloid). Nuklearmedizinische Methoden haben aber nur eine sehr ungenügende räumliche Auflösung, im Unterschied zur Kernspintomographie mit ihrer hohen räumlichen Auflösung, die im Bereich von Bruchteilen von Millimetern liegt. Die direkte Röntgen-Lymphographie (Injektion einer öligen Kontrastmittelsuspension in ein präpariertes Lymphgefäß) ist eine nur noch selten benutzte, invasive Methode, die nur wenige Lymphabflußstationen darstellen kann. Experimentell werden in Tierexperimenten auch fluoreszenzmarkierte Dextrane verwendet, um nach deren interstitiellen Applikation den Lymphabfluß beobachten zu können. Allen gebräuchlichen Markern für die Darstellung von Lymphwegen und Lymphknoten nach interstitieller/intrakutaner Applikation ist also gemein, daß es sich um Substanzen mit partikulärem Charakter ("particulates", z.B. Emulsionen und Nanokristallsuspensionen) oder große Polymere handelt (s.a. WO 90/14846). Die bisher beschriebenen Zubereitungen erwiesen sich jedoch aufgrund ihrer mangelnden lokalen und systemischen Verträglichkeit sowie ihrer geringen Lymphgängigkeit, die eine unzureichende diagnostischen Effizienz bedingt, zumeist für die indirekte Lymphographie als ungeeignet.

Insgesamt besteht daher ein großer Bedarf an einem lymphspezifischen MRT-Kontrastmittel mit geeigneten pharmazeutischen sowie pharmakologischen Eigenschaften. Bei den pharmazeutischen Eigenschaften stehen zunächst die möglichst hohe Kontrastmittelbeladung sowie eine ausreichende Stabilität im Vordergrund. Bei den pharmakologischen Eigenschaften steht neben einer diagnostisch relevanten und möglichst gleichmäßigen Lymphanreicherung über mehrere (bzw. bei intravenöser Applikation über alle) Lymphstationen hinweg vor allem eine rasche und vollständige Ausscheidung des Kontrastmittels, zur Vermeidung einer unnötigen Belastung des Gesamtorganismus, im Vordergrund. Darüber hinaus müssen entsprechende Zubereitungen über eine ausreichende lokale und akute Verträglichkeit verfügen.

Hinsichtlich der Anwendung in der radiologischen Praxis ist neben einer möglichst einfachen Anwendbarkeit entsprechender Zubereitungen deren rascher "Wirkungseintritt" von zentraler Bedeutung. So sollte möglichst bereits innerhalb weniger Stunden nach der Kontrastmittelapplikation eine Bildgebung möglich sein.

WO 95/33494 offenbart Emulsionen von paramagnetischen Kontrastmitteln enthaltend organische Chelatoren mit einer ungesättigten aliphatischen Gruppe.

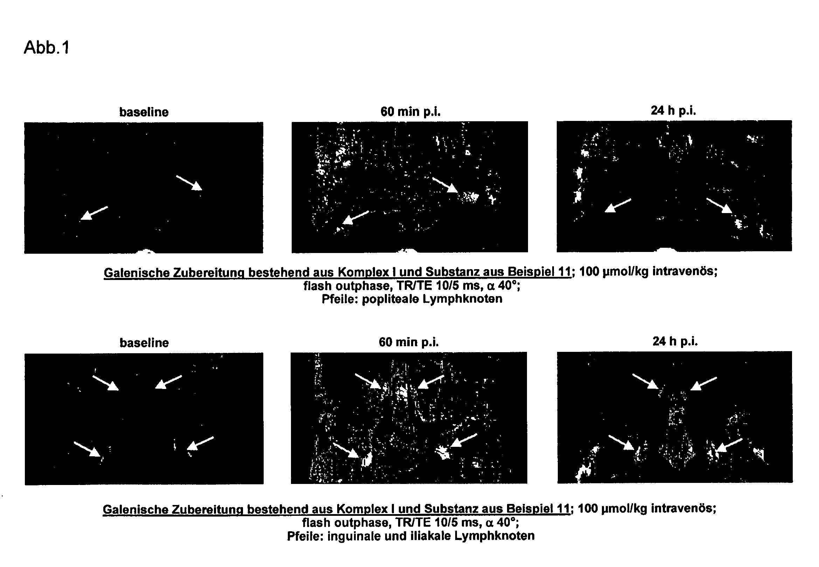

Kontrastmittel, die zur Darstellung der Lymphknoten geeignet sind, werden bereits in der deutschen Offenlegungsschrift DE 196 03 033 beschrieben. Dort sind perfluoralkylhaltige Metallkomplexe offenbart, die bevorzugt als Lymphographika eingesetzt werden (siehe Abbildung 1 der DE 196 03 033). In der deutschen Offenlegungsschrift DE 197 29 013 werden ähnliche Metallkomplexe beschrieben, die insbesondere als blood-pool-agents geeignet sind. Diese Verbindungen sind bereits recht gut als Kontrastmittel in der Lymphographie geeignet, gleichwohl ist es wünschenswert, die pharmazeutischen und pharmakologischen Eigenschaften einer Kontrastmittelformulierung weiter zu verbessern.

Aufgabe der Erfindung ist es daher, neue galenische Formulierungen zur Verfügung zu stellen, die als Kontrastmittel insbesondere für die Darstellung der Lymphknoten geeignet sind und die die obengenannten pharmazeutischen und pharmakologischen Anforderungen erfüllen.

Diese Aufgabe wird durch die galenischen Formulierungen der vorliegenden Erfindung gelöst.

Die galenischen Formulierungen der vorliegenden Erfindung enthalten paramagnetische perfluoralkylhaltige Verbindungen, welche z.B. in den Offenlegungsschriften DE 196 03 033, DE 197 29 013 und WO 97/26017 beschrieben worden sind, und zusätzlich diamagnetische perfluoralkylhaltige Substanzen. Die paramagnetischen perfluoralkylhaltigen Verbindungen sind Verbindungen der allgemeinen Formel I

R^{F}-A I

Das Molekülteil A steht beispielsweise für eine Gruppe L - M, wobei L für einen Linker und M für einen Metallkomplex, bestehend aus einem offenkettigen oder cyclischen Chelator, steht, welcher als Zentralatom ein Atom der Ordnungszahlen 21 - 29, 39, 42, 44 oder 57 - 83 enthält. Der Linker L ist dabei eine direkte Bindung, eine Methylengruppe, eine -NHCO-Gruppe, eine Gruppe 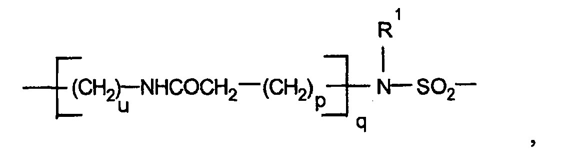 wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 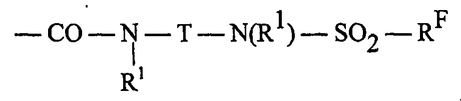 oder 1 bis 2 gegebenenfalls
substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
R¹, R^{F} und p und q die oben angegebenen Bedeutungen haben und
T eine C₂-C₁₀-Kette bedeutet, die gegebenenfalls durch 1 bis 2 Sauerstoffatome oder 1 bis 2 -NHCO-Gruppen unterbrochen ist.

Der Metallkomplex M steht dabei beispielsweise für die folgenden Metallkomplexe:
◆ einen Komplex der allgemeinen Formel II 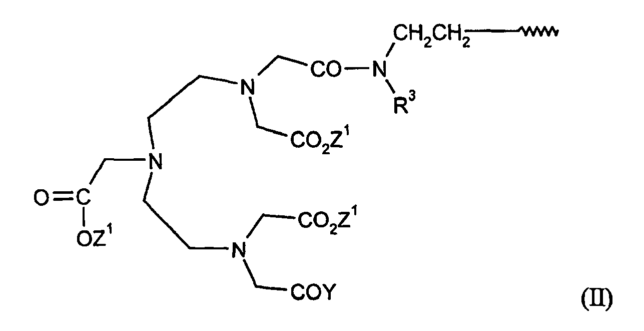 in der R³, Z¹ und Y unabhängig voneinander sind und
   - R³: die Bedeutung von R¹ hat oder -(CH₂)ₘ-L-R^{F} bedeutet, wobei m 0, 1 oder 2 ist und L und R^{F} die o.g. Bedeutung haben,
   - Z¹: unabhängig voneinander ein Wasserstoffatom oder ein Metallionenäquivalent der Ordnungszahlen 21 - 29, 39, 42, 44 oder 57 - 83 bedeutet,
   - Y: -OZ¹ oder 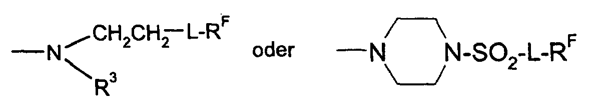
   bedeutet, wobei Z¹, L, R^{F} und R³ die o. g. Bedeutungen haben.
◆ einen Komplex der allgemeinen Formel III 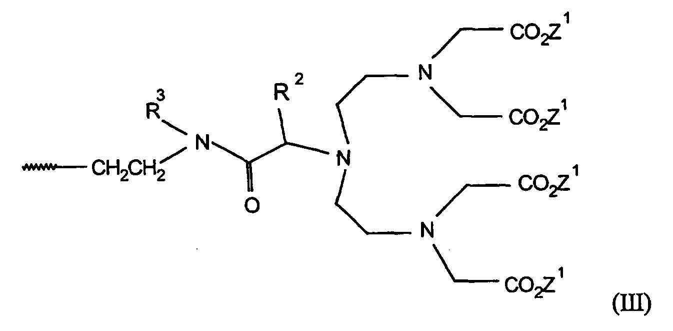 in der R³ und Z¹ die oben genannten Bedeutungen aufweisen und R² die Bedeutung von R¹ hat.
◆ einen Komplex der allgemeinen Formel IV 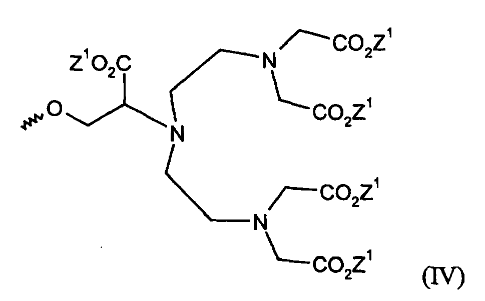 in der Z¹ die oben genannte Bedeutung hat.
◆ einen Komplex der allgemeinen Formel V 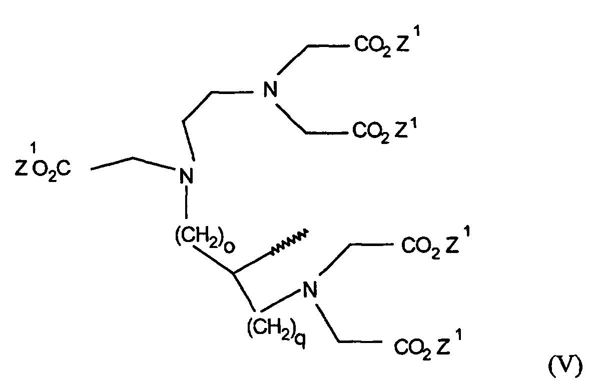 in der Z¹ die oben genannte Bedeutung hat und o und q für die Ziffern 0 oder 1 stehen und die Summe o + q = 1 ergibt.
◆ einen Komplex der allgemeinen Formel VI 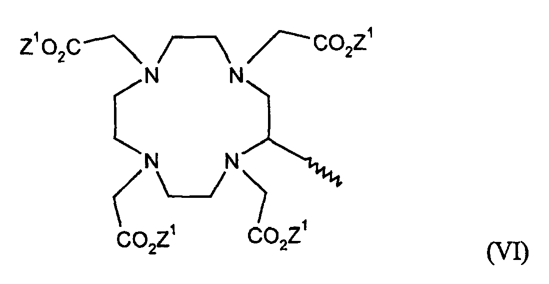 in der Z¹ die oben genannte Bedeutung hat.
◆ einen Komplex der allgemeinen Formel VII 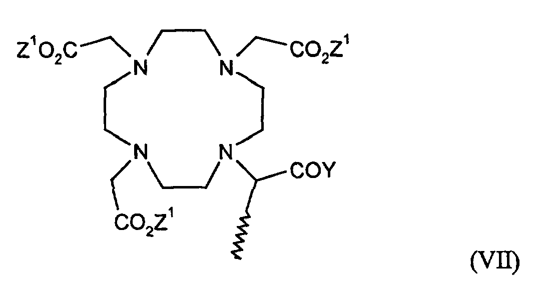 in der Z¹ und Y die oben genannten Bedeutungen haben.
◆ einen Komplex der allgemeinen Formel VIII 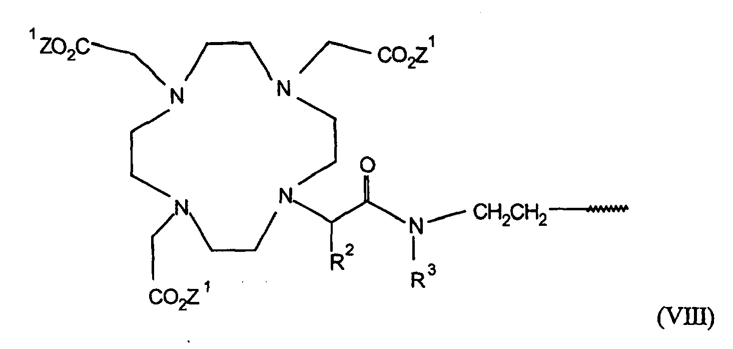 in der R³ und Z¹ die oben genannten Bedeutungen haben und R² die o.g. Bedeutung von R¹ hat.
◆ einen Komplex der allgemeinen Formel IX 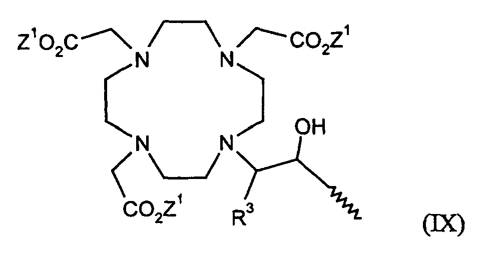 in der R³ und Z¹ die oben genannten Bedeutungen haben.
◆ einen Komplex der allgemeinen Formel X 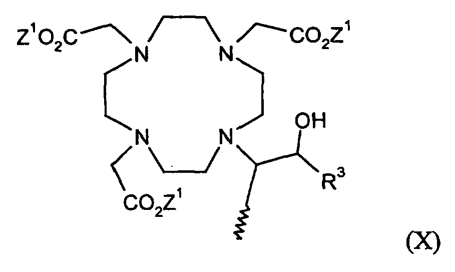 in der R³ und Z¹ die oben genannten Bedeutungen haben.
◆ einen Komplex der allgemeinen Formel XI 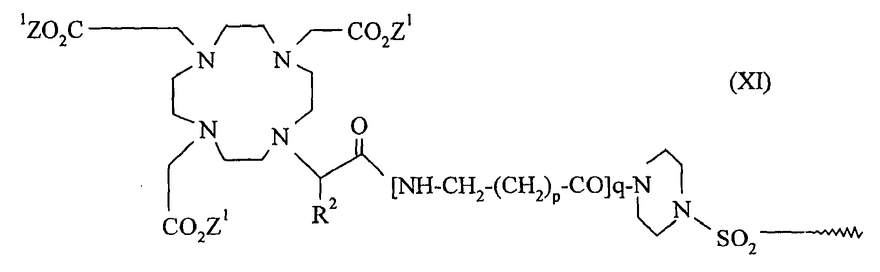 in der Z¹ p und q die oben genannte Bedeutung haben und R² die Bedeutung von R¹ hat.
◆ einen Komplex der allgemeinen Formel XII 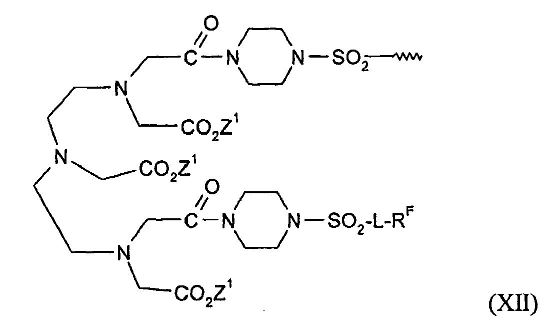 in der L, R^{F} und Z¹ die oben genannten Bedeutungen haben.
◆ einen Komplex der allgemeinen Formel XIII 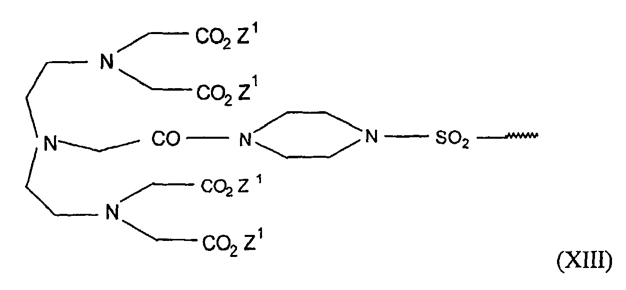 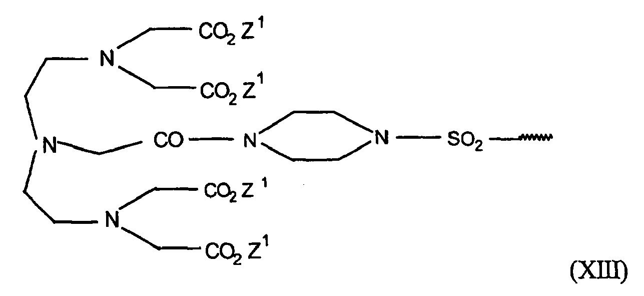 in der Z¹ die oben genannte Bedeutung hat.

Derartige Verbindungen und deren Herstellung sind in der deutschen Offenlegungsschrift DE 196 03 033 A1 und in der Internationalen Patentanmeldung WO 97/26017 beschrieben worden.

Weiter kann das Molekülteil A gemäß Formel I die folgende Struktur aufweisen: 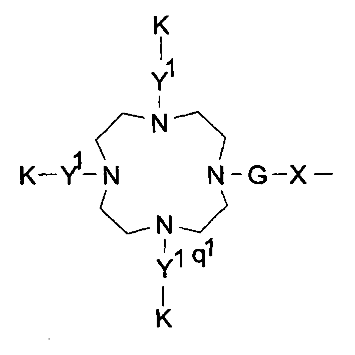 wobei
- q¹ eine Zahl 0, 1, 2 oder 3 ist,
- K für einen Komplexbildner oder Metallkomplex oder deren Salze organischer und/oder anorganischer Basen oder Aminosäuren oder Aminosäureamide steht,
- X eine direkte Bindung zur Perfluoralkylgruppe, eine Phenylengruppe oder eine C₁-C₁₀-Alkylenkette ist, die gegebenenfalls 1 - 15 Sauerstoff-, 1 - 5 Schwefelatome,
   1 - 10 Carbonyl-, 1 - 10 (NR)-, 1 - 2 NRSO₂-, 1 - 10 CONR-, 1 Piperidin-, 1 - 3 SO₂-, 1 - 2 Phenylengruppen enthält oder gegebenenfalls durch 1 - 3 Reste R^{F} substituiert ist, worin R für ein Wasserstoffatom, eine Phenyl-, Benzyl- oder eine C₁-C₁₅-Alkylgruppe steht, die gegebenenfalls 1 - 2 NHCO-, 1 - 2 CO-Gruppen, 1 - 5 Sauerstoffatome enthält und gegebenenfalls durch 1 - 5 Hydroxy-, 1 - 5 Methoxy-, 1 - 3 Carboxy-, 1 - 3 R^{F}-Reste substituiert ist
- Y' eine direkte Bindung oder eine Kette der allgemeinen Formel II¹ oder III¹ ist: 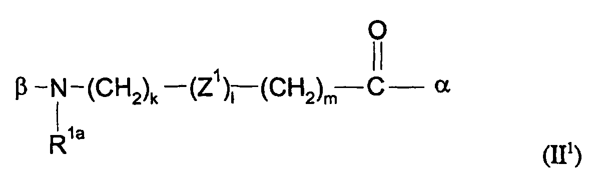 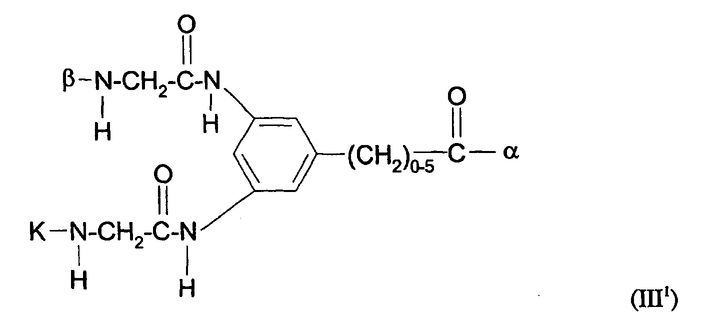 worin
   ■ R^{1a} ein Wasserstoffatom, eine Phenylgruppe, eine Benzylgruppe oder eine C₁-C₇
      Alkylgruppe ist, die gegebenenfalls substituiert ist mit einer Carboxy-, einer Methoxy- oder einer Hydroxygruppe,
   ■ Z¹ eine direkte Bindung, eine Polyglycolethergruppe mit bis zu 5 Glycoleinheiten oder ein
      Molekülteil der allgemeinen Formel IV¹ ist

      -CH(R^{2a})- (IV¹)

      worin R^{2a} eine C₁-C₇-Carbonsäure, eine Phenylgruppe, eine Benzylgruppe oder eine -(CH₂)₁₋₅-NH-K-Gruppe ist,
   ■ α die Bindung an das Stickstoffatom der Gerüstkette, β die Bindung zum Komplexbildner oder Metallkomplex K darstellt,
   ■ und in der die Variablen k und m für natürliche Zahlen zwischen 0 und 10 und 1 für 0 oder 1 stehen,
   und wobei
- G eine CO- oder SO₂-Gruppe ist.

Derartige Verbindungen und deren Herstellung sind in der deutschen Offenlegungsschrift DE 197 29 013 A1 beschrieben worden.

Weiter kann das Molekülteil A gemäß allgemeiner Formel I für eine Gruppe L¹-M¹ stehen, worin L¹ für einen Linker und M¹ für einen Metallkomplex steht. Der Linker L¹ ist dabei ein Molekülteil gemäß allgemeiner Formel XIV 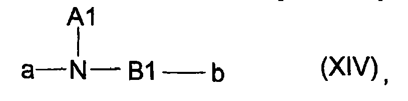 worin
N ein Stickstoffatom darstellt,
A1 ein Wasserstoffatom, eine geradkettige oder verzweigte C₁-C₃₀-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 -OR⁴-Gruppen, mit R⁴ in der Bedeutung eines Wasserstoffatoms oder eines C₁-C₇-Alkylrestes, oder -B1-R^{F} bedeutet,
B1 eine geradkettige oder verzweigte C₁-C₃₀-Alkylengruppe, die gegebenenfalls unterbrochen ist durch 1-10 Sauerstoffatome, 1-5 -NH-CO- Gruppen, 1-5 -CO-NH- Gruppen, durch eine (gegebenenfalls durch eine COOH-Gruppe substituierte) Phenylengruppe, 1-3 Schwefelatome,
   1-2 -N(B2)-SO₂- Gruppen, und/oder 1-2 -SO₂-N(B2)- Gruppen mit B2 in der Bedeutung von A1, eine NHCO-Gruppe, eine CONH-Gruppe, eine N(B2)-SO₂-Gruppe, oder eine -SO₂-N(B2)- Gruppe und/oder gegebenenfalls substituiert ist mit dem Rest R^{F},
   bedeutet,
und worin a die Bindung zum Metallkomplex M und b die Bindung zur Perfluoralkylgruppe R^{F} darstellt.

Der Metallkomplex M¹ steht dabei für einen Metallkomplex der allgemeinen Formel XV 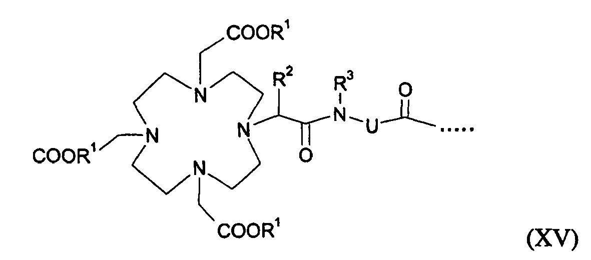 wobei
- R¹: für ein Wasserstoffatom oder ein Metallionenäquivalent der Ordnungszahlen 21-29, 31, 32, 37-39, 42-44, 49 oder 57-83,
R² und R³ für ein Wasserstoffatom, eine C₁-C₇-Alkylgruppe, eine Benzylgruppe, eine Phenylgruppe, -CH₂OH oder -CH₂-OCH₃,
U für den Rest L, wobei aber L und U unabhängig voneinander gleich oder verschieden sein können, stehen.

Derartige Verbindungen und deren Herstellung werden in der deutschen Patentanmeldung mit dem Aktenzeichen 199 14 101.0 sowie in den nachfolgenden Beispielen beschrieben.

Besonders bevorzugt sind Metallkomplexe, bei denen das Zentralatom ein Gadoliniumatom (Ordnungszahl 64) ist. Metallkomplexe mit cyclischen Chelatoren sind gegenüber denen mit offenkettigen Chelatoren bevorzugt.

Besonders bevorzugte Gadoliniumkomplexe sind der Gadoliniumkomplex von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan (Herstellung siehe WO 97/26017, Beispiel 33),
der Gadoliniumkomplex von 10-[2-Hydroxy-4-aza-5-oxo-7-oxa-10,10,11,11,12,12,13,13,14,14,15,15,16,16,17,17,17-heptadecafluorheptadecyl]-1,4,7-tris(carboxymethyl)-1,4,7,10-tetraazacyclododecan (Herstellung siehe DE 196 03 033, Beispiel 2),
1,4,7-Tris{1,4,7-tris(N-carboxylatomethyl)-10-(N-1-methyl-3,6-diaza-2,5,8-trioxooctan-1,8-diyl)-1,4,7,10-tetraazacyclododecan, Gd-Komplex}-10-(N-2H,2H,4H,4H,5H,5H-3-oxa-perfluor-tridecanoyl)-1,4,7,10-tetraazacyclododecan (Herstellung siehe DE 197 29 013, Beispiel 1),
1,4,7-Tris{1,4,7-tris[(N-carboxylatomethyl)]-10-[N-1-methyl-3-aza-2,5-dioxopentan-1,5-diyl]-1,4,7,10-tetraazacyclododecan, Gd-Komplex}-10-[2-(N-ethyl-N-perfluoroctylsulfonyl)-amino]-acetyl-1,4,7,10-tetraazacyclododecan (Herstellung siehe DE 197 29 013, Beispiel 12),
der Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan (Herstellung siehe DE 196 03 033, Beispiel 1),
1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex (Herstellung siehe Beispiele),
1,4,7-Tris(carboxylatomethyl)-10-[(3-aza-4-oxo-hexan-5-yl)-säure-N-(1H, 1H, 2H, 2H, 4H, 4H, 5H, 5H-3-oxa-perfluortridecyl)-amid]-1,4,7,10-tetraazacyclododecan, Gadoliniumkomplex (Herstellung siehe Beispiele),
1,4,7-Tris(carboxylatomethyl)-10-{(3-aza-4-oxo-hexan-5-yl)-säure-[N-3,6,9,12,15-pentaoxa)-hexadexyl)-N-(1H, 1H, 2H, 2H, 4H, 4H, 5H, 5H-3-oxa)-perfluortridecyl]-amid}-1,4,7,10-tetraazacyclododecan, Gadoliniumkomplex (Herstellung siehe Beispiele),
sowie 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex (Herstellung siehe Beispiele).

Die diamagnetischen perfluoralkylhaltigen Substanzen sind solche der allgemeinen Formel XVI:

R^{F}-L²-B² XVI

worin R^{F} einen geradkettigen oder verzweigten Perfluoralkylrest mit 4 bis 30 Kohlenstoffatomen darstellt, L² für einen Linker und B² für eine hydrophile Gruppe steht. Der Linker L² ist eine direkte Bindung, eine -SO₂-Gruppe oder eine geradkettige oder verzweigte Kohlenstoffkette mit bis zu 20 Kohlenstoffatomen, welche mit ein oder mehreren -OH, -COO⁻, -SO₃-Gruppen substituiert sein kann und/oder gegebenenfalls ein oder mehrere -O-, -S-, -CO-, -CONH-, -NHCO-, -CONR-, -NRCO-, -SO₂-, -PO₄⁻-,-NH-, -NR-Gruppen, einen Arylring oder ein Piperazin enthält, wobei R für einen C₁- bis C₂₀-Alkylrest steht, welcher wiederum ein oder mehrere O-Atome enthalten kann und/oder mit -COO⁻ oder SO₃-Gruppen substituiert sein kann.

Die hydrophile Gruppe B² ist ein Mono- oder Disaccharid, eine oder mehrere benachbarte -COO⁻ oder -SO₃⁻-Gruppen, eine Dicarbonsäure, eine Isophthalsäure, eine Picolinsäure, eine Benzolsulfonsäure, eine Tetrahydropyrandicarbonsäure, eine 2,6-Pyridindicarbonsäure, ein quartäres Ammoniumion, eine Aminopolycarbonsäure, eine Aminodipolyethylenglycolsulfonsäure, eine Aminopolyethylenglycolgruppe, eine SO₂-(CH₂)₂-OH-Gruppe, eine Polyhydroxyalkylkette mit mindestens zwei Hydroxylgruppen oder eine oder mehrere Polyethylenglycolketten mit mindestens zwei Glycoleinheiten, wobei die Polyethylenglycolketten durch eine -OH oder -OCH₃-Gruppe terminiert sind. Derartige Substanzen sind teilweise bereits bekannt, teilweise wurden solche Substanzen zur Herstellung der erfindungsgemäßen Formulierungen neu synthetisiert. Bekannte perfluoralkylhaltige Substanzen und deren Herstellung werden in den folgenden Publikationen beschrieben:
J. G. Riess, Journal of Drug Targeting, 1994, Vol. 2, pp. 455-468;
J. B. Nivet et al., Eur. J. Med. Chem., 1991, Vol. 26, pp. 953-960;
M.-P. Krafft et al., Angew. Chem., 1994, Vol. 106, No. 10, pp. 1146-1148;
M. Lanier et al., Tetrahedron Letters, 1995, Vol. 36, No. 14, pp. 2491-2492;
F. Guillod et al., Carbohydrate Research, 1994, Vol. 261, pp. 37-55;
S. Achilefu et al., Journal of Fluorine Chemistry, 1995, Vol. 70, pp. 19-26;
L. Clary et al., Tetrahedron, 1995, Vol. 51, No. 47, pp. 13073-13088;
F. Szoni et al., Journal of Fluorine Chemistry, 1989, Vol. 42, pp. 59-68;
H. Wu et al., Supramolecular Chemistry, 1994, Vol. 3, pp. 175-180;
F. Guileri et al., Angew. Chem. 1994, Vol. 106, No. 14, pp. 1583-1585;
M.-P. Krafft et al., Eur. J. Med. Chem., 1991, Vol. 26, pp.545-550;
J. Greiner et al., Journal of Fluorine Chemistry, 1992, Vol. 56, pp. 285-293;
A. Milius et al., Carbohydrate Research, 1992, Vol. 229, pp. 323-336;
J. Riess et al., Colloids and Surfaces A, 1994, Vol. 84, pp. 33-48;
G. Merhi et al., J. Med. Chem., 1996, Vol. 39, pp. 4483-4488;
V. Cirkva et al., Jounal of Fluorine Chemistry, 1997, Vol. 83, pp. 151-158;
A. Ould Amanetoullah et al., Journal of Fluorine Chemistry, 1997, Vol. 84, pp. 149-153;
J. Chen et al., Inorg. Chem., 1996, Vol. 35, pp. 1590-161;
L. Clary et al., Tetrahedron Letters, 1995, Vol. 36, No. 4, pp. 539-542;
MM. Chaabouni et al., Journal of Fluorine Chemistry, 1990, Vol. 46, pp. 307-315;
A. Milius et al., New J. Chem., 1991, Vol. 15, pp.337-344;
M.-P. Krafft et al., New J. Chem., 1990, Vol. 14, pp. 869-875;
J.-B. Nivet et al., New J. Chem., 1994, Vol. 18, pp. 861-869;
C. Santaella et al., New J. Chem., 1991, Vol. 15, pp.685-692;
C. Santaella et al., New J. Chem., 1992, Vol. 16, pp. 399-404;
A. Milius et al., New J. Chem., 1992, Vol. 16, pp. 771-773;
F. Szönyi et al., Journal of Fluorine Chemistry, 1991, Vol. 55, pp. 85-92;
C. Santaella et al., Angew. Chem., 1991, Vol. 103, No. 5, pp. 584-586;
M.-P. Krafft et al., Angew. Chem., 1993, Vol. 105, No. 5, pp. 783-785;
EP 0 548 096 B1.

Bevorzugte diamagnetische perfluoralkylhaltige Substanzen sind solche, bei denen die hydrophile Gruppe B² zusammen mit dem Linker L¹ ein Rest ausgewählt aus der Gruppe der folgenden Reste ist (n ist dabei eine Zahl zwischen 1 und 10):

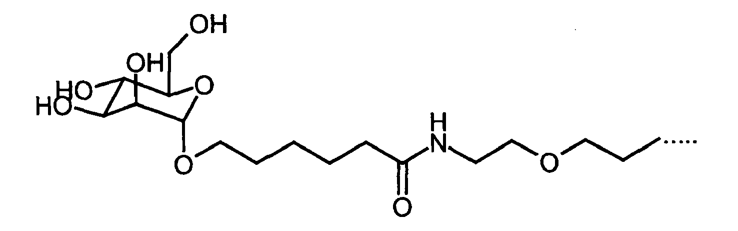

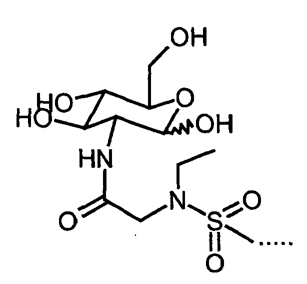

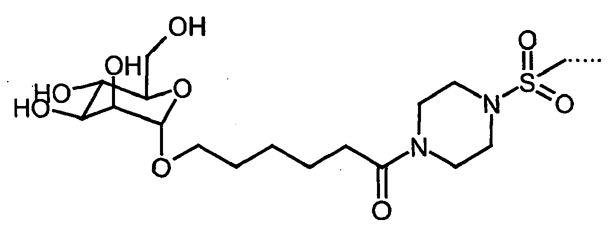

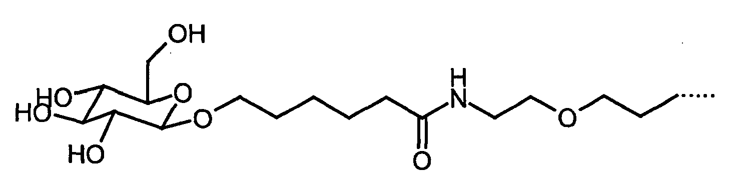

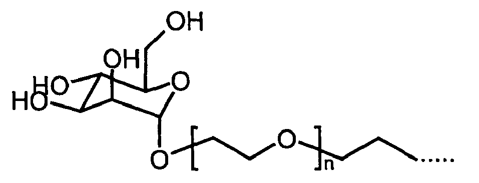

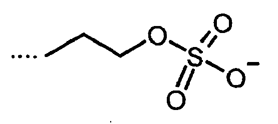

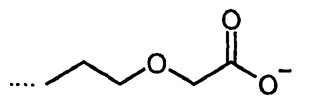

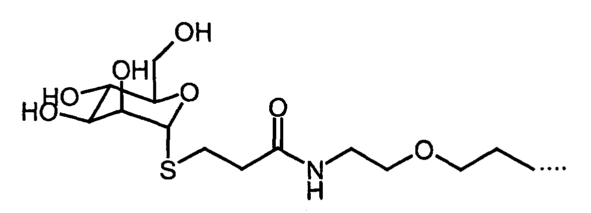

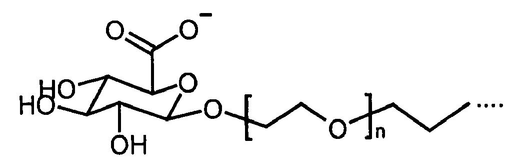

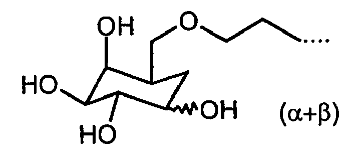

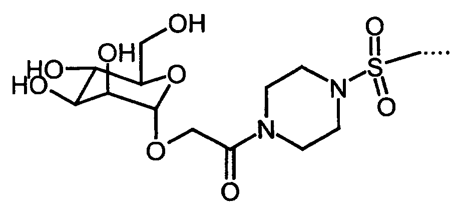

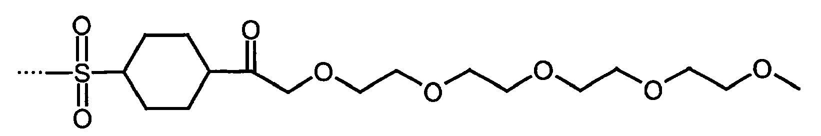

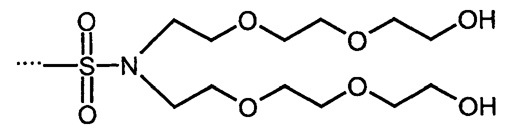

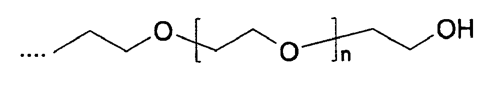

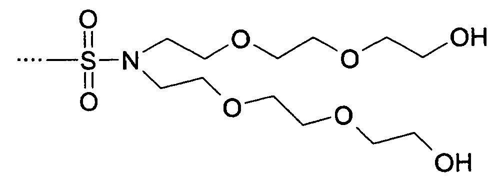

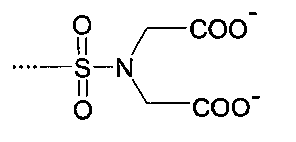

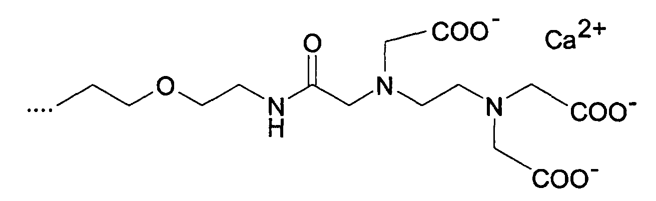

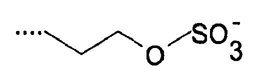

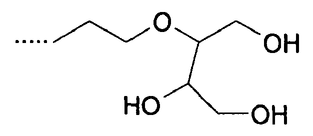

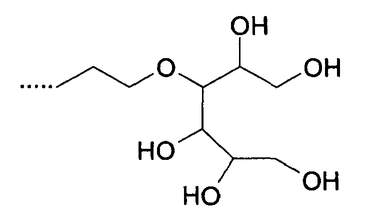

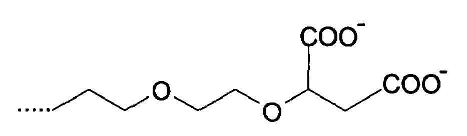

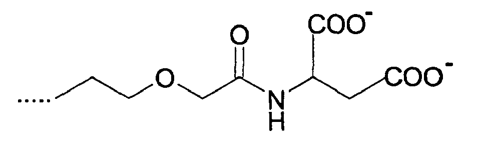

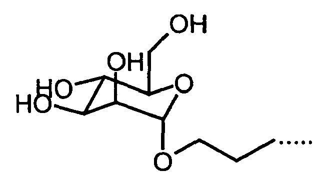

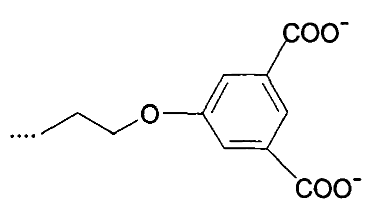

Aus dieser Gruppe sind bevorzugte diamagnetische perfluoralkylhaltige Substanzen solche mit einem Monosaccharid als hydrophiler Gruppe B².

Besonders bevorzugte diamagnetische perfluoralkylhaltige Verbindungen enthalten einen Perfluoralkylrest R_{f} mit 6 bis 12 Kohlenstoffatomen, einen Linker L², welcher eine -SO₂-Gruppe oder eine geradkettige oder verzweigte Kohlenstoffkette mit bis zu 20 Kohlenstoffatomen darstellt, die wiederum ein oder mehrere -O-, -CO-, -CONH-,-NHCO-, -CONR-, -NRCO-, -SO₂-Gruppen oder ein Piperazin enthält, worin R die oben angegebene Bedeutung hat, und ein Monosaccharid als hydrophile Gruppe B².

Weitere geeignete diamagnetische perfluoralkylhaltige Verbindungen sind Konjugate aus Cyclodextrin und perfluoralkylhaltigen Verbindungen. Diese Konjugate bestehen aus α-, β- oder γ-Cyclodextrin und Verbindungen der allgemeinen Formel XVIII

A¹-L³-R^{F} (XVIII)

worin A¹ für ein Adamantan-, Biphenyl- oder Anthracenmolekül, L³ für einen Linker und R^{F} für einen geradkettigen oder verzweigten Perfluoralkylrest mit 4 bis 30 Kohlenstoffatomen steht. Die hydrophile Gruppe B² wird bei diesen Konjugaten aus dem Cycodextrin und der Gruppe A¹ als Einschlußverbindung gebildet. Der Linker L³ ist eine geradkettige Kohlenwasserstoffkette mit 1 bis 20 Kohlenstoffatomen, welche durch ein oder mehrere Sauerstoffatome, ein oder mehrere CO-, SO₂-, CONH-, NHCO-, CONR-, NRCO-, NH-, NR-Gruppen oder ein Piperazin unterbrochen sein kann, wobei R ein C₁-C₅-Alkylrest ist.

Bevorzugte Verbindungen sind die folgenden Verbindungen:

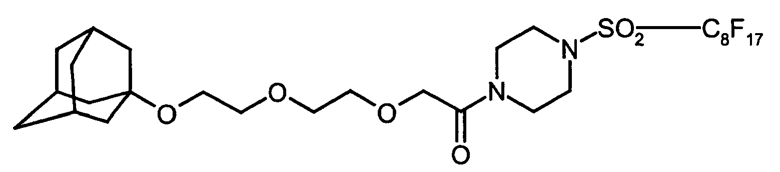

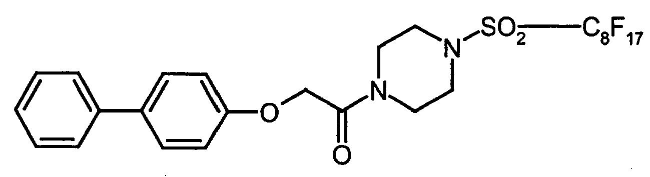

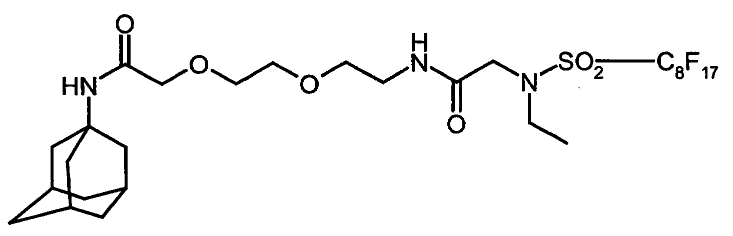

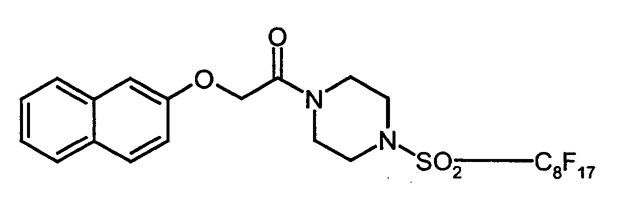

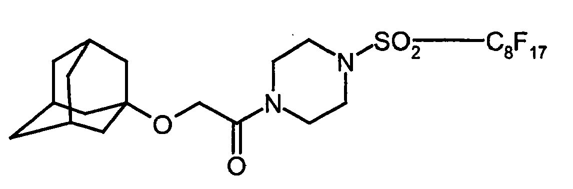

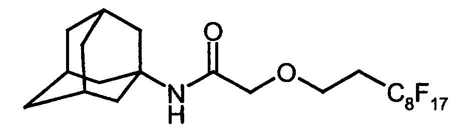

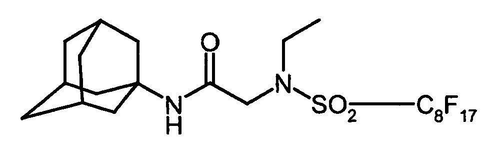

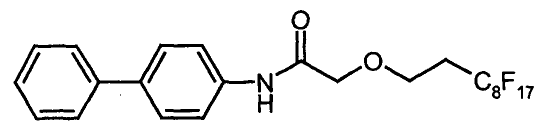

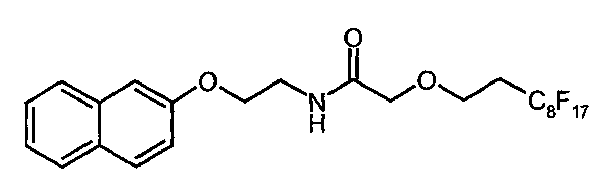

Die galenischen Formulierungen der vorliegenden Erfindung enthalten die paramagnetischen und diamagnetischen perfluoralkylhaltigen Verbindungen in einem Mischungsverhältnis zwischen 5:95 und 95:5. Bevorzugt sind Mischungsverhältnisse zwischen 40:60 und 60:40 der beiden Substanzen. Beide Substanzen werden in millimolaren Konzentrationen verwendet. Es werden Konzentrationen zwischen 0.5 und 1000 mmol/l Lösungsmittel erreicht. Das Lösungsmittel ist bevorzugt Wasser. Die Metallkonzentration der Formulierungen liegt bevorzugt in einem Bereich von 50 - 250 mmol/l, da nur dann diagnostisch relevante Bilder in der Kernspintomographie erhalten werden.

Bevorzugt sind Mischungen aus paramagnetischen und diamagnetischen perfluoralkylhaltigen Verbindungen, bei denen die Perfluoralkylketten eine Länge von 6 bis 12 Kohlenstoffatomen haben. Besonders bevorzugt sind Mischungen, bei denen sowohl die paramagnetischen als auch die diamagnetischen perfluoralkylhaltigen Verbindungen eine Perfluoralkylkette mit 8 Kohlenstoffatomen aufweisen.

Die neuen galenischen Formulierungen zeigen überraschende Vorteile bei ihrer Verwendung als Kontrastmittel in der Kernspintomographie. Gegenüber den bereits bekannten Kontrastmitteln zeigen sie eine verbesserte Verträglichkeit und eine nahezu vollständige Ausscheidung. Weiter ist auch die lokale Verträglichkeit höher als bei den bisher bekannten Kontrastmitteln, und gleichzeitig zeigen die neuen Formulierungen eine höhere Organspezifität. Die Anreicherung in den Lymphknoten ist höher als bei den bekannten Kontrastmitteln für die Lymphographie. Dies führt zu einer höheren Relaxivity und damit zu einer verbesserten Bildgebung.

Die Herstellung der galenischen Formulierungen erfolgt dadurch, daß die paramagnetischen perfluoralkylhaltigen Verbindungen (Komponente A) und die diamagnetischen perfluoralkylhaltigen Substanzen (Komponente B) in Molenbrüchen zwischen 0.05 und 0.95 an Komponente A oder B eingewogen und in einem geeigneten Lösungsmittel gelöst werden. Ein besonders gut geeignetes Lösungsmittel ist Wasser. Dieser Lösung werden dann übliche galenische Zusätze wie z.B. Pufferlösungen und das Ca-Salz des Komplexbildners im Überschuß zugegeben. Bei 10 bis 100°C werden die Lösungen stark gerührt. Alternativ können die Lösungen bei 10 bis 100°C in einem Ultraschallbad behandelt werden. Eine weitere Alternative besteht darin, daß man die Lösungen mit Mikrowellen behandelt.

Bei Stoffen, die sich als Einzelkomponenten nicht in Wasser lösen, erweist es sich als vorteilhaft, einen Lösungsvermittler wie Alkohol (z.B. Methanol oder Ethanol) oder ein anderes mit Wasser mischbares Lösungsmittel zuzusetzen und dieses dann langsam abzudestillieren. Die Destillation kann unter Vakuum erfolgen. Der Rückstand wird anschließend in Wasser gelöst und die Lösung filtriert. Es ist auch möglich, jede Komponente für sich in jeweils einem Lösungsmittel getrennt zu lösen, dann zusammenzufügen und wie oben weiterzuverfahren. Als vorteilhaft hat es sich erwiesen, eine relativ stark konzentrierte Lösung (> 100 mmol) des Metallkomplexes (Komponente A) vorzulegen und dann Komponente B pur zuzugeben und wie oben erwähnt die Lösung zu Rühren oder mit Ultraschall bzw. Mikrowellen zu behandeln.

Beim Menschen können die galenischen Formulierungen der vorliegenden Erfindung lokal (entweder subkutan oder direkt perkutan in das interessierende Gewebe) injiziert werden. Es sind mehrere Injektionsorte (Quaddeln) mit einem jeweiligen Injektionsvolumen von 0.2 bis 1 ml gruppiert um den interessierenden Bereich herum (z.B. Tumor) möglich. Das injizierte Gesamtvolumen sollte dabei 5 ml in keinem Fall übersteigen. Das bedeutet, daß in der Formulierung eine Metallkonzentration von 50 - 250 mmol/l vorliegen muß, damit eine potentielle klinische Dosis von 5 - 10 µmol/kg Körpergewicht mit diesem Volumen appliziert werden kann. Der Applikationsort hängt davon ab, ob ein bestimmtes Lymphabflußgebiet aus dem dazu zugeordneten Gewebe spezifisch angefärbt werden soll (z.B. bei gynäkologischen oder Rektumtumoren), oder ob das unbekannte Abflußgebiet einer bestimmten Läsion (ergo der Bereich für eine mögliche therapeutische Intervention z.B. beim Melanom oder Mammakarzinom) dargestellt werden soll. Um eine klinisch relevante Aussage zum Lymphknotenstatus z.B. bei malignen Tumoren zu erhalten, ist eine Anreicherung über drei aufeinanderfolgende Lymphknotenstationen mit relativ gleichmäßiger Verteilung (i.d.R. Konzentrationsabfall zwischen der ersten und der dritten Station nicht größer als Faktor von 3-4) wünschenswert. Für die MRT-Bildgebung werden im normalen Lymphknotengewebe, wo die Anreicherung der Verbindung erfolgt, Gadoliniumkonzentrationen von mindestens 50 µmol/l und höchstens 2500 µmol/l benötigt. Die Bildgebung kann (je nach Injektionsort und Gewebe) nach 30 Minuten erfolgen und ist dann noch für weitere 4 bis 6 Stunden nach Injektion möglich. Da mit den erfindungsgemäßen Verbindungen von Gadoliniumkomplexen vor allem die T1-Relaxationszeiten des Lymphknotengewebes beeinflußt werden, sind schnelle T1-gewichtete Sequenzen (z.B. Gradientenechosequenzen mit TR von 10-20 ms, TE von 5 ms und Flipwinkeln von 40-80°) am besten in der Lage, ein MRT-Enhancement der Lymphknotenstationen nachzuweisen. Da Lymphknoten sehr häufig in Fettgewebe eingebettet sind und dieses eine sehr hohe Signalintensität auf solchen Sequenzen besitzt, bieten sich auch fettunterdrückte Meßmethoden an. Paramagnetische Gadoliniumkomplexe in Verbindung mit T1-gewichteten Meßsequenzen haben gegenüber Formulierungen von superparamagnetischen Eisenoxidpartikeln den großen Vorteil, daß sie MRT-Bilder mit höherer räumlicher Auflösung, mit geringeren Distorsionsartefakten (aufgrund von Suszeptibilitätsartefakten) und mit kürzerer Aufnahmezeit erlauben. Da eine positive Markierung der Lymphknoten erfolgt (d.h. Signalanstieg), sind auch MRT-Aufhahmen ohne Kontrastmittel zum Vergleich nicht mehr zwingend notwendig, und die Gesamtuntersuchungszeit pro Patient kann verkürzt werden.

Die erfindungsgemäßen galenischen Formulierungen sind besonders als Kontrastmittel für die Kernspintomographie geeignet. Neben der Darstellung der Lymphknoten gelingt mit den erfindungsgemäßen galenischen Formulierungen auch die Darstellung des blood-pools.

Die nachfolgenden Beispiele erläutern den Gegenstand der Erfindung, ohne ihn auf diese beschränken zu wollen.

| **Die Herstellung der verwendeten Metallkomplexe I - V beschrieben in Synthese der Metallkomplexe VI - IX** | |
|---|---|
| Komplex | **Literaturstelle** |
| I | WO 97/26017 |
| | Gadolinium-Komplex von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan |
| II | WO 97/26017 |
| | Gadolinium-Komplex von 10-[2-Hydroxy-4-aza-5-oxo-7-oxa-10,10,11,11, 12,12, 13,13,14,14,15,15,16,16,17,17,17-heptadecafluorheptadecyl]-1,4,7-tris(carboxymethyl)-1,4,7,10-tetraazacyclododecan |
| III | DE 197 29 013 |
| | 1,4,7-Tris{1,4,7-tris(N-carboxylatomethyl)-10-(N-1-methyl-3,6-diaza-2,5,8-trioxo-octan-1,8-diyl)1,4,7,10-tetraazacyclododecan, Gd-Komplex}-10-(N-2H,2H,4H,4H, 5H,5H-3-oxa-perfluor-tridecanoyl)-1,4,7,10-tetraazacyclododecan |
| IV | DE 197 29 013 |
| | 1,4,7-Tris{1,4,7-tris(N-carboxylatomethyl)-10-(N-1-methyl-3-aza-2,5-dioxo-pentan-1,5-diyl]-1,4,7,10-tetraazacyclododecan, Gd-Komplex}-10-[2-(N-ethyl-N-perfluoroctylsulfonyl)-amino]-acetyl-1,4,7,10-tetraazacyclododecan |
| V | WO 97/26017 |
| | Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10- tetraazacyclododecan |

### Metallkomplex VI

### a) 2H, 2H, 4H, 4H, 5H, 5H-3-oxa)-perfluortridecansäure-N-(2,3-dihydroxypropyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 5,47 g (60 mmol) 2,3-Dihydroxypropylamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Ethanol= 15:1) chromatographiert.
Ausbeute: 29,70 g (87 % d. Th.) eines farblosen Feststoffes

| Elementaranalyse: | | | | |
|---|---|---|---|---|
| ber.: | C 30,32 | H 2,20 | N 2,36 | F 54,35 |
| gef.: | C 30,12 | H 2,41 | N 2,18 | F 54,15 |

### b) N-(2,3-Dihydroxypropyl)-N-(1H, 1H, 2H, 2H, 4H, 4H, 5H, 5H-3-oxa-perfluortridecyl)-amin

30 g (48,8 mmol) der Titelverbindung aus Beispiel VIa werden in 300 ml Tetrahydrofuran gelöst und 50 ml 10 M Borandimethylsulfid (in Tetrahydrofuran) zugegeben. Man kocht 16 Stunden unter Rückfluß. Es wird auf 0°C abgekühlt und 300 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 60°C gerührt. Man dampft im Vakuum zur Trockne ein, nimmt den Rückstand in 300 ml 5 %iger aqu. Natronlauge auf und extrahiert 3 Mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/Methanol= 15:1).
Ausbeute: 24,07 g (85 % d. Th.) eines farblosen Feststoffes

| Elementaranalyse: | | | | |
|---|---|---|---|---|
| ber.: | C 31,05 | H 2,61 | N 2,41 | F 55,66 |
| gef.: | C 31,91 | H 2,78 | N 2,33 | F 55,47 |

### c) 1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,21 (15,88 mmol) der Titelverbindung aus Beispiel VIb zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,09 g (85 % d. Th.) eines farblosen, amorphen Pulvers
Wassergehalt: 6,3 %

| Elementaranalyse (berechnet auf wasserfreie Substanz): | | | | | |
|---|---|---|---|---|---|
| ber.: | C 34,26 | H 3,64 | N 7,05 | F 27,10 | Gd 13,19 |
| gef.: | C 34,12 | H 3,83 | N 6,91 | F 26,88 | Gd 12,93 |

### Metallkomplex VII

### 1,4,7-Tris(carboxylatomethyl)-10-[(3-aza-4-oxo-hexan-5-yl)-säure-N-(1H, 1H, 2H, 2H, 4H, 4H, 5H, 5H-3-oxa-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid und 3,66 g (31,76 mmol) N-Hydroxysuccinimid werden bei 60 °C in 100 ml Dimethylsulfoxid gelöst. Man kühlt auf 15 °C ab und gibt 3,51 (17 mmol) N,N'-Dicyclohexylcarbodiimid zu und rührt 5 Stunden bei 15 °C. Zur Abtrennung des Harnstoffes wird die Lösung filtriert. Zum Filtrat gibt man 8,63 g (15,88 mmol) der Titelverbindung aus Beispiel VIIIb und 5,06 g (50 mmol) Triethylamin zu und rührt 12 Stunden bei Raumtemperatur. Man gießt die Lösung in 1500 ml Diethylether/100 ml Aceton und rührt 30 Minuten. Der ausgefallene Feststoff wird abfiltriert und an Kieselgel RP-18 chromatographiert (Laufmittel: Gradient aus Tetrahydrofuran/Acetonitril/Wasser).
Ausbeute: 13,86 g (78 % d. Th.) eines farblosen, amorphen Pulvers
Wassergehalt: 9,3 %

| Elementaranalyse (berechnet auf wasserfreie Substanz): | | | | | |
|---|---|---|---|---|---|
| ber.: | C 33,28 | H 3,42 | N 7,51 | F 28,87 | Gd 14,05 |
| gef.: | C33,12 | H 3,61 | N 7,37 | F 28,69 | Gd 13,89 |

### Metallkomplex VIII

### a) 2H, 2H, 4H, 4H, 5H, 5H-3-oxa-perfluortridecansäureamid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 200 ml Dichlormethan gelöst. Dann wird bei 0°C Ammoniakgas für ca. 2 Stunden in die Lösung geleitet. Man rührt 4 Stunden bei 0°C nach, anschließend 2 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 27,85 g (93 % d. Th.)

| Elementaranalyse: | | | | |
|---|---|---|---|---|
| ber.: | C 27,66 | H 1,55 | N 2,69 | F 61,97 |
| gef.: | C 27,49 | H 1,72 | N 2,54 | F 61,81 |

### b) 1H, 1H, 2H, 2H, 4H, 4H, 5H, 5H-3-oxa-perfluortridecylamin, Hydrochlorid

27 g (51,8 mmol) der Titelverbindung aus Beispiel VIIIa werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 400 ml Ethanol/100 ml 10 %iger aqu. Salzsäure aufgenommen und 8 Stunden bei 60°C gerührt. Man dampft im Vakuum zur Trockne ein und kristallisiert den Rückstand aus wenig Ethanol/Diethylether um.
Ausbeute: 26,75 g (95 % d. Th.) eines farblosen, kristallinen Feststoffes

| Elementaranalyse: | | | | | |
|---|---|---|---|---|---|
| ber.: | C 26,51 | H 2,04 | N 2,58 | F 59,41 | Cl 6,52 |
| gef.: | C 26,37 | H 2,21 | N 2,46 | F 59,25 | Cl. 6,38 |

### c) 3,6,9,12,15-Pentaoxahexadecansäure-N-(1H, 1H, 2H, 2H, 4H, 4H, 5H, 5H-3-oxa)-perfluortridecyl)-amid

Zu 26,5 g (48,74 mmol) der Titelverbindung aus Beispiel VIIIb und 14,8 g (146,2 mmol) Triethylamin, gelöst in 300 ml Dichlormethan tropft, gibt man bei 0°C 14,24 g (50 mmol) 3,6,9,12,15-Pentaoxahexadecansäurechlorid und rührt 3 Stunden bei 0°C. Man gibt 300 ml 5 % ige aqu. Salzsäure zu und rührt 30 Minuten gut durch. Die organische Phase wird abgetrennt, über Magnesiumsulfat getrocknet und im Vakuum zur Trockne eingedampft. Der Rückstand wird an Kieselgel chromatographiert (Laufmittel: Dichlormethan/Aceton: 20:1).
Ausbeute: 32,03 g (87 % d. Th.) eines farblosen Öls

| Elementaranalyse: | | | | |
|---|---|---|---|---|
| ber.: | C 36,57 | H 4,00 | N 1,85 | F 42,75 |
| gef.: | C 36,46 | H 4,12 | N 1,76 | F 42,53 |

### d) N-(3,6,9,12,15-Pentaoxahexadecyl)-N-(1H, 1H, 2H, 2H, 4H, 4H-3-oxa)-perfluortridecyl)-amid

31 g (41,03 mmol) der Titelverbindung aus Beispiel VIIIc werden in 300 ml Tetrahydrofuran gelöst und 25 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2 Propanol= 15:1).
Ausbeute: 27,68 g (91 % d. Th.)

| Elementaranalyse: | | | | |
|---|---|---|---|---|
| ber.: | C 37,26 | H 4,35 | N 1,89 | F 43,56 |
| gef.: | C 37,11 | H 4,51 | N 1,73 | F 43,41 |

### e) 1,4,7-Tris(carboxylatomethyl)-10-{(3-aza-4-oxo-hexan-5-yl)-säure-[N-3,6,9,12,15-pentaoxa)-hexadexyl)-N-(1H, 1H, 2H, 2H, 4H, 4H, 5H, 5H-3-oxa)-perfluortridecyl]-amid}-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 11,77 (15,88 mmol) der Titelverbindung aus Beispiel VIIId zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 18,05 g (84 % d. Th.) eines farblosen, amorphen Pulvers
Wassergehalt: 6,2 %

| Elementaranalyse (berechnet auf wasserfreie Substanz): | | | | | |
|---|---|---|---|---|---|
| ber.: | C 37,28 | H 4,47 | N 6,21 | F 23,87 | Gd 11,62 |
| gef.: | C 37,11 | H 4,61 | N 6,03 | F 23,64 | Gd 11,42 |

### Metallkomplex IX

### a) 2H, 2H, 4H, 4H, 5H, 5H-3-oxa-perfluortridecansäure-N-(5-hydroxy-3-oxa-pentyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 6,25 g (60 mmol) 5-Hydroxy-3-oxa-pentylamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 15:1) chromatographiert.
Ausbeute: 32,20 g (92 % d. Th.) eines farblosen Feststoffes

| Elementaranalyse: | | | | |
|---|---|---|---|---|
| ber.: | C 31,54 | H 2,65 | N 2,30 | F 53,01 |
| gef.: | C 31,61 | H 2,84 | N 2,14 | F 52,85 |

### b) N-(5-Hydroxy-3-oxa-pentyl)-N-(1H, 1H, 2H, 2H, 4H, 4H, 5H, 5H-3-oxa-perfluortridecyl)-amin

30 g (49,24 mmol) der Titelverbindung aus Beispiel IXa werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 10 Stunden bei 50°C gerührt. Man dampft im Vakuum zur Trockne ein, nimmt den Rückstand in 300 ml 5 %iger aqu. Natronlauge auf und extrahiert 3 Mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 26,09 g (89 % d. Th.) eines farblosen Feststoffes

| Elementaranalyse: | | | | |
|---|---|---|---|---|
| ber.: | C 32,28 | H 3,05 | N 2,35 | F 54,25 |
| gef.: | C 32,12 | H 3,21 | N 2,18 | F 54,09 |

### c) 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,45 (15,88 mmol) der Titelverbindung aus Beispiel IXb zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,10 g (84 % d. Th.) eines farblosen, amorphen Pulvers
Wassergehalt: 5,7 %

| Elementaranalyse (berechnet auf wasserfreie Substanz): | | | | | |
|---|---|---|---|---|---|
| ber.: | C 34,83 | H 3,84 | N 6,96 | F 26,76 | Gd 13,03 |
| gef.: | C 34,65 | H 3,96 | N 6,84 | F 26,62 | Gd 12,91 |

### Beispiel 1

### a) 1,2,3,4,6-Penta-O-acetyl-α,β-D-mannopyranose

Auf analoge Weise, wie in der Literatur beschrieben [M.L.Wolfrom und A.Thompson in Methods in Carbohydrate Chemistry (R.L. Whistler, M.L. Wolfrom and J.N. BeMiller, Eds.), Academic Press, New York, Vol.II, 53 , pp. 211-215, (1963)] liefert die Umsetzung von 150 g (832.5 mmol) α,β-D-Mannopyranose mit einem Gemisch aus 1500 ml absolutem Pyridin und 1500 ml Essigsäureanhydrid nach Aufarbeitung 315 g (96,7 %) der oben genannten Titelverbindung als Rohprodukt in Form eines viskosen und farblosen Öls. Durch ¹H-NMR-spektroskopische Untersuchung der so erhaltenen Titelverbindung konnte das α zu β-Verhältnis beider Anomeren mit 4:1 bestimmt werden. Auf eine Trennung der α,β-Anomeren der oben genannten Titelverbindung kann zur Durchführung der nachfolgenden Reaktionsschritte verzichtet werden.

| Elementaranalyse: | | |
|---|---|---|
| ber.: | C 49,21 | H 5,68 |
| gef.: | C 49,12 | H 5,78 |

### b) 6-[1-O-α-(2,3,4,6-Tetra-O-acetyl-D-mannopyranosyl)-hexansäureethylester]

Auf analoge Weise, wie in der Literatur für die Synthese von Aryl Glycopyranosiden beschrieben [J. Conchie und G.A. Levvy in Methods in Carbohydrate Chemistry (R.L. Whistler, M.L. Wolfrom and J.N. BeMiller, Eds.), Academic Press, New York, Vol.II, 90, pp. 345-347, (1963)] führt die Umsetzung von 156,2 g (400 mmol) der Titelverbindung aus Beispiel 1a) als α, β-Anomerengemisch mit 67 ml (400 mmol) 6-Hydroxy-hexansäureethylester und 60,8 ml (520 mmol) Zinn-IV-chlorid in insgesamt 600 ml 1,2-Dichlorethan nach säulenchromatographischer Aufreinigung (Eluent: Hexan/ Essigsäureethylester 2:1) zur Bildung von 100,05 g (51 % d. Th.) der oben genannten Titelverbindung als farbloses und viskoses Öl. Durch ¹H-NMR-spektroskopische Untersuchung der so erhaltenen Titelverbindung konnte gezeigt werden, daß es sich bei der oben genannten Titelverbindung ausschließlich um das reine α-Anomere handelt.

| Elementaranalyse: | | |
|---|---|---|
| ber.: | C 52,94 | H 6,77 |
| gef.: | C 52,80 | H 6,78 |

### c) 6-[1-O-α-(2,3,4,6-Tetra-O-benzyl-D-mannopyranosyl)-hexansäure

Eine gerührte Suspension von 141,0 g (289 mmol) der Titelverbindung aus Beispiel 1b) in 200 ml Dioxan wird bei Raumtemperatur und unter gleichzeitigem kräftigen Rühren portionsweise mit insgesamt 238,5 g (4,26mol) fein gepulvertem Kaliumhydroxydpulver versetzt . Zur Erhöhung der Rührfähigkeit wird das Reaktionsgemisch mit weiteren 200 ml Dioxan versetzt und die so erhaltene Suspension im Anschluß zur Siedehitze erhitzt und bei dieser Temperatur mit insgesamt 372 ml (3,128 mol) Benzylbromid über einen Zeitraum von zwei Stunden tropfenweise versetzt. Nach einer Reaktionszeit von 4 Stunden bei 110 °C gefolgt von 12 Stunden bei Raumtemperatur wird das Reaktionsgemisch zum Zwecke der Aufarbeitung in insgesamt 2,5 Liter Eiswasser langsam eingegossen und die Wasserphase im Anschluß vollständig mit Diethylether extrahiert. Nach dem Waschen der so erhaltenen Etherphase und dem anschließenden Trocknen der selbigen über Natriumsulfat wird vom Salz abgesaugt und der Diethylether im Vakuum abgezogen. Überschüssiges Benzylbromid wird anschließend im Ölpumpenvakuum quantitativ bei einer Ölbadtemperatur von 180 °C aus dem Reaktionsgemisch abdestilliert. Der so erhaltene, harzig-ölige Rückstand wird an Kieselgel unter Verwendung von Essigsäureethylester/Hexan (1:10) als Eluent gereinigt.
Ausbeute: 172,2, g (91,0 % d. Th.) der oben genannten Titelverbindung in Form eines farblosen und äußerst viskosen Öls

| Elementaranalyse: | | |
|---|---|---|
| ber.: | C 75,68 | H 7,16 |
| gef.: | C 75,79 | H 7,04 |

### d) 6-[1-O-α-(2,3,4,6-Tetra-O-benzyl-D-mannopyranosyl)-hexansäure-N-(3-oxa-1H,1H,2H,2H,4H,4H,5H,5H-perfluortridecyl)-amid

In 1200 ml trockenem Tetrahydrofuran werden 100g (134 mmol) der in Beispiel 1c) beschriebenen Säure sowie 13.5 g (134 mmol) Triethylamin gelöst. Nach dem Abkühlen auf -15°C tropft man unter Rühren eine Lösung von 18.45 g (135 mmol) Chlorameisensäureisobutylester in 200 ml trockenem Tetrahydrofuran langsam hinzu, wobei die Innentemperatur -10°C nicht überschreitet. Nach einer Reaktionszeit von 15 Minuten bei -15°C tropft man eine Lösung von 165.5 g (134 mmol) 1-Amino-1H,1H,2H,2H-perfluorodecan und 13.5 g (134 mmol) Triethylamin in 250 ml trockenem Tetrahydrofuran bei -20°C hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 300 ml Essigsäureethylester aufgenommen und zweimal mit je 400 ml gesättigter Natriumhydrogencarbonatlösung sowie einmal mit 500 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (10:5:1) als Eluent gereinigt.
Ausbeute: 143,8 g (86,9 % d. Th.)

| Elementaranalyse: | | | | |
|---|---|---|---|---|
| ber.: | C 57,38 | H 4,98 | N 1,13 | F 26,15 |
| gef.: | C 57,30 | H 5,44 | N 1,01 | F 26,25 |

### e) 6-[1-O-α-D-mannopyranosyl)-hexansäure N-(3-oxa-1H,1H,2H,2H,4H,4H,5H,5H-perfluortridecyl)-amid

40,0 g (32,38 mmol) der Titelverbindung aus Beispiel 1d) werden in 750 ml 2-Propanol gelöst und mit 2,0 g Palladium-Katalysator (10 % Pd/C) versetzt. Die Reaktionslösung wird für 12 Stunden bei 22°C und 1 Atmosphäre Wasserstoffdruck hydriert. Anschließend filtriert man vom Katalysator ab und engt das Filtrat zur Trockne ein. Der verbleibende Rückstand wird in 300 ml Dimethylsulfoxid aufgenommen und aus der so erhaltenen Produktlösung erhält man durch Versetzen mit insgesamt 1000ml Diethylether nach dem Absaugen des ausgefallenen Feststoffes 21,52 g (88,0 % d. Th.) der oben genannten Titelverbindung als farbloses und kristallines Pulver mit dem Zersetzungsschmelzpunkt von 88,5 °C.

| Elementaranalyse : | | | | |
|---|---|---|---|---|
| ber.: | C 36,01 | H 5,92 | N 1,75 | F 40,34 |
| gef.: | C 36,07 | H 6,08 | N1,76 | F 40,66 |

### f) Herstellung einer Formulierung aus Gadoliniumkomplex I und 6-[1-O-α-D-mannopyranosyl)-hexansäure N-(3-oxa-1H,1H,2H,2H,4H,4H,5H,5H-perfluortridecyl)-amid

Zu 35 ml einer Lösung des Gadoliniumkomplexes I (280 mmol/L) gelöst in 0,45 % aqu. Kochsalzlösung (pH 7,4; 0,25 mg/L CaNa₃DTPA) gibt man 3,17 g (4,2 mmol) der Titelverbindung aus Beispiel 1e und füllt mit 0,9 %iger aqu. Kochsalzlösung auf insgesamt 98 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 2

### a) 1-O-α-D-[(1-Perfluoroctylsulfonylpiperazin-4-carbonyl)-pentyl-5]-2,3,4,6-tetra-O-benzyl-mannopyranose

In 800 ml eines Gemisches aus Tetrahydrofuran/Acetonitril (Mischungsverhältnis 7:3) werden 74,59 g (100 mmol) der in Beispiel 1c) beschriebenen Säure sowie 10,11 g (100 mmol) Triethylamin gelöst. Anschließend wird bei Raumtemperatur tropfenweise mit 500 ml einer Tetrahydrofuran lösung von 58.0 g (102.0 mmol) 1-Perfluoroctylsulfonylpiperazin; 10,11 g (100 mmol) Triethylamin und 16.84 g (110 mmol) 1-Hydroxybenzotriazol versetzt. Die so erhaltene Reaktionslösung wird bei -5 °C mit einer Lösung von 22,7 g (110 mmol) Dicyclohexylcarbodiimid, gelöst in 100 ml Tetrahydrofuran, versetzt und anschließend bei -5 °C noch für zwei weitere Stunden gerührt. Nach dem Auftauen der Reaktionslösung wird bei Raumtemperatur weitere 12 Stunden gerührt, vom ausgefallenen Dicyclohexylharnstoff abfiltriert und das erhaltene Filtrat im Vakuum bis zur Trockne eingeengt. Der verbleibende Rückstand wird in 600 ml Essigsäureethylester aufgenommen und zweimal mit je 300 ml gesättigter Natriumhydrogencarbonatlösung sowie zweinmal mit je 300 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Aceton/2-Propanol (16:2:1) als Eluent gereinigt
Ausbeute: 113,01 g (79,8 % d. Th.) eines farblosen und viskosen Öls

| Elementaranalyse: | | | | | |
|---|---|---|---|---|---|
| ber.: | C 58,52 | H 4,27 | N 1,98 | S 2,26 | F 22,80 |
| gef.: | C 58,42 | H 4,41 | N 1,80 | S 2.28 | F 23,02 |

### b) 1-O-α-D-[(1-Perfluoroctylsulfonyl-piperazin-4-carbonyl)-pentyl-5]-mannopyranose

50 g (35,30 mmol) der Titelverbindung aus Beispiel 2a) werden in einer Mischung bestehend aus 500 ml 2-Propanol und 50 ml Wasser gelöst und 2 g Palladiumkatalysator (10 % Pd auf Aktivkohle) hinzugegeben. Man hydriert für 12 Stunden bei Raumtemperatur. Es wird vom Katalysator abfiltriert und das Filtrat im Vakuum zur Trockne eingedampft. Der Rückstand wird in 200 ml Methanol gelöst und das Reaktionsprodukt durch Versetzen mit insgesamt 800 ml Diethylether zur Fällung gebracht. Nach dem Absaugen des so erhaltenen Feststoffs wird dieser im Vakuum bei 50°C getrocknet.
Ausbeute: 29,51 g (99 % d. Th.) eines amorphen Feststoffes

| Elementaranalyse: | | | | | |
|---|---|---|---|---|---|
| ber.: | C 34,13 | H 3,46 | N 3,32 | S 3,80 | F 38,23 |
| gef.: | C 34,28 | H 3,81 | N 3,25 | S 3,80 | F 38.01 |

### c) Herstellung einer Formulierung aus Gadoliniumkomplex II und 1-O-α-D-[(1-Perfluoroctylsulfonyl-piperazin-4-carbonyl)-pentyl-5]-mannopyranose

Zu 47 ml einer Lösung des Gadoliniumkomplexes II (250 mmol/L) gelöst in 0,45 % aqu. Natriumchloridlösung) gibt man 9,92 g (11,75 mmol) der Titelverbindung aus Beispiel 2b und erwärmt für 10 Minuten in der Mikrowelle. Die Lösung wird auf Raumtemperatur abgekühlt, durch ein 0,2 µm-Filter filtriert und das Filtrat in vials abgefüllt. Eine so hergestellte Lösung kann direkt für biologische Experimente eingesetzt werden. (Die Konzentration beträgt 250 mmol Gd/L).

### Beispiel 3

### a) 2-Acetamido-2-deoxy-1,3,4,6-(tetra-O-benzyl)-α,β-D-glucopyranose

Zu einer gerührten Suspension von 20.16 g (700 mmol; 80 % ig in Mineralöl) Natriumhydrid in 150 ml Dimethylsulfoxid gibt man bei Raumtemperatur insgesamt 24,0 g (108,5 mmol) 2-Acetamido-2-deoxy-α,β-D-glucopyranose, gelöst in 500 ml absolutem Dimethylsulfoxid , tropfenweise hinzu. Anschließend läßt man noch 120 Minuten bei Raumtemperatur nachrühren und tropft dann 159,5 g (1,26 mol) Benzylchlorid hinzu. Die so erhaltene Reaktionslösung wird im Anschluß für weitere 12 Stunden bei Raumtemperatur gerührt. Zur Aufarbeitung wird die Reaktionslösung langsam in 1,5 Liter Eiswasser eingegossen und anschließend erschöpfend mit Diethylether extrahiert. Die vereingten Diethyletherphasen werden im Anschluß zweimal mit je 600 ml gesättigter Natriumhydrogencarbonatlösung sowie zweimal mit je 800 ml Wasser gewaschen. Nach dem Trocknen der organischen Phase über Natriumsulfat wird vom Salz abgesaugt und das Lösungsmittel im Vakuum abgezogen. Der verbleibende ölige Rückstand wird an Kieselgel unter Verwendung von Essigsäureethylester/Hexan (1: 5) als Eluent gereinigt.
Ausbeute: 48,68 g (73,6 % d. Th.) der oben genannten Titelverbindung in Form eines viskosen und farblosen Öls

| Elementaranalyse: | | | |
|---|---|---|---|
| ber.: | C 70,92 | H 6,45 | N 6,89 |
| gef.: | C 71,43 | H 6,44 | N 7,02 |

### b) 1-O-Benzyl-3,4,6-tri-O-benzyl-2-amino-2-deoxy-α,β-D-glucopyranose

30.0 g (49,2 mmol) der Titelverbindung aus Beispiel 3a) werden in einer Mischung aus 750 ml Methanol und 215 ml Wasser suspendiert und bei Raumtemperatur mit insgesamt 440 ml (49,2 mmol) einer 0,112 molaren wässrigen Perchlorsäurelösung tropfenweise versetzt. Nach beendeter Zugabe wird die Reaktionslösung noch 10 Minuten bei Raumtemperatur gerührt und die so erhaltene, nun homogene, Reaktionslösung wird im Anschluß im Vakuum bis zur Trockne eingeengt. Durch Versetzen des verbleibenden öligen Rückstandes mit einer Mischung aus gleichen Teilen Hexan und Dichlormethan wird dieser zur Kristallisation gebracht. Das kristalline Reaktionsprodukt wird abgesaugt, mit Hexan gewaschen und im Vakuum bei Raumtemperatur getrocknet.
Ausbeute: 27,08g (86 % d. Th.) an oben genannter Titelverbindung in Form ihres Perchlorates, welches als farblose, kristalline Verbindung vorliegt.
Schmelzpunkt : 180,5 - 181,5 °C

| Elementaranalyse: | | | | |
|---|---|---|---|---|
| ber.: | C 63,68 | H 5,98 | N 2,19 | Cl 5.54 |
| gef.: | C 63,43 | H 6,04 | N 2,02 | Cl 5.71 |

### c) 1,3,4,6-Tetra-O-benzyl-2-desoxy-2-[acetyl-(2-amino-N-ethyl-N-perfluoroctylsulfonyl)-amino]-1-α,β-D-glucopyranose

In 350 ml trockenem Tetrahydrofuran werden 20,8 g (35,6 mmol) der 2-[N-Ethyl-N-perfluoroctylsulfonyl)-aminoessigsäure sowie 3,60 g (35,6 mmol) Triethylamin gelöst. Nach dem Abkühlen der Reaktionslösung auf -15°C bis -20°C tropft man bei dieser Temperatur unter Rühren eine Lösung von 4,92 g (35,6 mmol) Chlorameisensäureisobutylester in 75 ml trockenem Tetrahydrofuran langsam hinzu, wobei die Zutropfgeschwindigkeit so zu wählen ist, daß eine Innentemperatur von -10°C nicht überschritten wird. Nach einer Reaktionszeit von 15 Minuten bei -15°C tropft man anschließend eine Lösung von 22,78 g (35,6 mmol) des Perchlorats (Titelverbindung aus Beispiel 3b) und 3,60g (35,6 mmol) Triethylamin, in 100 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 250 ml Essigsäureethylester aufgenommen und zweimal mit je 100 ml gesättigter Natriumhydrogencarbonatlösung sowie einmal mit 200 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester/Hexan (1:5) als Eluent gereinigt.
Ausbeute: 33,3 g (84,6% d. Th.) der oben genannten Titelverbindung als farbloses und stark viskoses Öl

| Elementaranalyse: | | | | | |
|---|---|---|---|---|---|
| ber.: | C 49,92 | H 3,92 | N 2,53 | F 29,18 | S 2,90 |
| gef.: | C 49,99 | H 4,11 | N 2,69 | F 29,22 | S 3,01 |

### d) 2-Desoxy-2-[acetyl-(2-amino-N-ethyl-N-perfluoroctylsulfonyl)-amino]-1-α,β-D-glucopyranose

20,0 g (18,06 mmol) der Titelverbindung aus Beispiel 3c) werden in 250 ml 2-Propanol gelöst und mit 1,5 g Palladium-Katalysator (10 % Pd/C) versetzt. Die Reaktionslösung wird für 12 Stunden bei 22°C und 1 Atmosphäre Wasserstoffdruck hydriert. Anschließend filtriert man vom Katalysator ab und engt das Filtrat zur Trockne ein. Der verbleibende Rückstand wird in 300 ml Dimethylsulfoxid aufgenommen und aus der so erhaltenen Produktlösung erhält man durch Versetzen mit 750 ml einer Mischung aus gleichen Teilen Diethylether und Essigsäureethylester nach dem Absaugen des ausgefallenen Feststoffes 12,65g (93,8 % d. Th.) der oben genannten Titelverbindung als farbloses und kristallines Pulver. Die oben genannte Titelverbindung liegt als α/β-Anomerengemisch vor, wobei das Verhältnis bezüglich der beiden möglichen Anomeren durch ¹H-NMR-spektroskopische Untersuchungen zu ca. 1:1,2 bestimmt wurde. Demnach handelt es sich bei der Titelverbindung um ein fast annähernd gleichverteiltes α/β-Anomerengemisch .
Schmelzpunkt: 132,5 - 133 °C.

| Elementaranalyse: | | | | | |
|---|---|---|---|---|---|
| ber.: | C 28,97 | H 2,57 | N 3,75 | F 43,27 | S 4,30 |
| gef.: | C 29,09 | H 2,56 | N 3,84 | F 43,36 | S 4,42 |

### e) Herstellung einer Formulierung aus Gadoliniumkomplex III und 2-Desoxy-2-[acetyl-(2-amino-N-ethyl-N-perfluoroctylsulfonyl)-amino]-1-α,β-D-glucopyranose

Zu 51 ml einer Lösung des Gadoliniumkomplexes III (300 mmol/L), gelöst in 0,45 %iger Natriumchloridlösung (pH 7,4 / 0,25 mg/L CaNa₃DTPA) gibt man eine Lösung aus 4,90 g (6,57 mmol) der Titelverbindung aus Beispiel 3d, gelöst in 200 ml Ethanol, zu und rührt 2 Stunden bei 50°C. Die Lösung wird im Vakuum zur Trockne eingeengt und der Rückstand mit dest. Wasser auf insgesamt 153 ml aufgefüllt. Man rührt 10 Minuten bei 40°C und filtriert über ein 0,2 µm Filter. Das Filtrat wird in vials abgefüllt. Eine so hergestellte Lösung kann direkt für biologische Experimente eingesetzt werden. (Die Konzentration beträgt 100 mmol Gd/L).

### Beispiel 4

### a) 1,2,3,4,6-Penta-O-acetyl-α-D-glucopyranose

Auf analoge Weise, wie zur Synthese der Titelverbindung 1a) beschrieben, liefert die Umsetzung von 100 g (555,0 mmol) α-D-Glucopyranose mit einem Gemisch aus 1000 ml absolutem Pyridin und 1000 ml Essigsäureanhydrid nach Aufarbeitung und Umkristallisation aus 95 %igem wässrigem Ethanol 190,6 g (88,0 %) der oben genannten Titelverbindung als farblose und kristalline Verbindung. Durch ¹H-NMR-spektroskopische Untersuchung der so erhaltenen Titelverbindung konnte das α zu β-Verhältnis von beiden möglichen Anomeren mit ≥ 98:2 bestimmt werden. Demnach handelt es sich bei der Titelverbindung um das ausschließlich α-konfigurierte Anomere.
Schmelzpunkt : 110,5 °C

| Elementaranalyse: | | |
|---|---|---|
| ber.: | C 49,21 | H 5,68 |
| gef.: | C 49,24 | H 5,68 |

### b) 5-(Ethoxycarbonyl)pentyl -2,3,4,6-tetra-O-acetyl-α-D-glucopyranosid

Auf analoge Weise, wie bei der Synthese der Titelverbindung aus Beispiel 1b) beschrieben, liefert die Umsetzung von 130,0 g (332,8 mmol) der Titelverbindung aus Beispiel 4a) mit 55.8 ml (332,8 mmol) 6-Hydroxy-hexansäureethylester und 50,6 ml (520 mmol) Zinn-IV-chlorid in 500 ml 1,2-Dichlorethan nach säulenchromatographischer Aufreinigung (Eluent : Hexan/Essigsäureethylester 2:1) 101,85 g (62,4 % d. Th.) der oben genannten Titelverbindung als farbloses und viskoses Öl. Nach ¹H-NMR-spektroskopischer Untersuchung der Titelverbindung konnte anhand der Größe der Kopplungskonstanten von J_{1,2} =8,8 Hz eindeutig auf das Vorliegen der β-Konfiguration am anomeren Zentrum geschlossen werden, welche zudem die einzig vorliegende Konfiguration am Anomeriezentrum darstellt. Somit konnte die oben genannte Titelverbindung nur in Form des β-konfigurierten Anomeren dargestellt werden.

| Elementaranalyse: | | |
|---|---|---|
| ber.: | C 52,94 | H 6,77 |
| gef.: | C 52,77 | H 6,70 |

### c) 5-(Carboxy)pentyl -2,3,4,6-tetra-O-benzyl-α-D-glucopyranosid

Eine gerührte Suspension von 100,0 g (204,96 mmol) der Titelverbindung aus Beispiel 4b) in 150 ml Dioxan wird bei Raumtemperatur und unter gleichzeitigem, kräftigen Rühren portionsweise mit insgesamt 169,14 g (3,02 mol) fein gepulvertem Kaliumhydroxydpulver versetzt . Zur Erhöhung der Rührfähigkeit wird das Reaktionsgemisch mit weiteren 150 ml Dioxan versetzt und die so erhaltene Suspension im Anschluß zur Siedehitze erhitzt und bei dieser Temperatur mit insgesamt 264 ml (2,218 mol) Benzylbromid über einen Zeitraum von zwei Stunden tropfenweise versetzt. Nach einer Reaktionszeit von 4 Stunden bei 110 °C gefolgt von 12 Stunden bei Raumtemperatur wird das Reaktionsgemisch zum Zwecke der Aufarbeitung in insgesamt 2,0 Liter Eiswasser langsam eingegossen und die Wasserphase im Anschluß vollständig mit Diethylether extrahiert. Nach dem Waschen der so erhaltenen Etherphase und dem anschließenden Trocknen der organischen Phase über Natriumsulfat wird vom Salz abgesaugt und der Diethylether im Vakuum abgezogen. Überschüssiges Benzylbromid wird anschließend im Ölpumpenvakuum quantitativ bei einer Ölbadtemperatur von 180°C aus dem Reaktionsgemisch abdestilliert. Der so erhaltene , verbleibende ölige Rückstand wird an Kieselgel unter Verwendung von Essigsäureethylester/Hexan (1:10) als Eluent gereinigt.
Ausbeute: 128,8 g (84,3 % d. Th.) der oben genannten Titelverbindung in Form eines farblosen und äußerst viskosen Öls

| Elementaranalyse: | | |
|---|---|---|
| ber.: | C 75,68 | H 7,16 |
| gef.: | C 75,66 | H 7,23 |

### d) 2,3,4,6-Tetra-O-benzyl-1-O-β-D-[6-hexansäure-N-(3-oxa-1H,1H,2H,2H,4H,4H, 5H,5H-perfluortridecyl)-amid]-glucopyranose

In 825 ml trockenem Tetrahydrofuran werden 68,5 g (91,79 mmol) der in Beispiel 4c) beschriebenen Säure sowie 9,25 g (91,79 mmol) Triethylamin gelöst. Nach dem Abkühlen der Reaktionslösung auf -15°C bis -20°C tropft man bei dieser Temperatur unter Rühren eine Lösung von 12,64 g (92,5 mmol) Chlorameisensäureisobutylester in 150 ml trockenem Tetrahydrofuran langsam hinzu, wobei die Zutropfgeschwindigkeit so zu wählen ist, daß eine Innentemperatur von -10°C nicht überschritten wird. Nach einer Reaktionszeit von 15 Minuten bei -15°C tropft man anschließend eine Lösung von 46.40 g (91,79 mmol) 1H, 1H, 2H,2H-heptadecafluoro-1-(2-aminoethyoxy)-decan und 9,25 g (91,79 mmol) Triethylamin , als Lösung in 200 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 250 ml Essigsäureethylester aufgenommen und zweimal mit je 300 ml gesättigter Natriumhydrogencarbonatlösung sowie einmal mit 400 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (10:5:1) als Eluent gereinigt.
Ausbeute: 104,7 g (92,4% d. Th.) der o. g. Titelverbindung als farbloses und stark viskoses Öl

| Elementaranalyse: | | | | |
|---|---|---|---|---|
| ber.: | C 57,38 | H 4,98 | N 1,13 | F 26,15 |
| gef.: | C 57,27 | H 5,09 | N 1,11 | F 26,08 |

### e) 1-O-β-D-[6-Hexansäure-N-(3-oxa-1H,1H,2H,2H,4H,4H,5H,5H-perfluortridecyl)-amid]-glucopyranose

40,0 g (32,38 mmol) der Titelverbindung aus Beispiel 4d) werden in 750 ml 2-Propanol gelöst und mit 2,0 g Palladium-Katalysator (10 % Pd/C) versetzt. Die Reaktionslösung wird für 12 Stunden bei 22°C und 1 Atmosphäre Wasserstoffdruck hydriert. Anschließend filtriert man vom Katalysator ab und engt das Filtrat zur Trockne ein. Der verbleibende Rückstand wird in 300 ml Dimethylsulfoxid aufgenommen und aus der so erhaltenen Produktlösung erhält man durch Versetzen mit insgesamt 1000ml Diethylether und nachfolgendem Absaugen des ausgefallenen Feststoffes 22,05 g (90,2 % d. Th.) der Titelverbindung als farbloses und kristallines Pulver mit einem Zersetzungsschmelzpunkt von 122-124 °C.

| Elementaranalyse : | | | | |
|---|---|---|---|---|
| ber.: | C 36,01 | H 5,92 | N 1,75 | F 40,34 |
| gef.: | C 36,07 | H 6,08 | N 1,76 | F 40,66 |

### f) Herstellung einer Formulierung aus Gadoliniumkomplex IV und 1-O-β-D-[6-Hexansäure-N-(3-oxa-1H,1H,2H,2H,4H,4H,5H,5H-perfluortridecyl)-amid]-glucopyranose

Zu 37 ml einer Lösung des Gadoliniumkomplexes IV (300 mmol/L) gelöst in 0,45 % aqu. Kochsalzlösung (pH 7,4; 0,25 mg/L CaNa₃DTPA) gibt man 20,29 g (25,9 mmol) der Titelverbindung aus Beispiel 4e und füllt mit 0,9 %iger aqu. Kochsalzlösung auf insgesamt 111 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 5

### a) 1-O-(1H,1H,2H,2H-Perfluorodecyl)-(2,3,4,6-tetra-O-acetyl)-α-D-mannopyranose

Die Umsetzung von 50 g (128,09 mmol) der Titelverbindung aus Beispiel 1a), welche als 4:1 Gemisch bezüglich der α, β-Anomeren eingesetzt wird, mit einer Lösung von 75,84 g (128,1 mmol) 1-Hydroxy-1H,1H,2H,2H-perfluorodecan in 150 ml 1,2-Dichlorethan sowie insgesamt 19,47 g (166,53 mmol) Zinn-IV-chlorid, in Analogie wie für die Synthesen der Titelverbindungen aus den Beispielen 1b) und 4b) beschrieben,führt nach Aufarbeitung und säulenchromatographischer Reinigung (Eluent: Hexan/Essigsäureethylester, 2:1) zur Bildung von 74,2 g (63.4 % d. Th.) der oben genannten Titelverbindung in Form eines viskosen und farblosen Öls. Nach ¹H-NMR-spektroskopischer Untersuchung der Titelverbindung , konnte anhand der Größe der Kopplungskonstanten von J_{1,2} = 1.3 Hz eindeutig auf das Vorliegen der α-Konfiguration am anomeren Zentrum geschlossen werden, welche zudem die ausschließlich vorliegende Konfiguration am Anomeriezentrum ist, so daß demnach die oben genannte Titelverbindung nur in Form des reinen α-konfigurierten Anomeren dargestellt werden konnte.

| Elementaranalyse: | | | |
|---|---|---|---|
| ber.: | C 44,65 | H 2,53 | F 35,32 |
| gef.: | C 44,77 | H 2,61 | F 35,09 |

### b) 1-O-(1H,1H,2H,2H-Perfluorodecyl)-α-D-mannopyranose

25 g (27,33 mmol) der Titelverbindung aus Beispiel 5a) werden 400 ml absolutem Methanol suspendiert und bei 5 °C mit einer katalytischen Menge Natriummethanolat versetzt. Nach einer Reaktionszeit von 3h bei Raumtemperatur zeigt die Dünnschichtchromatographische Kontrolle (Eluent: Chloroform / Methanol 9:1) des Reaktionsverlaufs bereits quantitative Umsetzung an. Zum Zwecke der Aufarbeitung wird die nun klare Reaktionslösung durch Versetzen mit Amberlite IR 120 (H⁺-Form)- Kationenaustauscherharz neutralisiert, vom Austauscher abgesaugt und das so erhaltene methanolische Filtrat im Vakuum bis zur Trockne abgezogen. Der erhaltene kristalline Rückstand wird durch zweimaliges Umkristallisieren aus Ethanol gereinigt. Nach ¹H-NMR-spektroskopischer Untersuchung der Titelverbindung, konnte anhand der Größe der Kopplungskonstanten von J_{1,2} = 1.0 Hz eindeutig auf das Vorliegen der α-Konfiguration am anomeren Zentrum geschlossen werden. Die vorliegende α-Konfiguration ist die am Anomeriezentrum ausschließlich vorliegende Konfiguration, , d.h. die Menge an möglicherweise gebildetem β-konfigurierten Anomeren der Titelverbindung liegt unterhalb der ¹H-NMR-spektroskopischen Nachweisgrenze. Die oben genannte Titelverbindung wurde demnach nur in Form des reinen α-konfigurierten Anomeren dargestellt.
Ausbeute: 16,2 g (94,6 % d. Th.) eines farblosen und kristallinen Feststoffes Schmelzpunkt: 172-174 °C unter Zersetzung.

| Elementaranalyse : | | | |
|---|---|---|---|
| ber.: | C 30,69 | H 2,41 | F 51,57 |
| gef.: | C 30,57 | H 2,48 | F 51,65 |

### c) Herstellung einer Formulierung aus Gadoliniumkomplex II und 1-O-(1H,1H,2H,2H-Perfluorodecyl)-α-D-mannopyranose

Zu 50 ml einer Lösung des Gadoliniumkomplexes II (150 mmol/L), gelöst in 0,45 %iger Natriumchloridlösung (pH 7,4 / 0,25 mg/L CaNa₃DTPA), gibt man eine Lösung aus 2,01 g (3,21 mmol) der Titelverbindung aus Beispiel 5b, gelöst in 200 ml Ethanol zu und rührt 2 Stunden bei 50°C. Die Lösung wird im Vakuum zur Trockne eingeengt und der Rückstand mit dest. Wasser auf insgesamt 75 ml aufgefüllt. Man rührt 10 Minuten bei 40°C und filtriert über ein 0,2 µm Filter. Das Filtrat wird in vials abgefüllt. Eine so hergestellte Lösung kann direkt für biologische Experimente eingesetzt werden. (Die Konzentration beträgt 100 mmol Gd/L).

### Beispiel 6

### a) 1-O-(1H,1H,2H,2H-Perfluorododecyl)-2,3,4,6-tetra-O-acetyl-α-D-mannopyranose

Die Umsetzung von 35 g (89,66 mmol) der Titelverbindung aus Beispiel 1a), welche als 4:1 Gemisch bezüglich der α, β-Anomeren eingesetzt wird, mit einer Lösung von 50,60 g (89,7 mmol) 1-Hydroxy-1H,1H,2H,2H-perfluorododecan in 100 ml 1,2-Dichlorethan sowie insgesamt 13,63 g (16,61 mmol) Zinn-IV-chlorid, in Analogie wie für die Synthesen der Titelverbindungen aus den Beispielen 1b), 4b) und 5b) beschrieben, führt nach Aufarbeitung und säulenchromatographischer Reinigung (Eluent: Hexan/Essigsäureethylester = 2:1) zur Bildung von 62.49 g (68.7 % d. Th.) der oben genannten Titelverbindung in Form eines viskosen und farblosen Öls. Nach ¹H-NMR-spektroskopischer Untersuchung der Titelverbindung , konnte anhand der Größe der Kopplungskonstanten von J_{1,2} = 1.4 Hz eindeutig auf das Vorliegen der α-Konfiguration am anomeren Zentrum geschlossen werden, welche zudem die ausschließlich vorliegende Konfiguration am Anomeriezentrum ist, so daß demnach die oben genannte Titelverbindung nur in Form des reinen α-konfigurierten Anomeren dargestellt werden konnte.

| Elementaranalyse: | | | |
|---|---|---|---|
| ber.: | C 42,62 | H 2,28 | F 39,32 |
| gef.: | C 42,55 | H 2,38 | F 39,40 |

### b) 1-O-(1H,1H,2H,2H-Perfluorododecyl)-α-D-mannopyranose

25 g (24,64 mmol) der Titelverbindung aus Beispiel 6a) werden 400 ml absolutem Methanol suspendiert und bei 5 °C mit einer katalytischen Menge Natriummethanolat versetzt. Nach einer Reaktionszeit von 3h bei Raumtemperatur zeigt die dünnschichtchromatographische Kontrolle (Eluent: Chloroform/Methanol=9:1) des Reaktionsverlaufs bereits quantitative Umsetzung an. Zum Zwecke der Aufarbeitung wird die nun klare Reaktionslösung durch Versetzen mit Amberlite IR 120 (H⁺-Form)-Kationenaustauscherharz neutralisiert, vom Austauscher abgesaugt und das so erhaltene methanolische Filtrat im Vakuum bis zur Trockne abgezogen. Der erhaltene kristalline Rückstand wird durch zweimaliges Umkristallisieren aus einem Gemisch aus 2-Propanol /Ethanol (1:1) gereinigt. Nach ¹H-NMR-spektroskopischer Untersuchung der Titelverbindung , konnte anhand der Größe der Kopplungskonstanten von J_{1,2} = 0.9 Hz eindeutig auf das Vorliegen der α-Konfiguration am anomeren Zentrum geschlossen werden. Die vorliegende α-Konfiguration ist die am Anomeriezentrum ausschließlich vorliegende Konfiguration, d.h. die Menge an möglicherweise gebildetem β-konfigurierten Anomeren der Titelverbindung liegt unterhalb der ¹H-NMR-spektroskopischen Nachweisgrenze. Die oben genannte Titelverbindung wurde demnach nur in Form des reinen α-konfigurierten Anomeren dargestellt. Ausbeute: 16,96 g (90,8% d. Th.) eines farblosen und kristallinen Feststoffes Schmelzpunkt : 187-188 °C unter Zersetzung.

| Elementaranalyse : | | | |
|---|---|---|---|
| ber.: | C 29,77 | H 2,08 | F 54,93 |
| gef.: | C 29,70 | H 2,28 | F 54,83 |

### c) Herstellung einer Formulierung aus Gadoliniumkomplex V und 1-O-(1H,1H,2H,2H-Perfluorododecyl)-α-D-mannopyranose

Zu 52 ml einer Lösung des Gadoliniumkomplexes V (180 mmol/L) gelöst in 0,45 % aqu. Natriumchloridlösung, gibt man 1,70 g (2,34 mmol) der Titelverbindung aus Beispiel 6b und erwärmt für 10 Minuten in der Mikrowelle. Die Lösung wird auf Raumtemperatur abgekühlt, durch ein 0,2 µm-Filter filtriert und das Filtrat in vials abgefüllt. Eine so hergestellte Lösung kann direkt für biologische Experimente eingesetzt werden. (Die Konzentration beträgt 180 mmol Gd/L)

### Beispiel 7

### a) 2,3,4,6-Tetra-O-acetyl)-1-O-α-D-[3,6,9-trioxa-(C₁₂-C₁₉-heptadecafluor)-nonadecyl]-mannopyranose

Die Umsetzung von 20 g (51,23 mmol) der Titelverbindung aus Beispiel 1a), welche als 4:1 Gemisch bezüglich der α, β-Anomeren eingesetzt wird, mit einer Lösung von 30,54 g (51,23 mmol) 1-Hydroxy-tris-(1H,1H,2H,2H-O)-1H,1H,2H,2H-perfluorodecan in 100 ml 1,2-Dichlorethan sowie insgesamt 5,98 g (51,23 mmol) Zinn-IV-chlorid, in Analogie wie für die Synthesen der Titelverbindungen aus den Beispielen 1b), 4b) und 5b) beschrieben, führt nach Aufarbeitung und säulenchromatographischer Reinigung (Eluent : Hexan/Essigsäureethylester= 1:1) zur Bildung von 34,22 g (72,1 % d. Th.) der oben genannten Titelverbindung in Form eines viskosen und farblosen Öls. Nach ¹H-NMR-spektroskopischer Untersuchung der Titelverbindung , konnte anhand der Größe der Kopplungskonstanten von J_{1,2} = 1.1 Hz eindeutig auf das Vorliegen der α-Konfiguration am anomeren Zentrum geschlossen werden, welche zudem die ausschließlich vorliegende Konfiguration am Anomeriezentrum ist, so daß demnach die oben genannte Titelverbindung nur in Form des reinen α-konfigurierten Anomeren dargestellt werden konnte.

| Elementaranalyse: | | | |
|---|---|---|---|
| ber.: | C 38,89 | H 3,81 | F 34,86 |
| gef.: | C 39,02 | H 3,77 | F 34,90 |

### b) 1-O-α-D-[3,6,9-Trioxa-(C₁₂-C₁₉-heptadecafluor)-nonadecyl]-mannopyranose

20 g (21,58 mmol) der Titelverbindung aus Beispiel 7a) werden 350 ml absolutem Methanol suspendiert und bei 5 °C mit einer katalytischen Menge Natriummethanolat versetzt. Nach einer Reaktionszeit von 3h bei Raumtemperatur zeigt die dünnschichtchromatographische Kontrolle (Eluent: Chloroform/Methanol = 6:1) des Reaktionsverlaufs bereits quantitative Umsetzung an. Zur Aufarbeitung wird die nun klare Reaktionslösung durch Versetzen mit Amberlite IR 120 (H⁺ - Form)-Kationenaustauscherharz neutralisiert, vom Austauscher abgesaugt und das so erhaltene methanolische Filtrat im Vakuum bis zur Trockne abgezogen. Der erhaltene kristalline Rückstand wird durch zweimaliges Umkristallisieren aus einem Gemisch aus Essigsäureethylester/2-Propanol/Ethanol (1:0.5:1) gereinigt. Nach ¹H-NMR-spektroskopischer Untersuchung der Titelverbindung konnte anhand der Größe der Kopplungskonstanten von J_{1,2} = 1.0 Hz eindeutig auf das Vorliegen der α-Konfiguration am anomeren Zentrum geschlossen werden. Die vorliegende α-Konfiguration ist die am Anomeriezentrum ausschließlich vorliegende Konfiguration, d.h. die Menge an möglicherweise gebildetem β-konfigurierten Anomeren der Titelverbindung liegt unterhalb der ¹H-NMR-spektroskopischen Nachweisgrenze. Die oben genannte Titelverbindung wurde demnach nur in Form des reinen α-konfigurierten Anomeren dargestellt.
Ausbeute: 15,20 g (92,9 % d. Th.) eines farblosen, kristallinen Feststoffes Schmelzpunkt: 141°C .

| Elementaranalyse : | | | |
|---|---|---|---|
| ber.: | C 34.84 | H 3.59 | F 42.58 |
| gef.: | C 34.72 | H 3.66 | F 42.67 |

### c) Herstellung einer Formulierung aus Gadoliniumkomplex VIII und 1-O-α-D-[3,6,9-Trioxa-(C₁₂-C₁₉-heptadecafluor)-nonadecyl]-mannopyranose

Zu 38 ml einer Lösung des Gadoliniumkomplexes VIII (300 mmol/L), gelöst in 0,45 % aqu. Kochsalzlösung (pH 7,4; 0,25 mg/L CaNa₃DTPA), gibt man 3,71 g (4,89 mmol) der Titelverbindung aus Beispiel 7b und führt mit 0,9 %iger aqu. Kochsalzlösung auf insgesamt 114 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 8

### a) 2,3,4,6-Tetra-O-acetyl-1-α-D-[3-thiopropionsäure-N-(3-oxa-1H,1H,2H,2H, 4H,4H,5H,5H-perfluortridecyl)-amid]-mannopyranose

In 500 ml trockenem Tetrahydrofuran werden 25,0 g (57,28 mmol); [Darstellung gemäß:
Ponpipom, Mitree M.; Bugianesi, Robert L.; Robbins, James C.; Doebber, T. W.; Shen, T. Y.; J.Med.Chem.; 24; 12; 1981; 1388-1395] 3-(Tetra-O-acetyl-α-D-mannopyranosylmercapto)- propionsäure sowie 5,77g (57,28 mmol) Triethylamin gelöst. Nach dem Abkühlen der Reaktionslösung auf -15°C bis -20°C tropft man bei dieser Temperatur unter Rühren eine Lösung von 7,82 g (57,28 mmol) Chlorameisensäureisobutylester in 100 ml trockenem Tetrahydrofuran langsam hinzu, wobei die Zutropfgeschwindigkeit so zu wählen ist, daß eine Innentemperatur von -10°C nicht überschritten wird. Nach einer Reaktionszeit von 15 Minuten bei -15°C tropft man im Anschluß eine Lösung von 29,05 g (57,28 mmol) 1H,1H, 2H,2H-heptadecafluoro-1-(2-aminoethyoxy)-decan und 5,77g (57,28 mmol) Triethylamin, als Lösung in 200 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 250 ml Essigsäureethylester aufgenommen und zweimal mit je 200 ml gesättigter Natriumhydrogencarbonatlösung sowie einmal mit 300 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (8:5:1) als Eluent gereinigt.
Ausbeute: 44,90 g (84,7% d. Th.) der oben genannten Titelverbindung als farbloses und stark viskoses Öl.

| Elementaranalyse: | | | | | |
|---|---|---|---|---|---|
| ber.: | C 37,63 | H 3,48 | N 1,51 | S 3,46 | F 34,89 |
| gef.: | C 37,77 | H 3,37 | N 1,61 | S 3,57 | F 35,21 |

### b) 1-α-D-[3-thiopropionsäure-N-(3-oxa-1H,1H,2H,2H,4H,4H,5H,5H-perfluonridecyl)-amid]-mannopyranose

30 g (32,41 mmol) der Titelverbindung aus Beispiel 8a) werden 400 ml absolutem Methanol suspendiert und bei 5 °C mit einer katalytischen Menge Natriummethanolat versetzt. Nach einer Reaktionszeit von 3h bei Raumtemperatur zeigt die dünnschichtchromatographische Kontrolle (Eluent: Chloroform/Methanol = 9:1) des Reaktionsverlaufs bereits quantitative Umsetzung an. Zur Aufarbeitung wird die nun klare Reaktionslösung durch Versetzen mit Amberlite IR 120 (H⁺-Form)-Kationenaustauscherharz neutralisiert, vom Austauscher abgesaugt und das so erhaltene methanolische Filtrat im Vakuum bis zur Trockne abgezogen. Der erhaltene kristalline Rückstand wird durch Umkristallisieren aus einem Gemisch aus Essigsäureethylester/Methanol (0.5:1) gereinigt. Nach ¹H-NMR-spektroskopischer Untersuchung der Titelverbindung , konnte anhand der Größe der Kopplungskonstanten von J_{1,2} = 1.1 Hz eindeutig auf das Vorliegen der α-Konfiguration am anomeren Zentrum geschlossen werden. Die vorliegende α-Konfiguration ist die am Anomeriezentrum ausschließlich vorliegende Konfiguration, d.h. die Menge an möglicherweise gebildetem β-konfigurierten Anomeren der Titelverbindung liegt unterhalb der ¹H-NMR-spektroskopischen Nachweisgrenze. Die oben genannte Titelverbindung wurde demnach nur in Form des reinen α-konfigurierten Anomeren dargestellt.
Ausbeute: 23,76 g (96,8 % d. Th.) eines farblosen und kristallinen Feststoffes Schmelzpunkt: 113 - 114.5 °C

| Elementaranalyse : | | | | | |
|---|---|---|---|---|---|
| ber.: | C 33,30 | H 3,19 | N 1,85 | S 4,23 | F 42,64 |
| gef.: | C 33,21 | H 3,26 | N 1,96 | S 4,08 | F 42,77 |

### c) Herstellung einer Formulierung aus Gadoliniumkomplex VI und 1-α-D-[3-thiopropionsäure-N-(3-oxa-1H,1H,2H,2H,4H,4H,5H,5H-perfluortridecyl)-amid]-mannopyranose

Zu 47 ml einer Lösung des Gadoliniumkomplexes VI (330 mmol/L), gelöst in 0,45 %iger Natriumchloridlösung (pH 7,4 / 0,25 mg/L CaNa₃DTPA), gibt man eine Lösung aus 27,41 g (36,19 mmol) der Titelverbindung aus Beispiel 8b, gelöst in 200 ml Ethanol, zu und rührt 2 Stunden bei 50°C. Die Lösung wird im Vakuum zur Trockne eingeengt und der Rückstand mit dest. Wasser auf insgesamt 155 ml aufgefüllt. Man rührt 10 Minuten bei 40°C und filtriert über ein 0,2 µm Filter. Das Filtrat wird in vials abgefüllt. Eine so hergestellte Lösung kann direkt für biologische Experimente eingesetzt werden. (Die Konzentration beträgt 100 mmol Gd/L).

### Beispiel 9

### a) 2,3,4,6-Tetra-O-acetyl-1-β-D-[3,6,9-trioxa-(C₁₂-C₁₉-heptadecafluor)-nonadecyl]-glucopyranosyluronsäure

Zu einer gerührten Lösung von 20,2 g (50,85 mmol) Methyl (1-bromo-2,3,4-tri-O-acety-α-D-glucopyranosid)uronat [Darstellung gemäß: Pelzer; Hoppe-Seyler's Z. Physiol.Chem.; 314; 1949; 234, 237 sowie Goebel; Babers; J.Biol.Chem.; 111; 1935; 347, 350 und Bollenback et al.; J.Amer.Chem.Soc.; 77; 1955; 3310, 3313.] und 60,64 g (101,7 mmol) 3,6,9-Trioxa-(C₁₂-C₁₉-heptadecafluor)-nonadecan-1-ol werden in 250 ml wasserfreiem Acetonitril gelöst und bei Raumtemperatur mit 13,0 g frisch gefälltem Silberoxid versetzt. Nach einer Reaktionszeit von 12 Stunden bei Raumtemperatur wird von den unlöslichen Silbersalzen abfiltriert, die Salze gut mit Dichlormethan nachgewaschen und das so erhaltene Filtrat im Vakuum bis zur Trockne abgezogen. Der verbleibende Rückstand wird durch Säulenchromatographie gereinigt (Eluent: Hexan/Essigsäureethylester= 3:1).
Ausbeute: 22,99 g (53,3 % d. Th.) der oben genannter Titelverbindung als farbloses, hoch viskoses Öl

| Elementaranalyse: | | | |
|---|---|---|---|
| ber.: | C 41,05 | H 3,92 | F 38,06 |
| gef.: | C 41,20 | H 3,76 | F 38,22 |

### b) 1-O-β-D-[3,6,9-trioxa-(C₁₂-C₁₉-heptadecafluor)-nonadecyl]-glucopyranosyluronsäure

10,0 g (11,78 mmol) der Titelverbindung aus Beispiel 9a) werden in 200 ml eines Gemisches, bestehend aus Methanol 0,5 molarer Natronlauge im Verhältnis von 2:1 unter rühren bei Taumtemperatur suspendiert . Nach einer Reaktionszeit von 12 h bei Raumtemperatur wird das nun klare Reaktionsgemisch zur Aufarbeitung durch Versetzen mit Amberlite IR 120 (H⁺-Form)-Kationenaustauscherharz neutralisiert, vom Austauscher abgesaugt und das so erhaltene methanolisch-wässrige Filtrat im Vakuum bis zur Trockne abgezogen. Der erhaltene kristalline Rückstand wird durch Umkristallisieren aus einem Gemisch aus Essigsäureethylester/Methanol (0,25:1) gereinigt. Nach ¹H-NMR-spektroskopischer Untersuchung der Titelverbindung, konnte anhand der Größe der Kopplungskonstanten von J_{1,2} = 9,2 Hz eindeutig auf das Vorliegen der β-Konfiguration am anomeren Zentrum geschlossen werden. Die vorliegende β-Konfiguration ist die am Anomeriezentrum ausschließlich vorliegende Konfiguration, d.h. die Menge an möglicherweise gebildetem β-konfigurierten Anomeren der Titelverbindung liegt unterhalb der ¹H-NMR-spektroskopischen Nachweisgrenze. Die oben genannte Titelverbindung wurde demnach nur in Form des reinen β-konfigurierten Anomeren dargestellt.
Schmelzpunkt: 78,5 °C

| Elementaranalyse: | | | |
|---|---|---|---|
| ber.: | C 34,21 | H 3,26 | F 41,81 |
| gef.: | C 34,38 | H 3,26 | F 41,90 |

### c) Herstellung einer Formulierung aus Gadoliniumkomplex I und 1-O-β-D-[3,6,9-trioxa-(C₁₂-C₁₉-heptadecafluor)-nonadecyl]-glucopyranosyluronsäure

Zu 38 ml einer Lösung des Gadoliniumkomplexes (280 mmol/L), gelöst in 0,45 % aqu. Kochsalzlösung (pH 7,4; 0,25 mg/L CaNa₃DTPA), gibt man 19,18 g (24,83 mmol) der Titelverbindung aus Beispiel 9b und füllt mit 0,9 %iger aqu. Kochsalzlösung auf insgesamt 53,2 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 200 mmol Gd/L).

### Beispiel 10

### a) 6-(2-Oxa-1H,1H,3H,3H,4H,4H-perfluordecyl)-O¹,O²,O³,O⁴-diisopropyliden-α-D-galactopyranose

Zu einer gerührten Suspension von 2,01 g (70,0 mmol; 80 % ig in Mineralöl) Natriumhydrid in 25 ml Dimethylformamid gibt man bei Raumtemperatur insgesamt 12,15 g (46,66 mmol) O¹,O²,O³,O⁴-diisopropyliden-α-Galactopyranose [Darstellung gemäß: Levene; Meyer; J.Biol.Chem.; 64; 1925; 473 sowie McCreath; Smith; J.Chem.Soc.; 1939; 387, 389 und Freudenberg; Hixon; Chem.Ber.; 56; 1923; 2119, 2122] gelöst in 200 ml absolutem Dimethylformamid, tropfenweise hinzu. Anschließend läßt man noch 120 Minuten bei Raumtemperatur nachrühren und tropft im Anschluß dann insgesamt 30,09 g (48,0 mmol) 1-Brom-1H,1H,2H,2H-perfluorododecan, gelöst in 150 ml absolutem Dimethylformamid langsam hinzu. Die so erhaltene Reaktionslösung wird im Anschluß für weitere 12 Stunden bei Raumtemperatur gerührt. Zur Aufarbeitung wird die Reaktionslösung langsam in 1 Liter Eiswasser eingegossen und anschließend erschöpfend mit Diethylether extrahiert. Die vereingten organischen Phasen werden im Anschluß zweimal mit je 200 ml gesättigter Natriumhydrogencarbonatlösung sowie zweimal mit je 200 ml Wasser gewaschen. Nach dem Trocknen der organischen Phase über Natriumsulfat wird vom Salz abgesaugt und das Lösungsmittel im Vakuum abgezogen. Der verbleibende ölige Rückstand wird an Kieselgel unter Verwendung von Essigsäureethylester/Hexan (1:10) als Eluent gereinigt.
Ausbeute: 29,8 g (79,3 % d. Th.) der oben genannten Titelverbindung in Form eines viskosen, farblosen Öls

| Elementaranalyse: | | | |
|---|---|---|---|
| ber.: | C 35,75 | H 2,87 | F 49,47 |
| gef.: | C 35,64 | H 2,98 | F 49,54 |

### b) 6-(2-Oxa-1H,1H,3H,3H,4H,4H-perfluordecyl)-α-D-galactopyranose

20 g (24,8 mmol) der Titelverbindung aus Beispiel 10a) werden mit 300 ml einer 1 %igen wässrigen Schwefelsäurelösung versetzt und für 3 Stunden bei 80 °C gerührt. Nach dem Abkühlen auf Raumtemperatur wird durch Versetzen mit wässriger Bariumhydroxydlösung neutralisiert und im Anschluß vom ausgefallenen Bariumsulfat abfiltriert und die so erhaltene klare wässrige Produktlösung gefriergetrocknet. Durch ¹H-NMR-spektroskopischer Untersuchung der Titelverbindung, konnte eindeutig das Vorliegen beider möglicher Konfigurationen am anomeren Zentrum gezeigt werden, wobei das vorliegende α/β-Konfigurationsverhältnis nach ¹H-NMR-spektroskopischer Untersuchung mit 1:1,4 (α:β) am Anomeriezentrum bestimmt wurde. Die oben genannte Titelverbindung wurde demnach nur in Form des 1:1,4 (α:β)-Anomerengemisches isoliert, d.h. auf eine Anomerentrennung wurde verzichtet. Ausbeute: 15,28 g (98,4 % d. Th.) der o. g. Titelverbindung als farbloses Lyophilisat

| Elementaranalyse (bezogen auf wasserfreie Substanz): | | | |
|---|---|---|---|
| ber.: | C 35,75 | H 2,87 | F 49,47 |
| gef.: | C 35,64 | H 2,98 | F 49,54 |

### c) Herstellung einer Formulierung aus Gadoliniumkomplex VII und 6-(2-Oxa-1H,1H, 3H,3H,4H,4H-perfluordecyl)-α-D-galactopyranose

Zu 43 ml einer Lösung des Gadoliniumkomplexes VII (250 mmol/L), gelöst in 0,45 %iger Natriumchloridlösung (pH 7,4 / 0,25 mg/L CaNa₃DTPA), gibt man eine Lösung aus 1,68 g (2,69 mmol) der Titelverbindung aus Beispiel 10b, gelöst in 200 ml Ethanol, zu und rührt 2 Stunden bei 50°C. Die Lösung wird im Vakuum zur Trockne eingeengt und der Rückstand mit dest. Wasser auf insgesamt 107,5 ml aufgefüllt. Man rührt 10 Minuten bei 40°C und filtriert über ein 0,2 µm Filter. Das Filtrat wird in vials abgefüllt. Eine so hergestellte Lösung kann direkt für biologische Experimente eingesetzt werden. (Die Konzentration beträgt 100 mmol Gd/L).

### Beispiel 11

### a) 1-0-□-D-[(1-perfluoroctylsulfonylpiperazin-4-carbonyl-)-methyl]-mannopyranose

30 g (52,8 mmol) 1-Perfluoroctylsulfonylpiperazin (Herstellung beschrieben in DE 196 03 033) und 31,73 g (53 mmol) 2,3,4,6-Tetra-O-benzyl-α-D-carboxymethylmannopyranose (Herstellung beschrieben in DE 197 28 954) werden in 300 ml Tetrahydrofuran gelöst. Bei 0°Cgibt man 24,73 g (100 mmol) EEDQ (=1,2-Dihydro-2-ethoxychinolin-1-carbonsäureethylester) zu und rührt 3 Stunden bei 0°C, anschließend 6 Stunden bei Raumtemperatur. Die Lösung wird im Vakuum zur Trockne eingedampft und der Rückstand durch Flash-Chromatographie an Kieselgel gereinigt (Laufmittel: Hexan/ Essigester= 10:1). Die produkthaltigen Fraktionen werden zur Trockne eingedampft, der Rückstand in einer Mischung aus 200 ml Methanol/150 ml Dichlormethan gelöst und 8 Stunden über Palladium/Kohle (10 % Pd/C 2 g) hydriert. Man filtriert vom Hydrierungskatalysator ab und dampft das Filtrat zur Trockne ein. Der Rückstand wird aus Aceton/ Diethylether umkristallisiert. Ausbeute: 30,39 g (73 % d. Th.) eines wachsartigen farblosen Feststoffes

| Elementaranalyse: | | | | | |
|---|---|---|---|---|---|
| ber.: | C 30,47 | H 2,68 | F 40,96 | N 3,55 | S 4,07 |
| gef.: | C 30,61 | H 2,75 | F 41,10 | N 3,46 | S 4,12 |

### b) Herstellung einer Formulierung aus Gadoliniumkomplex I und 1-0-□-D-[(1-perfluoroctylsulfonylpiperazin-4-carbonyl-)-methyl]-mannopyranose

Zu 32 ml einer Lösung des Gadoliniumkomplexes I (280 mmol/L), gelöst in 0,45 % aqu. Kochsalzlösung (pH 7,4; 0,25 mg/L CaNa₃DTPA), gibt man 4,71 g (5,97 mmol) der Titelverbindung aus Beispiel 11a und füllt mit 0,9 %iger aqu. Kochsalzlösung auf insgesamt 55 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 200 mmol Gd/L).

### Beispiel 12

### a) 3-Oxa-2H,2H,4H,4H,5H,5H-perfluortridecansäure, Natriumsalz

20 g (38,3 mmol) 3-Oxa-2H,2H,4H,4H,5H,5H-perfluortridecansäure (Herstellung beschrieben in DE 196 03 033) werden in 300 ml Ethanol gelöst und 7,7 ml 5 N aqu. Natronlauge zugegeben. Man dampft zur Trockne ein und trocknet den Rückstand im Vakuum-Trockenschrank (8 Stunden 60°C).
Ausbeute: 20,85 g (quantitativ) eines farblosen, kristallinen Pulvers

| Elementaranalyse: | | | | |
|---|---|---|---|---|
| ber.: | C 26,49 | H 1,11 | F 59,35 | Na 4,22 |
| gef.: | C 26,60 | H 1,19 | F 59,47 | Na 4,30 |

### b) Herstellung einer Formulierung aus Gadoliniumkomplex I und 3-Oxa-2H,2H,4H,4H, 5H,5H-perfluortridecansäure, Natriumsalz

Zu 32 ml einer Lösung des Gadoliniumkomplexes I (280 mmol/L), gelöst in 0,45 % aqu. Kochsalzlösung (pH 7,4; 0,25 mg/L CaNa₃DTPA), gibt man 2,09 g (3,84 mmol) der Titelverbindung aus Beispiel 12a und führt mit 0,9 %iger aqu. Kochsalzlösung auf insgesamt 90 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### c) Herstellung einer Formulierung aus Gadoliniumkomplex I und 3-Oxa-2H,2H,4H,4H, 5H,5H-perfluortridecansäure, Natriumsalz

Zu 32 ml einer Lösung des Gadoliniumkomplexes I (280 mmol/L) gelöst in 0,45 % aqu. Kochsalzlösung (pH 7,4; 0,25 mg/L CaNa₃DTPA) gibt man 1,00 g (1,84 mmol) der Titelverbindung aus Beispiel 12a und füllt mit 0,9 %iger aqu. Kochsalzlösung auf insgesamt 90 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### d) Herstellung einer Formulierung aus Gadoliniumkomplex I und 3-Oxa-2H,2H,4H,4H, 5H,5H-perfluortridecansäure, Natriumsalz

Zu 32 ml einer Lösung des Gadoliniumkomplexes I (280 mmol/L), gelöst in 0,45 % aqu. Kochsalzlösung (pH 7,4; 0,25 mg/L CaNa₃DTPA), gibt man 0,54 g (1,0 mmol) der Titelverbindung aus Beispiel 12a und füllt mit 0,9 %iger aqu. Kochsalzlösung auf insgesamt 90 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 13

### a) 1-Perfluoroctylsulfonyl-4-(3,6,9,12,15-pentaoxahexadecanoyl)-piperazin

20 g (35,2 mmol) Perfluoroctylsulfonylpiperazin (siehe Beispiel 11a) werden in 300 ml Dichlormethan gelöst und 5,06 g (50 mmol) Triethylamin zugegeben. Man kühlt auf 0°C und tropft innerhalb von 20 Minuten 14,24 g (50 mmol) 3,6,9,12,15-Pentaoxahexansäurechlorid zu und rührt 3 Stunden bei 0°C. Man setzt 400 ml 5 %ige aqu. Salzsäure zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel= Dichlormethan/Methanol: 15:1). Ausbeute: 26,44 (92 % d. Th.) eines wachsartigen Feststoffes

| Elementaranalyse: | | | | | |
|---|---|---|---|---|---|
| ber.: | C 33,83 | H 3,58 | N 3,43 | F 39,55 | S 3,93 |
| gef.: | C 33,96 | H 3,66 | N 3,50 | F 39,67 | S 3,82 |

### b) Herstellung einer Formulierung aus Gadoliniumkomplex I und 1-Perfluoroctylsulfonyl-4-(3,6,9,12,15-pentaoxahexadecanoyl)-piperazin

Zu 47 ml einer Lösung des Gadoliniumkomplexes I (280 mmol/L), gelöst in 0,45 % aqu. Kochsalzlösung (pH 7,4; 0,25 mg/L CaNa₃DTPA), gibt man 4,61 g (5,64 mmol) der Titelverbindung aus Beispiel 13a und führt mit 0,9 %iger aqu. Kochsalzlösung auf insgesamt 66 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 200 mmol Gd/L).

### Beispiel 14

### a) 1H,1H,2H,2H-Perfluordecyl-p-toluolsulfonsäureester

20 g (43,1 mmol) 1H,1H,2H,2H-Perfluordecanol werden in 200 ml Pyridin gelöst und bei 0°C portionsweise 9,53 g (50 mmol) p-Toluolsulfonsäurechlorid zugegeben. Man rührt 5 Stunden bei Raumtemperatur. Die Lösung wird in 1000 ml Eiswasser gegossen und 10 Minuten gerührt. Der Niederschlag wird abfiltriert, mit viel Wasser gewaschen und anschließend aus Aceton umkristallisiert.
Ausbeute: 22,04 g (97 % d. Th.) eines farblosen kristallinen Feststoffes

| Elementaranalyse: | | | | |
|---|---|---|---|---|
| ber.: | C 22,78 | H 0,76 | F 61,26 | S 6,08 |
| gef.: | C 22,89 | H 0,70 | F 61,39 | S 6,15 |

### b) C₁₈-C₂₅-Heptadeca-fluor-3,6,9,12,15-pentaoxa-pentacosan-1-ol

20 g (37,94 mmol) der Titelverbindung aus Beispiel 14a, 35,74 g (150 mmol) Pentaethylenglycol und 1 g 18-Krone-6 werden in 300 ml Tetrahydrofuran gelöst und 10,1 g (180 mmol) feingepulvertes Kaliumhydroxid zugegeben. Man rührt 10 Stunden bei Raumtemperatur. Man filtriert vom Feststoff ab und engt das Filtrat im Vakuum zur Trockne ein. Der Rückstand wird an Kieselgel chromatographiert (Laufmittel: Dichlormethan/ Methanol= 15:1).
Ausbeute: 5,45 g (21 % d. Th.) eines farblosen, viskosen Öls

| Elementaranalyse: | | | |
|---|---|---|---|
| ber.: | C 35,10 | H 3,68 | F 47,19 |
| gef.: | C 35,22 | H 3,77 | F 47,10 |

### c) Herstellung einer Formulierung aus Gadoliniumkomplex IX und C₁₈-C₂₅-Heptadeca-fluor-3,6,9,12,15-pentaoxa-pentacosan-1-ol

Zu 53 ml einer Lösung des Gadoliniumkomplexes IX (310 mmol/L), gelöst in 0,45 % aqu. Natriumchloridlösung), gibt man 44,98 g (65,72 mmol) der Titelverbindung aus Beispiel 14b und erwärmt für 10 Minuten in der Mikrowelle. Die Lösung wird auf Raumtemperatur abgekühlt, durch ein 0,2 µm-Filter filtriert und das Filtrat in vials abgefüllt. Eine so hergestellte Lösung kann direkt für biologische Experimente eingesetzt werden. (Die Konzentration beträgt 310 mmol Gd/L).

### Beispiel 15

### a) N,N-Bis(8-hydroxy-3,6,-dioxa-octyl)-perfluoroctylsulfonsäureamid

15 g (29,23 mmol) Perfluoroctylsulfonsäureamid und 22,16 g (87,7 ml) 9-(Tetrahydropyran-2-yl)-3,6,9-trioxa-nonylchlorid werden in 200 ml Acetonitril gelöst. Man gibt 41,46 g (300 mmol) Kaliumcarbonat und 1 g (6 mmol) Kaliumjodid zu und kocht 10 Stunden unter Rückfluß. Der Feststoff wird abfiltriert und das Filtrat im Vakuum zur Trockne eingedampft. Der Rückstand wird in 400 ml Ethanol gelöst und 30 ml 10 % aqu. Salzsäure zugegeben. Man rührt 2 Stunden bei Raumtemperatur. Es wird mit Natronlauge auf pH 7 gestellt und die Lösung im Vakuum eingeengt. Der Rückstand wird an Kieselgel chromatographiert (Laufmittel: Dichlormethan/Methanol= 10:1). Ausbeute: 11,38 g (51 % d. Th.) eines farblosen, viskosen Öls

| Elementaranalyse: | | | | | |
|---|---|---|---|---|---|
| ber.: | C 31,46 | H 3,43 | N 1,83 | F 42,30 | S 4,20 |
| gef.: | C 31,59 | H 3,50 | N 1,90 | F 42,46 | S 4,08 |

### b) Herstellung einer Formulierung aus Gadoliniumkomplex I und N,N-Bis(8-hydroxy-3,6,-dioxa-octyl)-perfluoroctylsulfonsäureamid

Zu 37 ml einer Lösung des Gadoliniumkomplexes I (280 mmol/L), gelöst in 0,45 % aqu. Kochsalzlösung (pH 7,4; 0,25 mg/L CaNa₃DTPA), gibt man 7,91 g (10,36 mmol) der Titelverbindung aus Beispiel 15a und füllt mit 0,9 %iger aqu. Kochsalzlösung auf insgesamt 104 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 16

### a) N,N-Bis(t-butyloxycarbonylmethyl)-perfluoroctylsulfonsäureamid

20 g (38,97 mmol) Perfluoroctylsulfonsäureamid und 20,73 g (150 mol) Kaliumcarbonat werden in 200 ml Aceton suspendiert und 17,56 g (90 mmol) Bromessigsäure-tert.-butylester zugegeben. Man kocht 3 Stunden unter Rückfluß. Der Feststoff wird abfiltriert und das Filtrat im Vakuum zur Trockne eingedampft. Der Rückstand wird an Kieselgel chromatographiert (Laufmittel: n-Hexan/Essigester= 10:1).
Ausbeute: 23,53 g (83 % d. Th.) eines farblosen, wachsartigen Feststoffs

| Elementaranalyse: | | | | | |
|---|---|---|---|---|---|
| ber.: | C 33,02 | H 3,05 | F 44,40 | N 1,93 | S 4,41 |
| gef.: | C 33,19 | H 3,11 | F 44,30 | N 1,99 | S 4,32 |

### b) N,N-Bis(carboxymethyl)-perfluoroctylsulfonsäureamid, Dinatriumsalz

23g (31,62 mmol) der Titelverbindung aus Beispiel 16a werden in 300 ml Trifluoressigsäure gelöst und 5 Stunden bei Raumtemperatur gerührt. Man dampft im Vakuum zur Trockne ein und kristallisiert den Rückstand aus Aceton um. Die Kristalle werden im Vakuum (bei 50°C getrocknet/Stunden).
Ausbeute: 17,7 g (91 % d. Th.) eines farblosen, kristallinen Pulvers
17 g (27,63 mmol) der so erhaltenen Di-Säure werden in 100 ml Wasser/300 ml Ethanol gelöst und 9,2 ml 3 N aqu. Natronlauge zugegeben. Man rührt 20 Minuten bei Raumtemperatur und dampft anschließend im Vakuum zur Trockne ein. Der Rückstand wird im Vakuum (60°C / 8 Stunden) getrocknet.
Ausbeute: 18,2 g farbloses, kristallines Pulver

| Elementaranalyse: | | | | | | |
|---|---|---|---|---|---|---|
| ber.: | C 21,87 | H 0,61 | N 2,12 | F 49,00 | S 4,86 | Na 6,98 |
| gef.: | C 22,00 | H 0,70 | N 2,20 | F 49,17 | S 4,93 | Na 7,10 |

### c) Herstellung einer Formulierung aus Gadoliniumkomplex II und N,N-Bis(carboxymethyl)-perfluoroctylsulfonsäureamid, Dinatriumsalz

Zu 41 ml einer Lösung des Gadoliniumkomplexes II (250 mmol/L), gelöst in 0,45 % aqu. Kochsalzlösung (pH 7,4; 0,25 mg/L CaNa₃DTPA), gibt man 2,89 g (4,39 mmol) der Titelverbindung aus Beispiel 16b und füllt mit 0,9 %iger aqu. Kochsalzlösung auf insgesamt 52 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 200 mmol Gd/L).

### Beispiel 17

### a) 1H,1H,2H,2H-Perfluordodecyl-schwefelsäuremonoester, Natriumsalz

10 g (17,73 mmol) 1H,1H,2H,2H-Perfluordodecanol werden in 300 ml Chloroform gelöst und bei 0°C 2,82 g (17,73 mmol) Schwefeltrioxid-Pyridin-Komplex zugegeben. Man rührt eine Stunde bei 0°C und dampft anschließend im Vakuum zur Trockne ein. Der Rückstand wird in 300 ml Ethanol gelöst und mit 17,8 ml 1N aqu. Natronlauge versetzt. Die Lösung wird zur Trockne eingedampft und der Rückstand im Vakuum getrocknet (60°C / 2 h).
Ausbeute: 11,81 g (quantitativ)

| Elementaranalyse: | | | | | |
|---|---|---|---|---|---|
| ber.: | C 21,64 | H 0,61 | F 59,89 | Na 3,45 | S 4,81 |
| gef.: | C 21,70 | H 0,72 | F 60,00 | Na 3,57 | S 4,92 |

### b) Herstellung einer Formulierung aus Gadoliniumkomplex V und 1H,1H,2H,2H-Perfluordodecyl-schwefelsäuremonoester, Natriumsalz

Zu 38 ml einer Lösung des Gadoliniumkomplexes V (290 mmol/L), gelöst in 0,45 % aqu. Natriumchloridlösung), gibt man 4,90 g (7,35 mmol) der Titelverbindung aus Beispiel 17a und erwärmt für 10 Minuten in der Mikrowelle. Die Lösung wird auf Raumtemperatur abgekühlt, durch ein 0,2 µm-Filter filtriert und das Filtrat in vials abgefüllt. Eine so hergestellt Lösung kann direkt für biologische Experimente eingesetzt werden. (Die Konzentration beträgt 290 mmol Gd/L).

### Beispiel 18

### a) 2H,2H,4H,4H,5H,5H-3-Oxa-perfluorpentadecansäure, Natriumsalz

10 g (16,07 mmol) 2H,2H,4H,4H,5H,5H-3-Oxa-perfluorpentadecansäure werden in 300 ml Ethanol gelöst und mit 16,1 ml 1N aqu. Natronlauge versetzt. Die Lösung wird zur Trockne eingedampft und der Rückstand im Vakuum getrocknet (60°C/2h). Ausbeute: 10,35 g (quantitativ) eines farblosen amorphen Pulvers

| Elementaranalyse: | | | | |
|---|---|---|---|---|
| ber.: | C 26,10 | H 0,94 | F 61,94 | Na 3,57 |
| gef.: | C 26,22 | H 1,00 | F 62,05 | Na 3,66 |

### b) Herstellung einer Formulierung aus Gadoliniumkomplex VI und 2H,2H,4H,4H,5H,5H-3-Oxa-perfluorpentadecansäure, Natriumsalz

Zu 45 ml einer Lösung des Gadoliniumkomplexes VI (270 mmol/L), gelöst in 0,45 %iger Natriumchloridlösung (pH 7,4 / 0,25 mg/L CaNa₃DTPA), gibt man eine Lösung aus 3,36 g (5,21 mmol) der Titelverbindung aus Beispiel 18a, gelöst in 200 ml Ethanol, zu und rührt 2 Stunden bei 50°C. Die Lösung wird im Vakuum zur Trockne eingeengt und der Rückstand mit dest. Wasser auf insgesamt 122 ml aufgefüllt. Man rührt 10 Minuten bei 40°C und filtriert über ein 0,2 µm Filter. Das Filtrat wird in vials abgefüllt. Eine so hergestellte Lösung kann direkt für biologische Experimente eingesetzt werden. (Die Konzentration beträgt 100 mmol Gd/L).

### Beispiel 19

### a) Ethylendiamin-N,N-tetraessigsäure-N-(1H,1H,2H,2H,4H,4H,5H,5H-3-oxaperfluortridecyl)-monoamid

Zu 30 g (117,1 mmol) EDTA-Bisanhydrid suspendiert in 200 ml Dimethylformamid und 50 ml Pyridin gibt man bei 50°C portionsweise 10,14 g (20 mmol) 1H,1H,2H,2H,4H,4H, 5H,5H-3-oxa-perfluortridecylamin zu und rührt 6 Stunden bei 50°C. Man gibt 10 ml Wasser zu, rührt 10 Minuten bei 50°C und dampft den Rückstand zur Trockne ein. Der Rückstand wird in wenig Wasser aufgenommen und mit Eisessig auf pH 4 gebracht. Der unlösliche Niederschlag wird abfiltriert und an RP-18 chromatographiert (Laufmittel: Acetonitril/Wasser/Gradient).
Ausbeute: 9,58 g (61 % d. Th.) eines farblosen Feststoffs
Wassergehalt: 8 %

| Elementaranalyse: | | | | |
|---|---|---|---|---|
| ber.: | C 33,64 | H 3,59 | N 5,35 | F 41,12 |
| gef.: | C 33,51 | H 3,69 | N 5,44 | F 41,24 |

### b) Ethylendiamin-N,N-tetraessigsäure-N-(1H,1H,2H,2H,4H,4H,5H,5H-3-oxaperfluortridecyl)-monoamid, Calciumsalz, Natriumsalz

9,0 g (11,46 mmol) der Titelsubstanz aus Beispiel 19a werden in 300 ml Wasser suspendiert und 11,4 ml 1-N-aqu. Natronlauge zugegeben. Anschließend gibt man 1,15 g (11,46 mmol) Calciumcarbonat zu und rührt 5 Stunden bei 50°C. Die Lösung wird filtriert und das Filtrat gefriergetrocknet.
Ausbeute: 9,7 g (100 % d. Th.) eines farblosen, amorphen Feststoffs
Wassergehalt: 7,5 %

| Elementaranalyse: | | | | | | |
|---|---|---|---|---|---|---|
| ber.: | C 31,25 | H 2,98 | N 4,97 | F 38,20 | Na 2,72 | Ca 4,74 |
| gef.: | C 31,40 | H 3,09 | N 5,10 | F 38,07 | Na 2,81 | Ca 4,82 |

### c) Herstellung einer Formulierung aus Gadoliniumkomplex I und Ethylendiamin-N,N-tetraessigsäure-N-(1H,1H,2H,2H,4H,4H,5H,5H-3-oxa-perfluortridecyl)-monoamid, Calciumsalz, Natriumsalz

Zu 43 ml einer Lösung des Gadoliniumkomplexes I (280 mmol/L), gelöst in 0,45 % aqu. Kochsalzlösung (pH 7,4; 0,25 mg/L CaNa₃DTPA), gibt man 2,54 g (3,01 mmol) der Titelverbindung aus Beispiel 19b und füllt mit 0,9 %iger aqu. Kochsalzlösung auf insgesamt 121 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 20

### a) 1H,1H,2H,2H-Perfluordecyl-(2,2-dimethyl-5-hydroxy-1,3-dioxepan-6-yl)-ether

30 g (64,64 mmol) 1H,1H,2H,2H-Perfluordecanol werden in 200 ml Tetrahydrofuran gelöst und bei 0°C 1,68 g (70 mmol) Natriumhydrid zugesetzt. Man rührt 2 Stunden bei Raumtemperatur, dann 4 Stunden bei 60°C. Die Lösung wird in einen Metallautoklaven gegeben, dann 9,31 g (64,64 mmol) 2,2-Dimethyl-1,3,6-trioxabicyclo[5.1.0]octan zugegeben und anschließend 10 Stunden auf 150°C erwärmt. Die Reaktionslösung wird auf eiswasser gegossen und 2 mal mit Diethylether extrahiert. Die vereinigten organischen Phasen werden zur Trockne eingedampft und der Rückstand and Kieselgel chromatographiert (Laufmittel: Dichlormethan/Aceton= 10:1)
Ausbeute: 16,12 g (41 % d. Th.) eines farblosen Feststoffs

| Elementaranalyse: | | | |
|---|---|---|---|
| ber.: | C 33,57 | H 2,82 | F 53,10 |
| gef.: | C 33,69 | H 2,90 | F 53,35 |

### b) 1H,1H,2H,2H-Perfluordecyl-(1-hydroxymethyl-2,3-dihydroxypropyl)-ether

15 g (24,66 mmol) der Titelverbindung aus Beispiel 20a werden in 300 ml Ethanol gelöst und 30 ml 10%ige aqu. Salzsäure zugegeben. Man erhitzt 5 Stunden unter Rückfluß. Es wird mit Natronlauge auf pH 7 gestellt, dann zur Trockne eingeengt und der Rückstand an RP-18 chromatographiert (Laufmittel: Acetonitril/Wasser/Gradient). Ausbeute: 12,75 g (91 % d. Th.) eines farblosen Feststoffes
Wassergehalt: 4,5 %

| Elementaranalyse: | | | |
|---|---|---|---|
| ber.: | C 29,59 | H 2,31 | F 56,84 |
| gef.: | C 29,48 | H 2,37 | F 56,99 |

### c) Herstellung einer Formulierung aus Gadoliniumkomplex IV und 1H,1H,2H,2H-Perfluordecyl-(1-hydroxymethyl-2,3-dihydroxypropyl)-ether

Zu 37 ml einer Lösung des Gadoliniumkomplexes IV (300 mmol/L), gelöst in 0,45 % aqu. Kochsalzlösung (pH 7,4; 0,25 mg/L CaNa₃DTPA), gibt man 9,46 g (16,65 mmol) der Titelverbindung aus Beispiel 20b und füllt mit 0,9 %iger aqu. Kochsalzlösung auf insgesamt 111 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 21

### a) 1H,1H,2H,2H-Perfluordecyl-[1,2-bis(2,2-dimethyl-1,3-dioxolan-4-yl)-2-hydroxyethyl]-ether

30 g (64,64 mmol) 1H,1H,2H,2H-Perfluordecanol werden in 200 ml Tetrahydrofuran gelöst und bei 0°C 1,68 g (70 mmol) Natriumhydrid zugesetzt. Man rührt 2 Stunden bei Raumtemperatur, dann 4 Stunden bei 60°C. Die Lösung wird in einen Metallautoklaven gegeben, dann 15,78 g (64,64 mmol) 1,2-Bis-(2,2-dimethyl-1,3-dioxolan-4-yl)-oxiran zugegeben und anschließend 10 Stunden auf 150°C erwärmt. Die Reaktionslösung wird auf eiswasser gegossen und 2 mal mit Diethylether extrahiert. Die vereinigten organischen Phasen werden zur Trockne eingedampft und der Rückstand and Kieselgel chromatographiert (Laufmittel: Dichlormethan/Aceton= 10:1)
Ausbeute: 14,2 g (31 % d. Th.) eines farblosen Feststoffs

| Elementaranalyse: | | | |
|---|---|---|---|
| ber.: | C 37,30 | H 3,56 | F 45,59 |
| gef.: | C 37,48 | H 3,66 | F 45,71 |

### b) 1H,1H,2H,2H-Perfluordecyl-[1,2-bis(1,2-dihydroxy-ethyl)-2-hydroxyethyl]-ether

14 g (19,76 mmol) der Titelverbindung aus Beispiel 21a werden in 300 ml Ethanol gelöst und 30 ml 10%ige aqu. Salzsäure zugegeben. Man erhitzt 5 Stunden unter Rückfluß. Es wird mit Natronlauge auf pH 7 gestellt, dann zur Trockne eingeengt und der Rückstand an RP-18 chromatographiert (Laufmittel: Acetonitril/Wasser/Gradient). Ausbeute: 10,55 g (85 % d. Th.) eines farblosen Feststoffes
Wassergehalt: 3,2 %

| Elementaranalyse: | | | |
|---|---|---|---|
| ber.: | C 30,59 | H 2,73 | F 51,41 |
| gef.: | C 30,73 | H 2,81 | F 51,58 |

### c) Herstellung einer Formulierung aus Gadoliniumkomplex II und 1H,1H,2H,2H-Perfluordecyl-[1,2-bis(1,2-dihydroxy-ethyl)-2-hydroxyethyl]-ether

Zu 41 ml einer Lösung des Gadoliniumkomplexes II (300 mmol/L), gelöst in 0,45 % aqu. Kochsalzlösung (pH 7,4; 0,25 mg/L CaNa₃DTPA), gibt man 11,98 g (19,07 mmol) der Titelverbindung aus Beispiel 21b und füllt mit 0,9 %iger aqu. Kochsalzlösung auf insgesamt 64 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 200 mmol Gd/L).

### Beispiel 22

### a) Perfluoroctylsulfonsäure-N,N-bis[(8-schwefelsäure-monoester, Natriumsalz)-3,6-dioxaoctyl]-amid

13,54 g (17,73 mmol) der Titelverbindung aus Beispiel 15a werden in 300 ml Chloroform gelöst und bei 0°C 2,82 g (17,73 mmol) Schwefeltrioxid-Pyridin-Komplex zugegeben. Man rührt eine Stunde bei 0°C und dampft anschließend im Vakuum zur Trockne ein. Der Rückstand wird in 300 ml Ethanol gelöst und mit 17,8 ml 1N aqu. Natronlauge versetzt. Die Lösung wird zur Trockne eingedampft und der Rückstand im Vakuum getrocknet (60°C / 2 h).
Ausbeute: 17,15 g (quantitativ)

| Elementaranalyse: | | | | | | |
|---|---|---|---|---|---|---|
| ber.: | C 24,83 | H 2,50 | F 33,83 | N 1,45 | S 9,94 | Na 4,75 |
| gef.: | C 24,96 | H 2,62 | F 33,97 | N 1,53 | S 10,05 | Na 4,86 |

### b) Herstellung einer Formulierung aus Gadoliniumkomplex I und Perfluoroctylsulfonsäure-N,N-bis[(8-schwefelsäure-monoester, Natriumsalz)-3,6-dioxaoctyl]-amid

Zu 43 ml einer Lösung des Gadoliniumkomplexes I (380 mmol/L), gelöst in 0,45 % aqu. Kochsalzlösung (pH 7,4; 0,25 mg/L CaNa₃DTPA), gibt man 142,29 g (147,06 mmol) der Titelverbindung aus Beispiel 22a und füllt mit 0,9 %iger aqu. Kochsalzlösung auf insgesamt 164 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 23

### a) 2-(2H,2H,3H,3H,5,5H,6H,6H-1,4,-Dioxaperfluortetradec-1-yl)-bernsteinsäurediethylester

30 g (59,03 mmol) 1H,1H,2H,2H,4H,4H,5H,5H-3-Oxa-perfluortridecanol werden in 300 ml Tetrahydrofuran und bei 0°C 1,68 g (70 mmol) Natriumhydrid zugegeben. Mna rührt eine Stunde bei 0°C, anschließend 5 Stunden bei 40°C. In diese 40°C heiße Lösung tropft man innerhalb von 10 Minuten 20,25 g (80 mmol) 2-Brom-Bernsteinsäurediethylester zu und rührt anschließend 12 Stunden bei dieser Temperatur. Man gibt 500 ml Eiswasser zu und extrahiert 2 mal mit 300 ml Diethylether. Die vereinigten organischen Phasen werden im Vakuum zur Trockne eingedampft und der Rückstand an Kieselgel chromatographiert (Laufmittel: n-Hexan/Ethanol = 20: 1):
Ausbeute: 12,05 g (30 % d. Th.)

| Elementaranalyse: | | | |
|---|---|---|---|
| ber.: | C 35,31 | H 3,11 | F 47,47 |
| gef.: | C 35,19 | H 3,20 | F 47,59 |

### b) 2-(2H,2H,3H,3H,5H,5H,6H,6H-1,4-dioxa-perfluortetradec-1-yl)-bersteinsäure, Dinatriumsalz

Zu 11,5 g (16,90 mmol) der Titelverbindung, gelöst in 300 ml Methanol, gibt man 50 ml 3-N-aqu. Natronlauge und refluxiert 8 Stunden. Man dampft zur Trockne ein und nimmt den Rückstand in 300 ml Wasser auf. Man extrahiert die wässrige Phase 2 mal mit 300 ml Diethylether. Man säuert die wässrige Phase mit konz. Salzsäure auf pH 1 an und extrahiert 2 mal mit 300 ml Chloroform. Die vereinigten Chloroformphasen werden über Magnesiumsulfat getrocknet und zur Trockne eingedampft. Der Rückstand wird in 300 ml Wasser gelöst und mit 5 %iger aqu. Natronlauge auf pH 7,4 eingestellt. Anschließend wird gefriergetrocknet.
Ausbeute: 10,50 g (93 % d. Th.) eines farblosen, amorphen Feststoffs
Wassergehalt: 5,7 %

| Elementaranalyse: | | | | |
|---|---|---|---|---|
| ber.: | C 28,76 | H 1,66 | F 48,33 | Na 6,88 |
| gef.: | C 28,88 | H 1,71 | F 48,25 | Na 6,95 |

### c) Herstellung einer Formulierung aus Gadoliniumkomplex II und 2-(2H,2H,3H,3H, 5H,5H,6H,6H-1,4-dioxa-perfluortetradec-1-yl)-bernsteinsäure, Dinatriumsalz

Zu 57 ml einer Lösung des Gadoliniumkomplexes II (300 mmol/L), gelöst in 0,45 % aqu. Kochsalzlösung (pH 7,4; 0,25 mg/L CaNa₃DTPA), gibt man 1,14 g (1,71 mmol) der Titelverbindung aus Beispiel 23b und füllt mit 0,9 %iger aqu. Kochsalzlösung auf insgesamt 154 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 24

### a) 2H,2H,4H,4H,5H,5H-3-Oxa-perfluortridecansäure-N-(succin-2-yl)-amid

Zu 20 g (38,30 mmol) 2H,2H,4H,4H,5H,5H-3-Oxa-perfluortridecansäure und 9,21 g (80 mmol) N-Hydroxysuccinimid- gelöst in 150 ml Dimethylformamid gibt man bei 0°C 16,51 g (80 mmol) N,N'-Dicyclohexylcarbodiimid zu und rührt 3 Stunden bei dieser Temperatur. Zu der so hergestellten Aktivester-Lösung gibt man eine auf 0°C gekühlte Lösung von 5,10 g (38,30 mmol) L-Asparaginsäure gelöst in 300 ml 5 %iger aqu. Natriumcarbonat-Lösung zu und rührt 2 Stunden bei 0°C. Man gießt auf 500 ml Eiswasser, filtriert vom ausgefallenen Dicyclohexylharnstoff ab und stellt mit konz. Salzsäure auf pH 1. Man extrahiert 3 mal mit 300 ml Chloroform. Die vereinigten, organischen Phasen werden zur Trockne eingeengt und der Rückstand an RP-18 chromatographiert (Laufmittel: Acetonitril/Wasser/Gradient). Die so erhaltene Disäure wird in 400 ml Wasser gelöst und mit 1N aqu. Natronlauge auf pH 7,4 gestellt. Man filtriert und das Filtrat wird gefriergetrocknet.
Wassergehalt: 6,3 %
Ausbeute: 21,13 g (81 % d.Th.) eines farblosen amorphen Pulvers

| Elementaranalyse: | | | | | |
|---|---|---|---|---|---|
| ber.: | C 28,21 | H 1,48 | N 2,06 | F 47,41 | Na 6,75 |
| gef.: | C 28,30 | H 1,53 | N 2,11 | F 47,53 | Na 6,83 |

### b) Herstellung einer Formulierung aus Gadoliniumkomplex III und 2H,2H,4H,4H, 5H,5H-3-Oxa-perfluortridecansäure-N-(succin-2-yl)-amid

Zu 37 ml einer Lösung des Gadoliniumkomplexes III (300 mmol/L), gelöst in 0,45 % aqu. Kochsalzlösung (pH 7,4; 0,25 mg/L CaNa₃DTPA), gibt man 422 mg (0,62 mmol) der Titelverbindung aus Beispiel 24a und füllt mit 0,9 %iger aqu. Kochsalzlösung auf insgesamt 111 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 25

### Herstellung einer Formulierung aus Gadoliniumkomplex VII und Perfluoroctansulfonsäure, Natriumsalz

Zu 43 ml einer Lösung des Gadoliniumkomplexes VII (250 mmol/L), gelöst in 0,45 %iger Natriumchloridlösung (pH 7,4 / 0,25 mg/L CaNa₃DTPA), gibt man eine Lösung aus 1,34 g (2,69 mmol) Perfluoroctansulfonsäure, Natriumsalz, gelöst in 200 ml Ethanol, zu und rührt 2 Stunden bei 50°C. Die Lösung wird im Vakuum zur Trockne eingeengt und der Rückstand mit dest. Wasser auf insgesamt 108 ml aufgefüllt. Man rührt 10 Minuten bei 40°C und filtriert über ein 0,2 µm Filter. Das Filtrat wird in vials abgefüllt. Eine so hergestellte Lösung kann direkt für biologische Experimente eingesetzt werden. (Die Konzentration beträgt 100 mmol Gd/L).

### Beispiel 26

### Herstellung einer Formulierung aus Gadoliniumkomplex VIII und Perfluordecansulfonsäure, Natriumsalz

Zu 49 ml einer Lösung des Gadoliniumkomplexes VIII (310 mmol/L), gelöst in 0,45 % aqu. Natriumchloridlösung), gibt man 3,03 g (5,06 mmol) Perfluordecansulfonsäure, Natriumsalz und erwärmt für 10 Minuten in der Mikrowelle. Die Lösung wird auf Raumtemperatur abgekühlt, durch ein 0,2 µm-Filter filtriert und das Filtrat in vials abgefüllt. Eine so hergestellte Lösung kann direkt für biologische Experimente eingesetzt werden. (Die Konzentration beträgt 310 mmol Gd/L).

### Beispiel 27

### a) (1H,1H,2H,2H-Perfluordecyl)-5-[(1,3-dicarboxy, Dinatriumsalz)-phenyl]-ether

Zu 20 g (80,62 mmol) Trinatriumsalz von 5-Hydroxy-isophtalsäure in 300 ml Dimethylformamid gibt man 42,5 g (80,62 mmol) der Titelverbindung aus Beispiel 14a und rührt 10 Stunden bei 60°C. Man gießt auf 1500 ml Eiswasser und stellt mit konz. Salzsäure auf pH 1. Man extrahiert 3 mal mit 300 ml Chloroform. Die vereinigten, organischen Phasen werden eingedampft und der Rückstand an RP-18 chromatographiert (Laufmittel: Acetonitril/Wasser/Gradient). Die so aufgereinigte Disäure wird in 400 ml Wasser gelöst und der pH mit 1N aqu. Natronlauge auf pH 7,4 gebracht. Man filtriert und das Filtrat wird gefriergetrocknet.
Ausbeute: 20,05 g (37 % d. Th.) eines farblosen amorphen Feststoffs
Wassergehalt: 5,0 %

| Elementaranalyse: | | | | |
|---|---|---|---|---|
| ber.: | C 32,16 | H 1,05 | F 48,05 | Na 6,84 |
| gef.: | C 32,30 | H 1,15 | F 48,20 | Na 6,95 |

### b) Herstellung einer Formulierung aus Gadoliniumkomplex IV und (1H,1H,2H,2H-Perfluordecyl)-5-[(1,3-dicarboxy, Dinatriumsalz)-phenyl]-ether

Zu 51 ml einer Lösung des Gadoliniumkomplexes IV (300 mmol/L), gelöst in 0,45 % aqu. Kochsalzlösung (pH 7,4; 0,25 mg/L CaNa₃DTPA), gibt man 6,86 g (10,2 mmol) der Titelverbindung aus Beispiel 27a und füllt mit 0,9 %iger aqu. Kochsalzlösung auf insgesamt 153 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 28

### Herstellung einer Formulierung aus Gadoliniumkomplex III und 3-Oxa-2H,2H,4H,4H, 5H,5H-perfluortridecansäure, Natriumsalz

Zu 4 ml einer Lösung des Gadoliniumkomplexes III (320 mmol/L), gelöst in 0,45 % aqu. Kochsalzlösung (pH 7,4; 0,25 mg/L CaNa₃DTPA), gibt man 434 mg (0,55 mmol) der Titelverbindung aus Beispiel 11a und füllt mit 0,9 %iger aqu. Kochsalzlösung auf insgesamt 12,8 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Konzentration beträgt 100 mmol Gd/L).

### Beispiel 29

### a) (Adamant-1-yl)-3-oxa-propionsäure-t-butylester

Zu 15,22 g (100 mmol) 1-Adamantanol in 300 ml 50 % aqu. Kalilauge, 200 ml Toluol gibt man bei 0°C 29,26 g (150 mmol) Bromessigsäure-tert. Butylester zu und rührt 2 Stunden kräftig durch. Man gießt auf 1500 ml Wasser und extrahiert 2 mal mit 300 ml Diethylether. Die vereinigten organischen Phasen werden über Magnesiumsulfat getrocknet und im Vakuum zur Trockne eingedampft. Der Rückstand wird an Kieselgel chromatographiert (Laufmittel: n-Hexan/Diethyleether 20: 1).
Ausbeute: 21,58 g (81 % d. Th.) eines viskosen, farblosen Öls

| Elementaranalyse: | | |
|---|---|---|
| ber.: | C 72,14 | H 9,84 |
| gef.: | C 72,26 | H 9,95 |

### b) (Adamant-1-yl)-3-oxa-propionsäure

20 g (75 mmol) der Titelverbindung aus Beispiel 29a werden bei 0°C in 200 ml Trifluoressigsäure gelöst und 8 Stunden bei Raumtemperatur gerührt. Man dampft zur Trockne ein und kristallisiert den Rückstand aus Diisopropylether um.
Ausbeute: 14,68 g (93 % d. Th.) farblose Blättchen

| Elementaranalyse: | | |
|---|---|---|
| ber.: | C 68,55 | H 8,63 |
| gef.: | C 68,41 | H 8,74 |

### c) 1-(Perfluoroctylsulfonyl)-4-[(adamant-1-yl)-oxapropionyl]-piperazin

14 g (66,6 mmol) der Titelverbindung aus Beispiel 29b und 37,50 g (66,6 mmol) 1-Perfluoroctylsulfonyl-piperazin werden in 300 ml Tetrahydrofuran gelöst und bei 0°C 32,15 g (130 mmol) 1,2 Dihydro-2-ethoxychinolin-1-carbonsäureethylester (=EEDQ) zugegeben. Man rührt 5 Stunden bei Raumtemperatur. Die Lösung wird im Vakuum zur Trockne eingedampft und der Rückstand an Kieselgel chromatographiert (Laufmittel: Dichlormmethan/Diethylether= 30:1).
Ausbeute: 43,05 g (85 % d. Th.) eines farblosen Feststoffs

| Elementaranalyse: | | | | | |
|---|---|---|---|---|---|
| ber.: | C 37,90 | H 3,31 | N 3,68 | S 4,22 | F 42,47 |
| gef.: | C 38,04 | H 3,42 | N 3,49 | S 4,11 | F 42,30 |

### d) Zubereitung aus 0,5 Teilen Gadoliniumkomplex I und 0,5 Teilen Einschlußverbindung aus β-Cyclodextrin-Hydrat und 1-(Perfluoroctylsulfonyl)-4-[(adamant-1-yl)-oxapropionyl]-piperazin

Zu 32 ml einer Lösung des Gadoliniumkomplexes I (280 mmol/L) gelöst in 0,45 % aqu. Kochsalzlösung (pH 7,4; 0,25 mg/L CaNa₃DTPA) gibt man 6,81 g (8,96 mmol) der Titelverbindung aus Beispiel 29c, 10,33 g (8,96) aus β-Cyclodextrinmonohydrat und füllt mit 0,9 %iger aqu. Kochsalzlösung auf insgesamt 98 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Gd-Konzentration beträgt 100 mmol Gd/L).

### Beispiel 30

### a) 3-Oxa-2H,2H,4H,4H,5H,5H-perfluortridecansäure-N-(1-adamantyl)-amid

Zu 15,12 g (100 mmol) 1-Amino-adamantan, 52,21 (100 mmol) 3-Oxa-2H,2H,4H,4H, 5H,5H-Perfluortridecansäure und 11,51 g (100 mmol) N-Hydroxysuccinimid, glöst in 300 ml Tetrahydrofuran gibt man bei 0°C 30,95 g (150 mmol) N,N-Dicyclohexylcarbodiimid. Man rührt 2 Stunden bei 0°C, anschließend 6 Stunden bei Raumtemperatur. Man filtriert vom ausgefallenen Harnstoff ab, dampft das Filtrat zur Trockne ein und chromatographiert den Rückstand an Kieselgel (Laufmittel: Dichlormethan/Aceton= 30: 1).
Ausbeute: 54,4 g (83 % d. Th.) eines wachsartigen Feststoffs

| Elementaranalyse: | | | | |
|---|---|---|---|---|
| ber.: | C 40,32 | H 3,38 | N 2,14 | F 49,28 |
| gef.: | C 40,47 | H 3,49 | N 2,03 | F 49,09 |

### b) Zubereitung aus 0,6 Teilen Gadoliniumkomplex II und 0,4 Teilen Einschlußverbindung aus β-Cyclodextrin-Hydrat und 3-Oxa-2H,2H,4H,4H,5H,5H-perfluortridecansäure-N-(1-adamantyl)-amid

Zu 41 ml einer Lösung des Gadoliniumkomplexes II (250 mmol/L) gelöst in 0,45 % aqu. Kochsalzlösung (pH 7,4; 0,25 mg/L CaNa₃DTPA) gibt man 4,48 g (6,83 mmol) der Titelverbindung aus Beispiel 30a und 7,87 g (6,83 mmol) β-Cyclodextrinmonohydrat und füllt mit 0,9 %iger aqu. Kochsalzlösung auf insgesamt 103 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Gd-Konzentration beträgt 100 mmol Gd/L).

### Beispiel 31

### a) 2-[N-(Ethyl)-N-(perfluoroctylsulfonyl)-amino]-essigsäure-N-(adamantyl)-amid

Zu 15,12 g (100 mmol) 1-Aminoadamantan, 58,52 g (100 mmol) N-(Ethyl)-N-(perfluoroctylsulfonyl)-aminoessigsäure und 11,51 g (100 mmol) N-Hydroxysuccinimid, gelöst in 300 ml Tetrahydrofuran gibt man bei 0°C 30,95 g (150 mmol) N,N-Dicyclohyexylcarbodiimid. Man rührt 2 Stunden bei 0°C, anschließend 6 Stunden bei Raumtemperatur. Man filtriert vom ausgefallenen Harnstoff ab, dampft das Filtrat zur Trockne ein und chromatographiert den Rückstand an Kieselgel (Laufmittel: Dichlormethan/Aceton= 30:1).
Ausbeute: 55,65 g (79 % d. Th.) eines amorphen Feststoffs

| Elementaranalyse: | | | | | |
|---|---|---|---|---|---|
| ber.: | C 37,51 | H 3,29 | F 45,85 | N 1,99 | S 4,55 |
| gef.: | C 37,64 | H 3,41 | F 45,99 | N 2,12 | S 4,43 |

### b) Zubereitung aus 0,6 Teilen Gadoliniumkomplex I und 0,4 Teilen Einschlußverbindung aus β-Cyclodextrin-Hydrat und 2-[N-(Ethyl)-N-(perfluoroctylsulfonyl)-amino]-essigsäure-N-(1-adamantyl)-amid

Zu 32 ml einer Lösung des Gadoliniumkomplexes I (280 mmol/L) gelöst in 0,45 % aqu. Kochsalzlösung (pH 7,4; 0,25 mg/L CaNa₃DTPA) gibt man 4,20 g (5,97 mmol) der Titelverbindung aus Beispiel 31a und 6,88 g (5,97 mmol) aus β-Cyclodextrinmonohydrat und füllt mit 0,9 %iger aqu. Kochsalzlösung auf insgesamt 90 ml auf. Man erwärmt 2 Stunden bei 60°C im Ultraschallbad. Die Lösung wird auf Raumtemperatur abgekühlt und mit aqu. 2 N Natronlauge auf pH 7,4 eingestellt. Man filtriert durch ein 0,2 µm Filter und füllt das Filtrat in vials ab. Eine so hergestellte Lösung kann direkt für biologische Experimente eingesetzt werden. (Die Gd-Konzentration beträgt 100 mmol Gd/L).

### Beispiel 32: Lymphknotenanreicherung in Meerschweinchen nach interstitieller Applikation

Eine galenische Formulierung bestehend aus Komplex 1 (Gd-GlyMe-DOTA-perfluoroctylsulfonamid) und der Verbindung aus Beispiel 11 (Mannose-perfluoroctylsulfonamid; Anteil 40 Mol%) wurde 30 und 60 min sowie 24 h nach subkutaner Gabe (10 µmol Gesamtgadolinium/kg KGW, Hinterpfote s.c.) in Meerschweinchen mit stimulierten Lymphknoten (komplettes Freund-Adjuvant; jeweils 0,1 ml i.m. in den rechten und linken Ober- und Unterschenkel; 2 Wochen vor Gabe der Prüfsubstanz) hinsichtlich ihrer Lymphknotenanreicherung in drei aufeinanderfolgenden Lymphknotenstationen (popliteal, inguinal, iliakal) untersucht. Hierbei wurden die nachfolgend aufgelisteten Ergebnisse (Ermittlung der Gadoliniumkonzentration mittels ICP-AES, MW ± SD, n=3) erhalten:

| | **Gd-Gehalt in** | | | | | |
|---|---|---|---|---|---|---|
| | **[µmol/l]** | | | **[% Dosis/g Gewebe]** | | |
| | **30' p.i.** | **60' p.i.** | **24 h p.i.** | **30' p.i.** | **60' p.i.** | **24 h p.i.** |
| popliteal | 1643 ± 450 | 1114 ± 470 | 131 ± 31 | 48.1 ± 13.2 | 32.6 ± 13.8 | 3.8 ± 0.9 |
| ing. prof. | 787 ± 302 | 779 ± 414 | 144 ± 72 | 23.1 ± 8.8 | 22.8 ± 12.1 | 4.2 ± 2.1 |
| iliakal | 685 ± 262 | 575 ± 349 | 120 ± 8 | 20.1 ± 7.7 | 16.8 ± 10.2 | 3.5 ± 0.2 |
| Blut | 4 ± 2 | 6 ± 1 | 2 ± 0 | 0.1 ± 0.1 | 0.2 ± 0.0 | 0.1 ± 0.0 |
| Urin | 0 ± 0 | 1 ± 1 | 1 ± 1 | 0.0 ± 0.0 | 0.0 ± 0.0 | 0.0 ± 0.0 |

### Beispiel 33: Retention des kontrastgebenden Metalls am Injektionsort nach interstitieller Applikation in Meerschweinchen

Nach s.c. Gabe von 10 µmol Gesamtgadolinium/kg KGW einer galenischen Formulierung bestehend aus Komplex I (Gd-GlyMe-DOTA-perfluoroctylsulfonamid) und der Verbindung aus Beispiel 11 (Mannose-perfluoroctylsulfon-amid; Anteil 40 Mol %) in die Meerschweinchenpfote wurde die Retention des Metalls am Injektionsort zu verschiedenen Zeitpunkten untersucht (MW ± SD, n=3).

| | **Gd-Gehalt am Applikationsort (Pfote)** | |
|---|---|---|
| | [µmol/l] | [% Dosis] |
| **30 min p.i.** | 1607 ± 184 | 101.6 ± 19.3 |
| **90 min p.i.** | 1219 ± 173 | 79.9 ± 13.2 |
| **24 h p.i.** | 57 ± 7 | 3.5 ± 0.5 |
| **7 d p.i.** | 49 ± 22 | 3.1 ± 1.5 |

### Beispiel 34: Organverteilung des kontrastgebenden Metalls nach interstitieller Applikation in Meerschweinchen

Nach subkutaner Gabe von 10 µmol Gesamtgadolinium/kg KGW einer galenischen Formulierung bestehend aus Komplex I (Gd-GlyMe-DOTA-perfluoroctylsulfonamid) und der Verbindung aus Beispiel 11 (Mannose-perfluoroctylsulfon-amid; Anteil 40 Mol%) in die Hinterpfote von Meerschweinchen mit stimulierten Lymphknoten (komplettes Freund-Adjuvant; jeweils 0,1 ml i.m. in den rechten und linken Ober- und Unterschenkel; 2 Wochen vor Gabe der Prüfsubstanz) wurde 7 Tage nach Applikation die Retention des Metalls in der Leber sowie in Nieren und Milz untersucht (MW ± SD, n=3).

| **7 d p.i.** | **Gd-Gehalt in [% Dosis]** |
|---|---|
| **Leber** | 2.4 ± 0.6 |
| **Nieren** | 0.2 ± 0.0 |
| **Milz** | 0.0 ± 0.0 |

### Beispiel 35: Lymphknotendarstellung (MRT) nach intravenöser Gabe des Kontrastmittels in Meerschweinchen

Die Abbildung 1 zeigt MR-Aufnahmen von poplitealen, inguinalen und iliakalen Lymphknoten sowohl vor (baseline: Präkontrast), als auch 60 min bzw. 24 h nach intravenöser Applikation von 100 µmol Gd/kg KGW einer galenischen Formulierung bestehend aus Gd-GlyMe-DOTA-perfluoroctyl-sulfonamid (Komplex I) und Mannose-perfluoroctylsulfon-amid (Beispiel 11; Anteil 40 Mol%) in Meerschweinchen mit stimulierten Lymphknoten (komplettes Freund-Adjuvant; jeweils 0,1 ml i.m. in den rechten und linken Ober- und Unterschenkel; 2 Wochen vor Gabe der Prüfsubstanz). Die T₁-gewichteten Gradientenecho-Aufnahmen (TR 10 ms, TE 5 ms, □ 40°) verdeutlichen den starken Signalanstieg in den verschiedenen Lymphknoten (Pfeile) im Vergleich zum Präkontrast-Bild. In der Tabelle sind die entsprechenden Enhancementwerte (in % zum Präkontrastwert) sowie Signalintensitätsquotienten Lymphknoten/Muskel aufgeführt (MW ± SD, n=3).

| **Zeit p.i.** | **Komplex I + Beispiel 11; 100 µmol Gd/kg (n=3)** | | | | | |
|---|---|---|---|---|---|---|
| | **Enhancement [%]** | | | **SI Lymphknoten/Muskel** | | |
| **[min]** | **popliteal** | **ing.profund** | **iliakal** | **popliteal** | **ing.profund** | **iliakal** |
| 0 min | - | - | - | 0.9 ± 0.2 | 1.2 ± 0.1 | 1.0 ± 0.1 |
| 60min | 170 ± 38 | 123 ± 20 | 122 ± 13 | 1.2 ± 0.2 | 1.4 ± 0.1 | 1.2 ± 0.1 |
| 24h | 114 ± 37 | 69 ± 7 | 67 ± 14 | 1.3 ± 0.4 | 1.4 ± 0.0 | 1.2 ± 0.0 |

### Beispiel 36: Lymphknotendarstellung (MRT) nach intravenöser Gabe des Kontrastmittels in Meerschweinchen

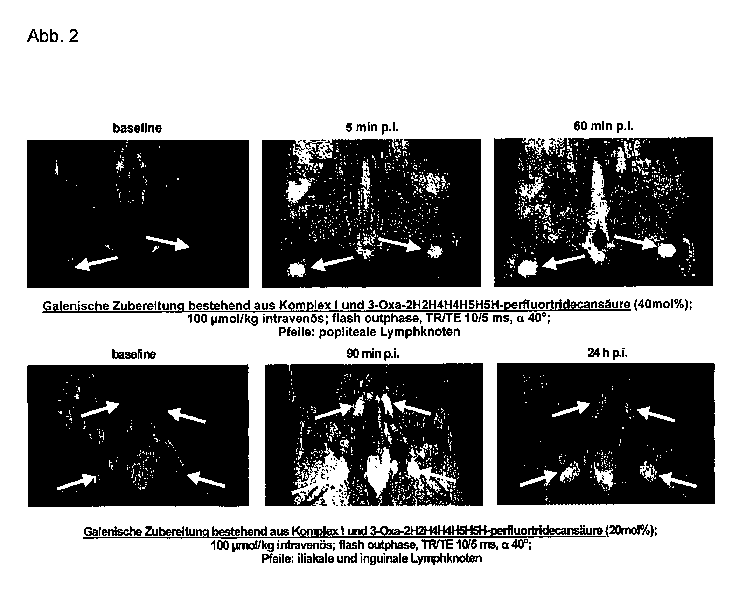

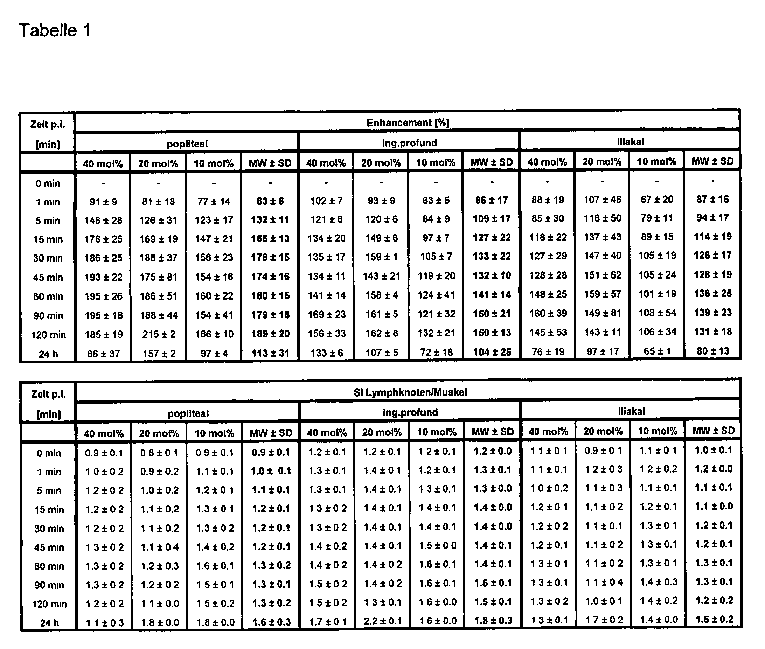

Die Abbildung 2 zeigt MR-Aufnahmen von poplitealen, inguinalen und iliakalen Lymphknoten sowohl vor (baseline: Präkontrast), als auch 5, 60, 90 min bzw. 24 h nach intravenöser Applikation von 100 µmol Gd/kg KGW einer galenischen Formulierung bestehend aus Komplex I (Gd-GlyMe-DOTA-perfluoroctyl-sulfonamid) und 3-Oxa-2H2H4H4H5H5H-perfluortridecansäure (Anteil 10, 20 oder 40 Mol%) in Meerschweinchen mit stimulierten Lymphknoten (komplettes Freund-Adjuvant; jeweils 0,1 ml i.m. in den rechten und linken Ober- und Unterschenkel; 2 Wochen vor Gabe der Prüfsubstanz). Die T₁-gewichteten Gradientenecho-Aufnahmen (TR 10 ms, TE 5 ms, α = 40°) verdeutlichen den starken Signalanstieg in den verschiedenen Lymphknoten (Pfeile) im Vergleich zum Präkontrast-Bild. In der Tabelle 1 sind die entsprechenden Enhancementwerte (in % zum Präkontrastwert) sowie Signalintensitätsquotienten Lymphknoten/Muskel aufgeführt (MW ± SD, n=3).

### Beispiel 37: Lymphknotendarstellung (MRT) nach intravenöser Gabe des Kontrastmittels in Meerschweinchen

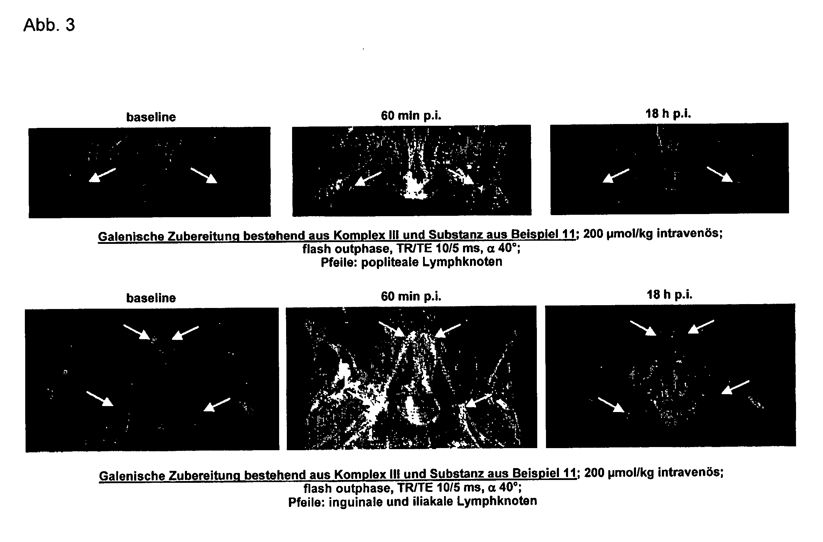

Die Abbildung 3 zeigt MR-Aufnahmen von poplitealen, inguinalen und iliakalen Lymphknoten sowohl vor (baseline: Präkontrast), als auch 60 min bzw. 18 h nach intravenöser Applikation von 200 µmol Gd/kg KGW einer galenischen Formulierung bestehend aus Komplex III (Gd-GlyMe-DOTA-trimer-perfluoroctyl-oxadecylamid) und der Verbindung aus Beispiel 11 (Mannose-perfluoroctylsulfonamid; Anteil: 40 mol%) in Meerschweinchen mit stimulierten Lymphknoten (komplettes Freund-Adjuvant; jeweils 0,1 ml i.m. in den rechten und linken Ober- und Unterschenkel; 2 Wochen vor Gabe der Prüfsubstanz). Die T₁-gewichteten Gradientenecho-Aufnahmen (TR 10 ms, TE 5 ms, α 40°) verdeutlichen den starken Signalanstieg in den verschiedenen Lymphknoten (Pfeile) im Vergleich zum Präkontrast-Bild. In der Tabelle sind die entsprechenden Enhancementwerte (in % zum Präkontrastwert) sowie Signalintensitätsquotienten Lymphknoten/Muskel aufgeführt (MW ± SD, n=3).

| **Zeit p.i.** | **Enhancement [%]** | | **SI Lymphknoten/Muskel** | |
|---|---|---|---|---|
| | **popliteal** | **iliakal** | **popliteal** | **iliakal** |
| 0 min | - | - | 0.7 ± 0.2 | 1.1 ± 0.1 |
| 60 min | 263 ± 167 | 139 ± 43 | 1.0 ± 0.1 | 1.1 ± 0.1 |
| 18h | 21 ± 21 | 18 ± 10 | 0.8 ± 0.2 | 1.2 ± 0.2 |

### Beispiel 38: Organverteilung (einschließlich Lymphknotenanreicherung) nach intravenöser Gabe des Kontrastmittels in Prostatakarzinom-tragenden Ratten

Nach intravenöser Applikation von 180 µmol Gesamtgadolinium/kg KGW einer galenischen Formulierung bestehend aus Gd-GlyMe-DOTA-perfluoroctylsulfonamid (Komplex I) und Mannose-perfluoroctylsulfonamid (Beispiel 11; Anteil 40 Mol %) in Ratten (Cop-Inzucht, mit 12 Tage zuvor i.m. implantiertem Prostatakarzinom Dunning R3327 MAT-Lu) wurde 10 Minuten, 1 und 24 Stunden nach Applikation der Metallgehalt in verschiedenen Organen sowie den Lymphknoten (gepoolt als mesenteriale und periphere Lymphknoten) bestimmt (MW ± SD, n=3).

| | **Komplex I + Substanz aus Beispiel 11** | | | | | |
|---|---|---|---|---|---|---|
| | **Gd-Konzentration [µmol/l]** | | | **% Dosis** | | |
| | **10 min p.i.** | **1h p.i.** | **24h p.i.** | **10 min p.i.** | **1h p.i.** | **24h p.i.** |
| **Leber** | 767 ± 37 | 683 ± 27 | 1350 ± 23 | 14,12 ± 1,74 | 11,49 ± 0,33 | 22,25 ± 0,84 |
| **Milz** | 713 ± 156 | 862 ± 17 | 1140 ± 60 | 0,65 ± 0.16 | 0,87 ± 0,03 | 1,22 ± 0,10 |
| **Pankreas** | 527 ± 118 | 407 ± 1 | 213 ± 16 | 1,03 ± 0,10 | 0,65 ± 0.03 | 0,39 ± 0,07 |
| **Niere** | 1116 ± 47 | 875 ± 68 | 1059 ± 48 | 3,42 ± 0,19 | 2,81 ± 0,13 | 3,98 ± 0,28 |
| **Lunge** | 1309 ± 125 | 980 ± 21 | 614 ± 22 | 3,93 ± 0,65 | 2,45 ± 0.02 | 1,34 ± 0,05 |
| **Herz** | 604 ± 46 | 407 ± 36 | 162 ± 7 | 0,96 ± 0,17 | 0,57 ± 0,08 | 0,20 ± 0,02 |
| **Gehirn** | 64 ± 5 | 39 ± 2 | 24 ± 2 | 0,20 ± 0,01 | 0,12 ± 0,01 | 0,08 ± 0,01 |
| **Muskel****** | 98 ± 18 | 109 ± 8 | 44 ± 3 | 0,09 ± 0,02 | 0,10 ± 0,00 | 0.04 ± 0,01 |
| **Tumor** | 97 ± 5 | 196 ± 7 | 248 ± 4 | 0,10 ± 0,04 | 0,50 ± 0,20 | 0,39 ± 0,08 |
| **Femur** | 176 ± 22 | 162 ± 16 | 120 ± 6 | 0,74 ± 0,06 | 0,69 ± 0,00 | 0,53 ± 0,05 |
| **mes. LK** | 406 ± 44 | 663 ± 71 | 574 ± 55 | 0,12 ± 0,03 | 0,17 ± 0,00 | 0,21 ± 0,02 |
| **periph. LK** | 205 ± 58 | 465 ± 33 | 400 ± 34 | 0,10 ± 0,03 | 0,26 ± 0,02 | 0,23 ± 0,04 |
| **Magen (entleert)** | 331 ± 18 | 325 ± 10 | 255 ± 16 | 0,92 ± 0,07 | 1,01 ± 0,02 | 0,70 ± 0,03 |
| **Darm (entleert)** | 441 ± 42 | 538 ± 26 | 434 ± 14 | 4,15 ± 0,63 | 4,39 ± 0,16 | 3,56 ± 0,04 |
| **(Blut)** * | 914 ± 158 | 546 ± - | 234 ± 11 | 29,88 ± 5,09 | 17,88 ± - | 7,66 ± 0,34 |
| **Restkörper**** | 346 ± 14 | 397 ± 38 | 245 ± 2 | 67,65 ± 2,70 | 77,18 ± 4,67 | 46,14 ± 0,58 |
| **Harn 0-24 h** | - | - | 11 ± 2 | - | - | 1,68 ± 0,34 |
| **Faeces 0-24h** | - | - | 3075 ± 748 | - | - | 18,90 ± 1,18 |
| | | **Summe Organe***** | | 98,17 ± 3,28 | 103,3 ± 3,77 | 81,02 ± 1,35 |
| | | **Bilanz ***** | | - | - | 101,6 ± 2,06 |

| | | | | | | |
|---|---|---|---|---|---|---|
| * Blutproben sind im Restkörper enthalten ** 58ml Blut/kg KGW *** Bilanz ohne Blutwerte, da diese im Restkörper enthalten **** nur Gewebealiquot | | | | | | |

### Beispiel 39: Organverteilung (einschließlich Lymphknotenanreicherung) nach intravenöser Gabe des Kontrastmittels in Prostatakarzinom-tragenden Ratten

Nach intravenöser Applikation von 200 µmol Gesamtgadolinium/kg KGW einer galenischen Formulierung bestehend aus Gd-GlyMe-DOTA-trimer-perfluoroctyl-oxadecylamid (Komplex III) und Mannose-perfluoroctylsulfonamid (Beispiel 11; Anteil: 40 mol%) in Ratten (Cop-Inzucht, mit 12 Tage zuvor i.m. implantiertem Prostatakarzinom Dunning R3327 MAT-Lu) wurde 10 Minuten, 1 und 24 Stunden nach Applikation der Metallgehalt in verschiedenen Organen sowie den Lymphknoten (gepoolt als mesenteriale und periphere Lymphknoten) bestimmt (MW ± SD, n=3).

| | **Komplex III und Subbstanz aus Beispiel 11** | | | | | |
|---|---|---|---|---|---|---|
| | **Gd-Konzentration [µmol/l]** | | | **% Dosis pro Gesamtgewebe** | | |
| | **10min p.i.** | **1h p.i.** | **24h p.i.** | **10min p.1.** | **1h p.i.** | **24h p.i.** |
| **Leber** | 183 ± 18 | 135 ± 12 | 61 ± 7 | 2,80 ± 0,20 | 2,09 ± 0,31 | 1,04 ± 0,01 |
| **Milz** | 165 ± 12 | 133 ± 10 | 58 ± 7 | 0,12 ± 0,00 | 0,10 ± 0,01 | 0,04 ± 0.01 |
| **Pankreas** | 249 ± 85 | 161 ± 11 | 25 ± 6 | 0,33 ± 0,11 | 0,22 ± 0,04 | 0,03 ± 0,00 |
| **Niere** | 2072 ± 214 | 1535 ± 654 | 378 ± 54 | 5,61 ± 0,56 | 4,23 ± 2,02 | 1,02 ± 0,13 |
| **Lunge** | 510 ± 25 | 391 ± 40 | 44 ± 2 | 1,05 ± 0,11 | 0,78 ± 0,11 | 0,09 ± 0,01 |
| **Herz** | 274 ± 25 | 262 ± 43 | 17 ± 2 | 0,32 ± 0,04 | 0,35 ± 0,11 | 0,02 ± 0.00 |
| **Gehirn** | 33 ± 3 | 22 ± 2 | 2 ± 0 | 0,11 ± 0,01 | 0,07 ± 0,00 | 0,01 ± 0,00 |
| **Muskel****** | 70 ± 1 | 51 ± 5 | 5 ± 2 | 0,07 ± 0,01 | 0,06 ± 0,02 | 0,00 ± 0,00 |
| **Tumor** | 194 ± 19 | 261 ± 12 | 47 ± 10 | 0,19 ± 0,03 | 0,24 ± 0,03 | 0,03 ± 0,01 |
| **Femur** | 125 ± 8 | 104 ± 10 | 11 ± 2 | 0,46 ± 0,04 | 0,41 ± 0,06 | 0,04 ± 0,00 |
| **mes. LK** | 266 ± 18 | 167 ± 4 | 52 ± 5 | 0,10 ± 0,01 | 0,06 ± 0,01 | 0,02 ± 0,00 |
| **periph. LK** | 260 ± 10 | 277 ± 32 | 43 ± 4 | 0,16 ± 0,02 | 0,20 ± 0,02 | 0,03 ± 0,00 |
| **Magen (entleert)** | 191 ± 25 | 143 ± 12 | 14 ± 3 | 0,49 ± 0,06 | 0,37 ± 0,03 | 0,04 ± 0,01 |
| **Darm (entleert)** | 200 ± 8 | 147 ± 4 | 25 ± 1 | 1.52 ± 0,02 | 1,14 ± 0,06 | 0,02 ± 0,01 |
| **Blut**** | 1039 ± 34 | 709 ± 28 | 17 ± 2 | 30,09 ± 0,81 | 20,51 ± 0.64 | 0,49 ± 0,07 |
| **Restkörper*** | 427 ± 10 | 428 ± 21 | 36 ± 4 | 67,77 ± 2,28 | 66,57 ± 4,46 | 5,77 ± 0,56 |
| **Harn 0-24 h** | - | - | 509 ± 93 | - | - | 81,34 ± 1,90 |
| **Faeces 0-24** h | - | - | 669 ± 224 | - | - | 8,33 ± 2,62 |
| | | Summe der Organe*** | | 80.42 ± 2,42 | 76,43 ± 4.43 | 8,31 ± 0,73 |
| | | Bilanz*** | | | | 97,98 ± 3,27 |

| | | | | | | |
|---|---|---|---|---|---|---|
| * Blutproben sind im Restkörper enthalten ** 58ml Blut/kg KGW *** Bilanz ohne Blutwerte, da diese im Restkörper enthalten **** nur Gewebealiquot | | | | | | |

## Patentansprüche

1. Galenische Formulierung, **dadurch gekennzeichnet, daß** sie paramagnetische perfluoralkylhaltige Verbindungen gemäß allgemeiner Formel I
R^{F}-A (I),
worin R^{F} einen geradkettigen oder verzweigten Perfluoralkylrest mit 4 bis 30 Kohlenstoffatomen darstellt und A ein Molekülteil ist, das 1 - 6 Metallkomplexe enthält,
und diamagnetische perfluoralkylhaltige Verbindungen gemäß allgemeiner Formel XVI
R^{F}-L¹-B² (XVI),
worin R^{F} einen geradkettigen oder verzweigten Perfluoralkylrest mit 4 bis 30 Kohlenstoffatomen darstellt, L¹ für einen Linker und B² für eine hydrophile Gruppe steht,
enthält.

2. Formulierung gemäß Anspruch 1, **dadurch gekennzeichnet, daß** das Verhältnis der paramagnetischen zu den diamagnetischen perfluoralkylhaltigen Verbindungen zwischen 5:95 und 95:5 liegt.

3. Formulierung gemäß Anspruch 1, **dadurch gekennzeichnet, daß** die paramagnetischen und diamagnetischen perfluoralkylhaltigen Verbindungen in einem wäßrigen Lösungsmittel gelöst vorliegen.

4. Formulierung gemäß Anspruch 1, **dadurch gekennzeichnet, daß** das Molekülteil A für eine Gruppe L - M steht, wobei L für einen Linker und M für einen Metallkomplex bestehend aus einem offenkettigen oder cyclischen Chelator steht, welcher als Zentralatom ein Atom der Ordnungszahlen 21 - 29, 39, 42, 44 oder 57 - 83 enthält.

5. Formulierung gemäß Anspruch 4, **dadurch gekennzeichnet, daß** der Linker L eine direkte Bindung, eine Methylengruppe, eine -NHCO-Gruppe, eine Gruppe 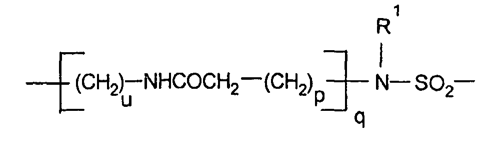 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3 - NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 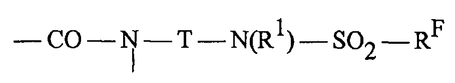 oder 1 bis 2 gegebenenfalls
substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
R^{F} einen geradkettigen oder verzweigten Perfluoralkylrest mit 4 bis 30 Kohlenstoffatomen darstellt, und
T eine C₂-C₁₀-Kette bedeutet, die gegebenenfalls durch 1 bis 2 Sauerstoffatome oder 1 bis 2 -NHCO-Gruppen unterbrochen ist.

6. Formulierung gemäß Anspruch 4, **dadurch gekennzeichnet, daß** der Metallkomplex M für einen Komplex der allgemeinen Formel II steht 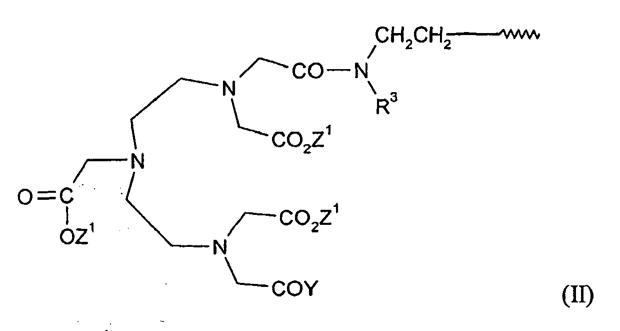 in der R³, Z¹ und Y unabhängig voneinander sind und
R³ die Bedeutung von R¹ hat oder -(CH₂)ₘ-L-R^{F} bedeutet, wobei
m 0, 1 oder 2 ist und
L eine direkte Bindung, eine Methylengruppe, eine -NHCO-Gruppe, eine Gruppe 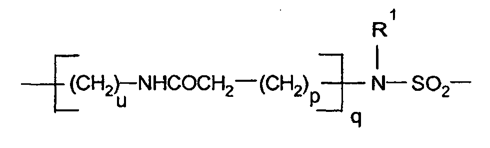 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 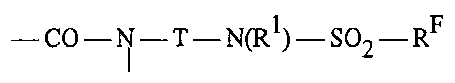 oder 1 bis 2 gegebenenfalls
substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
R^{F} einen geradkettigen oder verzweigten Perfluoralkylrest mit 4 bis 30 Kohlenstoffatomen darstellt,
T eine C₂-C₁₀-Kette bedeutet, die gegebenenfalls durch 1 bis 2 Sauerstoffatome oder 1 bis 2 -NHCO-Gruppen unterbrochen ist,
Z¹ unabhängig voneinander ein Wasserstoffatom oder ein Metallionenäquivalent der Ordnungszahlen 21 - 29, 39, 42, 44 oder 57 - 83 bedeutet,
Y -OZ¹ oder 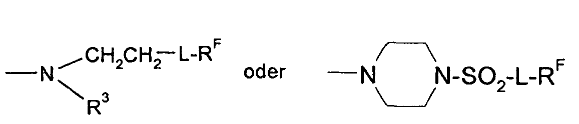 bedeutet.

7. Formulierung gemäß Anspruch 4, **dadurch gekennzeichnet, daß** der Metallkomplex M für einen Komplex der allgemeinen Formel III steht 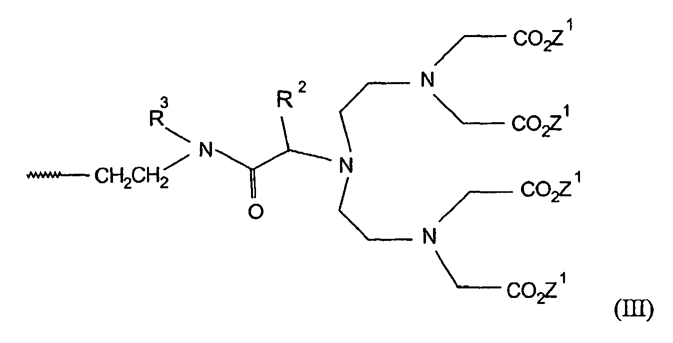 in der R³, Z¹ unabhängig voneinander sind und
R³ die Bedeutung von R¹ hat oder -(CH₂)ₘ-L-R^{F} bedeutet, wobei
m 0, 1 oder 2 ist und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
L eine direkte Bindung, eine Methylengruppe, eine -NHCO-Gruppe, eine Gruppe 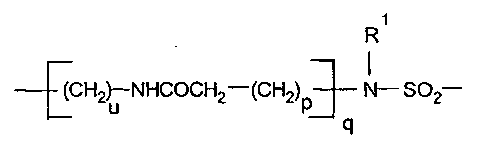 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 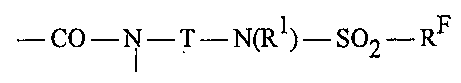 oder 1 bis 2 gegebenenfalls substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
R^{F} einen geradkettigen oder verzweigten Perfluoralkylrest mit 4 bis 30 Kohlenstoffatomen darstellt,
T eine C₂-C₁₀-Kette bedeutet, die gegebenenfalls durch 1 bis 2 Sauerstoffatome oder 1 bis 2 -NHCO-Gruppen unterbrochen ist, und
Z¹ unabhängig voneinander ein Wasserstoffatom oder ein Metallionenäquivalent der Ordnungszahlen 21 - 29, 39, 42, 44 oder 57 - 83 bedeutet,
und R² die Bedeutung von R¹ hat.

8. Formulierung gemäß Anspruch 4, **dadurch gekennzeichnet, daß** der Metallkomplex M für einen Metallkomplex der allgemeinen Formel IV steht 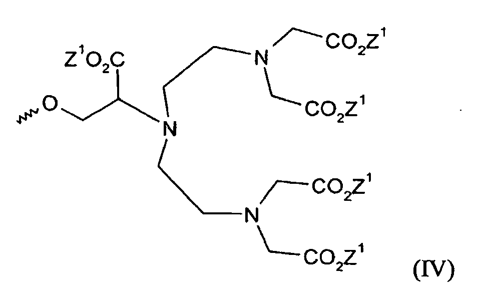 wobei Z¹ unabhängig voneinander ein Wasserstoffatom oder ein Metallionenäquivalent der Ordnungszahlen 21 - 29, 39, 42, 44 oder 57 - 83 bedeutet.

9. Formulierung gemäß Anspruch 4, **dadurch gekennzeichnet, daß** der Metallkomplex M für einen Metallkomplex der allgemeinen Formel V steht 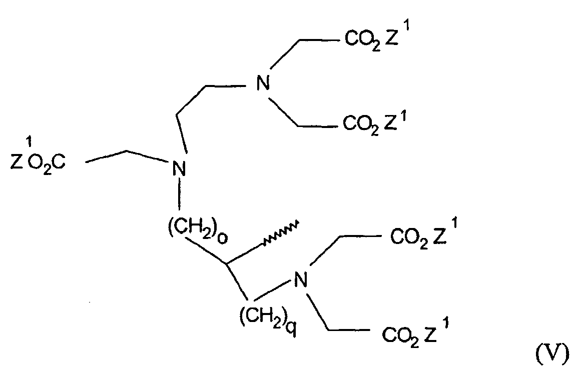 wobei Z¹ unabhängig voneinander ein Wasserstoffatom oder ein Metallionenäquivalent der Ordnungszahlen 21 - 29, 39, 42, 44 oder 57 - 83 bedeutet und o und q für die Ziffern 0 oder 1 stehen und die Summe o + q = 1 ergibt.

10. Formulierung gemäß Anspruch 4, **dadurch gekennzeichnet, daß** der Metallkomplex M für einen Metallkomplex der allgemeinen Formel VI steht 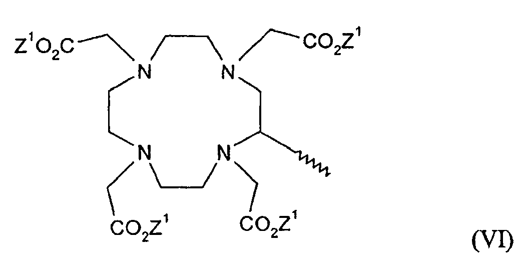 wobei Z¹ unabhängig voneinander ein Wasserstoffatom oder ein Metallionenäquivalent der Ordnungszahlen 21 - 29, 39, 42, 44 oder 57 - 83 bedeutet.

11. Formulierung gemäß Anspruch 4, **dadurch gekennzeichnet, daß** der Metallkomplex M für einen Metallkomplex der allgemeinen Formel VII steht 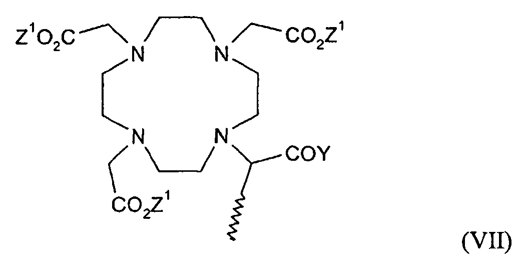 wobei Z¹ unabhängig voneinander ein Wasserstoffatom oder ein Metallionenäquivalent der Ordnungszahlen 21 - 29, 39, 42, 44 oder 57 - 83 bedeutet und
Y -OZ¹ oder 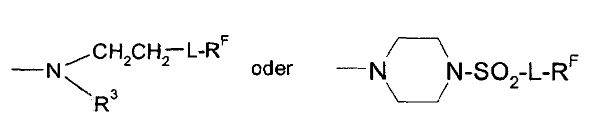 bedeutet, wobei
L eine direkte Bindung, eine Methylengruppe, eine -NHCO-Gruppe, eine Gruppe 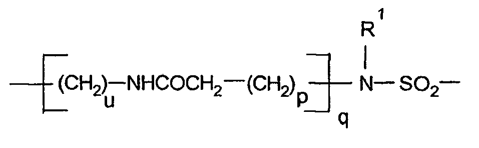 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 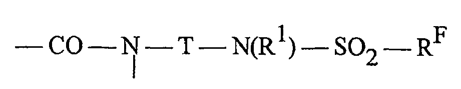 oder 1 bis 2 gegebenenfalls substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R¹ ein Wasserstoffatom, eine Methylgruppe, eine -CH₂-OH-Gruppe, eine - CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
R^{F} einen geradkettigen oder verzweigten Perfluoralkylrest mit 4 bis 30 Kohlenstoffatomen darstellt,
T eine C₂-C₁₀-Kette bedeutet, die gegebenenfalls durch 1 bis 2 Sauerstoffatome oder 1 bis 2 -NHCO-Gruppen unterbrochen ist, und
R³ die Bedeutung von R¹ hat oder -(CH₂)ₘ-L-R^{F} bedeutet, wobei
m 0, 1 oder 2 ist.

12. Formulierung gemäß Anspruch 4, **dadurch gekennzeichnet, daß** der Metallkomplex M ein Komplex der allgemeinen Formel VIII ist 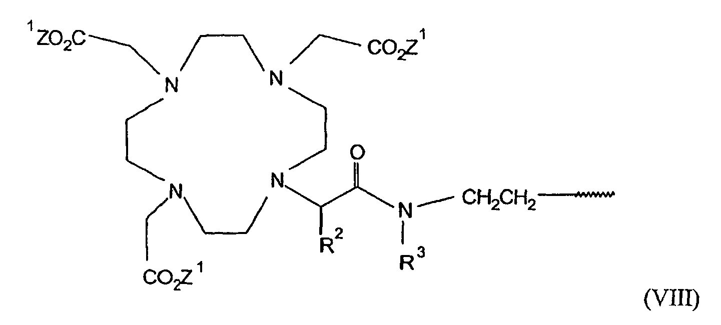 in der in der R³, Z¹ unabhängig voneinander sind und
R³ die Bedeutung von R¹ hat oder -(CH₂)ₘ-L-R^{F} bedeutet, wobei
m 0, 1 oder 2 ist und
L eine direkte Bindung, eine Methylengruppe, eine -NHCO-Gruppe, eine Gruppe 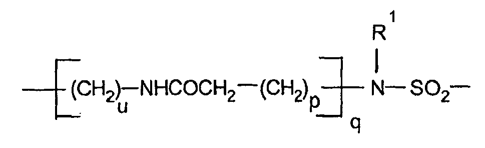 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 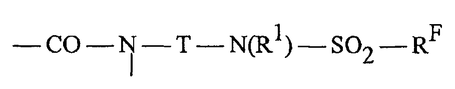 oder 1 bis 2 gegebenenfalls substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R¹ ein Wasserstoffatom, eine Methylgruppe, eine -CH₂-OH-Gruppe, eine-CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
R^{F} einen geradkettigen oder verzweigten Perfluoralkylrest mit 4 bis 30 Kohlenstoffatomen darstellt,
T eine C₂-C₁₀-Kette bedeutet, die gegebenenfalls durch 1 bis 2 Sauerstoffatome oder 1 bis 2 -NHCO-Gruppen unterbrochen ist und
Z¹ unabhängig voneinander ein Wasserstoffatom oder ein Metallionenäquivalent der Ordnungszahlen 21 - 29, 39, 42, 44 oder 57 - 83 bedeutet,
und R² die Bedeutung von R¹.

13. Formulierung gemäß Anspruch 4, **dadurch gekennzeichnet, daß** der Metallkomplex M ein Komplex der allgemeinen Formel IX ist 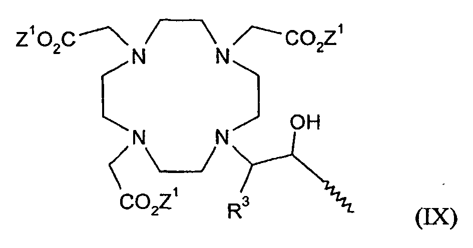 in der
Z¹ unabhängig voneinander ein Wasserstoffatom oder ein Metallionenäquivalent der Ordnungszahlen 21 - 29, 39, 42, 44 oder 57 - 83 bedeutet, und
R³ die Bedeutung von R¹ hat oder -(CH₂)ₘ-L-R^{F} bedeutet, wobei
m 0, 1 oder 2 ist und
L eine direkte Bindung, eine Methylengruppe, eine -NHCO-Gruppe, eine Gruppe 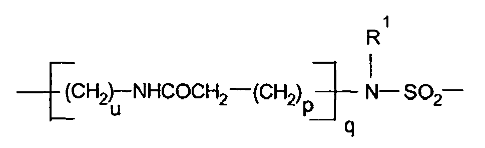 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 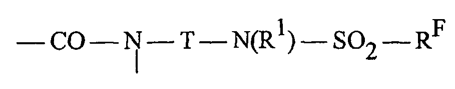 oder 1 bis 2 gegebenenfalls substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R¹ ein Wasserstoffatom, eine Methylgruppe, eine -CH₂-OH-Gruppe, eine-CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
R^{F} einen geradkettigen oder verzweigten Perfluoralkylrest mit 4 bis 30 Kohlenstoffatomen darstellt, und
T eine C₂-C₁₀-Kette bedeutet, die gegebenenfalls durch 1 bis 2 Sauerstoffatome oder 1 bis 2 -NHCO-Gruppen unterbrochen ist.

14. Formulierung gemäß Anspruch 4, **dadurch gekennzeichnet, daß** der Metallkomplex M ein Komplex der allgemeinen Formel X ist 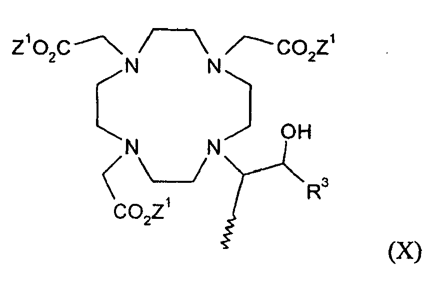
Z¹ unabhängig voneinander ein Wasserstoffatom oder ein Metallionenäquivalent der Ordnungszahlen 21 - 29, 39, 42, 44 oder 57 - 83 bedeutet, und
R³ die Bedeutung von R¹ hat oder -(CH₂)ₘ-L-R^{F} bedeutet, wobei
m 0, 1 oder 2 ist und
L eine direkte Bindung, eine Methylengruppe, eine -NHCO-Gruppe, eine Gruppe 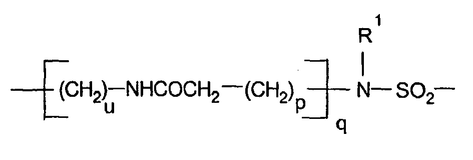 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 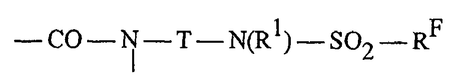 oder 1 bis 2 gegebenenfalls
substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R¹ ein Wasserstoffatom, eine Methylgruppe, eine -CH₂-OH-Gruppe, eine-CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, und
R^{F} einen geradkettigen oder verzweigten Perfluoralkylrest mit 4 bis 30
Kohlenstoffatomen darstellt, und
T eine C₂-C₁₀-Kette bedeutet, die gegebenenfalls durch 1 bis 2 Sauerstoffatome oder 1 bis 2 -NHCO-Gruppen unterbrochen ist.

15. Formulierung gemäß Anspruch 4, **dadurch gekennzeichnet, daß** der Metallkomplex M ein Komplex der allgemeinen Formel XI ist 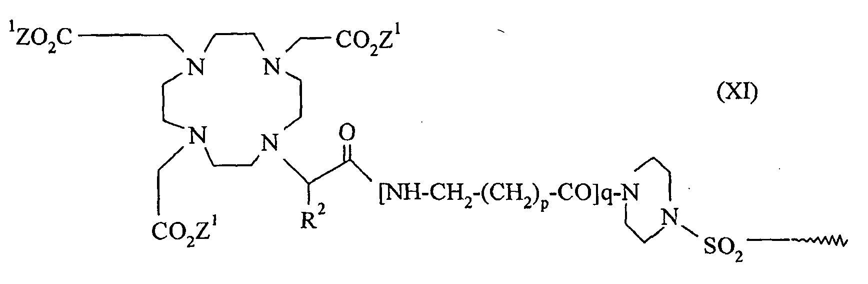 in der
Z¹ unabhängig voneinander ein Wasserstoffatom oder ein Metallionenäquivalent der Ordnungszahlen 21 - 29, 39, 42, 44 oder 57 - 83 bedeutet, und
p die Zahlen 0 bis 10, q die Zahlen 0 oder 1 und
R² ein Wasserstoffatom, eine Methylgruppe, eine -CH₂-OH-Gruppe, eine-CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen.

16. Formulierung gemäß Anspruch 4, **dadurch gekennzeichnet, daß** der Metallkomplex M ein Komplex der allgemeinen Formel XII ist 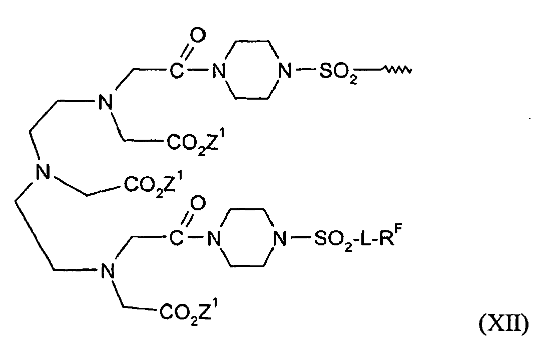 in der L eine direkte Bindung, eine Methylengruppe, eine -NHCO-Gruppe, eine Gruppe 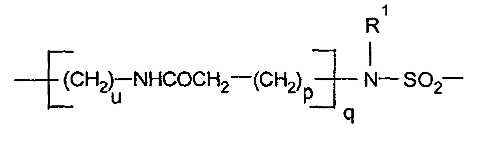 ist,
wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen,
oder eine geradkettige, verzweigte, gesättigte oder ungesättigte C₂-C₃₀-Kohlenstoffkette ist, die gegebenenfalls 1 bis 10 Sauerstoffatome, 1 bis 3-NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen, eine Gruppe 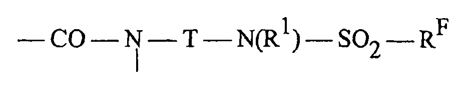 oder 1 bis 2 gegebenenfalls
substituierte Aryle enthält und/oder durch diese Gruppen unterbrochen ist, und/oder gegebenenfalls substituiert ist mit 1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
wobei p die Zahlen 0 bis 10, q die Zahlen 0 oder 1 und
R^{F} einen geradkettigen oder verzweigten Perfluoralkylrest mit 4 bis 30 Kohlenstoffatomen darstellt, und
Z¹ unabhängig voneinander ein Wasserstoffatom oder ein Metallionenäquivalent der Ordnungszahlen 21 - 29, 39, 42, 44 oder 57 - 83 bedeutet.

17. Formulierung gemäß Anspruch 4, **dadurch gekennzeichnet, daß** der Metallkomplex M ein Komplex der allgemeinen Formel XIII ist 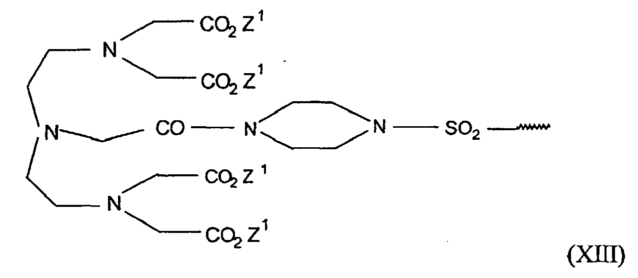 in der
Z¹ unabhängig voneinander ein Wasserstoffatom oder ein Metallionenäquivalent der Ordnungszahlen 21 - 29, 39, 42, 44 oder 57 - 83 bedeutet.

18. Formulierung gemäß Anspruch 1, **dadurch gekennzeichnet, daß** das Molekülteil A die folgende Struktur aufweist: 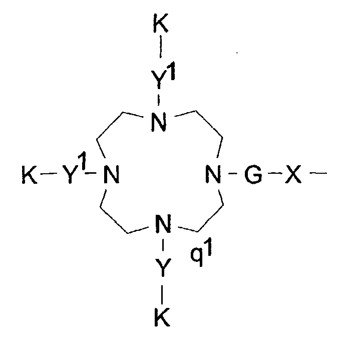 wobei
• q¹ eine Zahl 0, 1, 2 oder 3 ist,
• K für einen Komplexbildner oder Metallkomplex oder deren Salze organischer und/oder anorganischer Basen oder Aminosäuren oder Aminosäureamide steht,
• X eine direkte Bindung zur Perfluoralkylgruppe, eine Phenylengruppe oder eine C₁-C₁₀-Alkylenkette ist, die gegebenenfalls 1-15 Sauerstoff-, 1-5 Schwefelatome,
1 - 10 Carbonyl-, 1 - 10 (NR)-, 1 - 2 NRSO₂-, 1 - 10 CONR-, 1 Piperidin-, 1 - 3 SO₂-, 1 - 2 Phenylengruppen enthält oder gegebenenfalls durch 1 - 3 Reste R^{F} substituiert ist, worin R für ein Wasserstoffatom, eine Phenyl-, Benzyl- oder eine C₁-C₁₅-Alkylgruppe steht, die gegebenenfalls 1 - 2 NHCO-, 1 - 2 CO-Gruppen,
1 - 5 Sauerstoffatome enthält und gegebenenfalls durch 1 - 5 Hydroxy-, 1 - 5 Methoxy-, 1 - 3 Carboxy-, 1 - 3 R^{F}-Reste substituiert ist
• Y¹ eine direkte Bindung oder eine Kette der allgemeinen Formel II¹ oder III¹ ist: 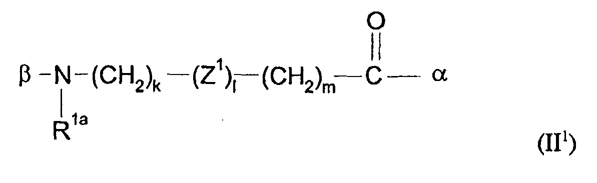 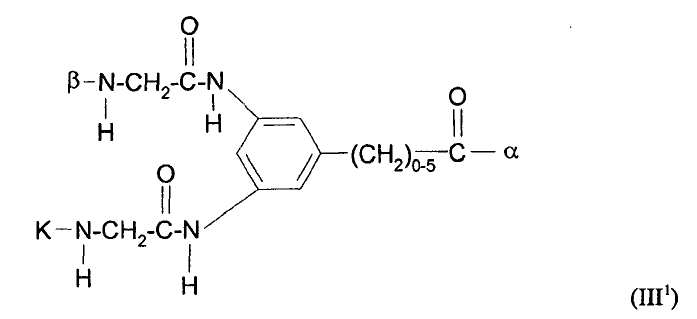 worin
■ R^{1a} ein Wasserstoffatom, eine Phenylgruppe, eine Benzylgruppe oder eine C₁-C₇
Alkylgruppe ist, die gegebenenfalls substituiert ist mit einer Carboxy-, einer Methoxy- oder einer Hydroxygruppe,
■ Z¹ eine direkte Bindung, eine Polyglycolethergruppe mit bis zu 5 Glycoleinheiten oder ein
Molekülteil der allgemeinen Formel IV¹ ist
-CH(R^{2a})- (IV¹)
worin R^{2a} eine C₁-C₇-Carbonsäure, eine Phenylgruppe, eine Benzylgruppe oder eine -(CH₂)₁₋₅-NH-K-Gruppe ist,
■ α die Bindung an das Stickstoffatom der Gerüstkette, β die Bindung zum Komplexbildner oder Metallkomplex K darstellt,
■ und in der die Variablen k und m für natürliche Zahlen zwischen 0 und 10 und 1 für 0 oder 1 stehen,
und wobei
• G eine CO- oder SO₂-Gruppe ist.

19. Formulierung gemäß Anspruch 4, worin der Linker L für ein Molekülteil gemäß allgemeiner Formel XIV steht 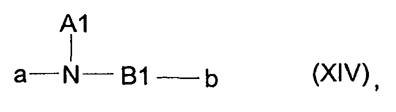 worin
N ein Stickstoffatom darstellt,
A1 ein Wasserstoffatom, eine geradkettige oder verzweigte C₁-C₃₀-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 -OR⁴-Gruppen, mit R⁴ in der Bedeutung eines Wasserstoffatoms oder eines C₁-C₇-Alkylrestes, oder -B1-R^{F} bedeutet,
B1 eine geradkettige oder verzweigte C₁-C₃₀-Alkylengruppe, die gegebenenfalls unterbrochen ist durch 1-10 Sauerstoffatome, 1-5 -NH-CO- Gruppen, 1-5 -CO-NH- Gruppen, durch eine (gegebenenfalls durch eine COOH-Gruppe substituierte) Phenylengruppe, 1-3 Schwefelatome,
1-2 -N(B2)-SO₂- Gruppen, und/oder 1-2 -SO₂-N(B2)- Gruppen mit B2 in der Bedeutung von A1, eine NHCO-Gruppe, eine CONH-Gruppe, eine N(B2)-SO₂-Gruppe, oder eine -SO₂-N(B2)- Gruppe und/oder gegebenenfalls substituiert ist mit dem Rest R^{F},
bedeutet,
und worin a die Bindung zum Metallkomplex M und b die Bindung zur Perfluoralkylgruppe R^{F} darstellt.

20. Formulierung gemäß Anspruch 4, **dadurch gekennzeichnet, daß** der Metallkomplex M für einen Metallkomplex der allgemeinen Formel XV steht 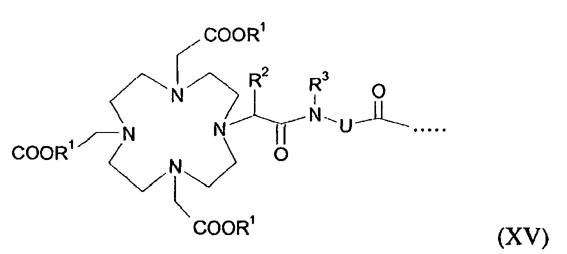 wobei R¹ für ein Wasserstoffatom oder ein Metallionenäquivalent der Ordnungszahlen 21-29, 31, 32, 37-39, 42-44, 49 oder 57-83,
R² und R³ für ein Wasserstoffatom, eine C₁-C₇-Alkylgruppe, eine Benzylgruppe, eine Phenylgruppe, -CH₂OH oder -CH₂-OCH₃ steht,
U für den Rest L steht, wobei aber L und U unabhängig voneinander gleich oder verschieden sein können, und
der Linker L für ein Molekülteil gemäß allgemeiner Formel XIV steht 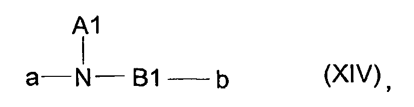 worin
N ein Stickstoffatom darstellt,
A1 ein Wasserstoffatom, eine geradkettige oder verzweigte C₁-C₃₀-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 -OR⁴-Gruppen, mit R⁴ in der Bedeutung eines Wasserstoffatoms oder eines C₁-C₇-Alkylrestes, oder -B1-R^{F} bedeutet,
B1 eine geradkettige oder verzweigte C₁-C₃₀-Alkylengruppe, die gegebenenfalls unterbrochen ist durch 1-10 Sauerstoffatome, 1-5 -NH-CO- Gruppen, 1-5 -CO-NH- Gruppen, durch eine (gegebenenfalls durch eine COOH-Gruppe substituierte) Phenylengruppe, 1-3 Schwefelatome,
1-2 -N(B2)-SO₂- Gruppen, und/oder 1-2 -SO₂-N(B2)- Gruppen mit B2 in der Bedeutung von A1, eine NHCO-Gruppe, eine CONH-Gruppe, eine N(B2)-SO₂-Gruppe, oder eine -SO₂-N(B2)- Gruppe und/oder gegebenenfalls substituiert ist mit dem Rest R^{F},
bedeutet,
und worin a die Bindung zum Metallkomplex M und b die Bindung zur Perfluoralkylgruppe R^{F} darstellt.

21. Formulierung gemäß einem der vorangegangenen Ansprüche, **dadurch gekennzeichnet, daß** das Zentralatom des Metallkomplexes ein Gadoliniumatom (Ordnungszahl 64) ist.

22. Formulierung gemäß Anspruch 1, **dadurch gekennzeichnet, daß** der Linker L¹ eine direkte Bindung, eine -SO₂-Gruppe oder eine geradkettige oder verzweigte Kohlenstoffkette mit bis zu 20 Kohlenstoffatomen ist, welche mit einer oder mehreren
-OH, -COO⁻, -SO₃-Gruppen substituiert sein kann und/oder gegebenenfalls eine oder mehrere -O-, -S-, -CO-, -CONH-, -NHCO-, -CONR-, -NRCO-, -SO₂-, -PO₄⁻-, -NH-, -NR-Gruppen, einen Arylring oder ein Piperazin enthält, wobei R für einen C₁- bis C₂₀-Alkylrest steht, welcher wiederum ein oder mehrere O-Atome enthalten kann und/oder mit -COO⁻ oder SO₃-Gruppen substituiert sein kann.

23. Formulierung gemäß Anspruch 1, **dadurch gekennzeichnet, daß** die hydrophile Gruppe B² ein Mono- oder Disaccharid, eine oder mehrere benachbarte -COO⁻ oder -SO₃⁻-Gruppen, eine Dicarbonsäure, eine Isophthalsäure, eine Picolinsäure, eine Benzolsulfonsäure, eine Tetrahydropyrandicarbonsäure, eine 2,6-Pyridindicarbonsäure, ein quartäres Ammoniumion, eine Aminopolycarbonsäure, eine Aminodipolyethylenglycolsulfonsäure, eine Aminopolyethylenglycolgruppe, eine SO₂-(CH₂)₂-OH-Gruppe, eine Polyhydroxyalkylkette mit mindestens zwei Hydroxylgruppen oder eine oder mehrere Polyethylenglycolketten mit mindestens zwei Glycoleinheiten ist, wobei die Polyethylenglycolketten durch eine -OH oder-OCH₃-Gruppe terminiert sind.

24. Formulierung gemäß Anspruch 1, **dadurch gekennzeichnet, daß** die Perfluoralkylketten der perfluoralkylhaltigen Metallkomplexe und der anderen perfluoralkylhaltigen Verbindungen 6 bis 12 Kohlenstoffatome enthalten.

25. Formulierung gemäß Anspruch 24, **dadurch gekennzeichnet, daß** die Perfluoralkylketten jeweils 8 Kohlenstoffatome enthalten.

26. Formulierung gemäß Anspruch 1, **dadurch gekennzeichnet, daß** sie eine Metallkonzentration von 50 bis 250 mmol/l aufweist.

27. Formulierung gemäß Anspruch 1, **dadurch gekennzeichnet, daß** die hydrophile Gruppe B² zusammen mit dem Linker L¹ ein Rest ausgewählt aus der Gruppe der folgenden Reste ist (n ist dabei eine Zahl zwischen 1 und 10):

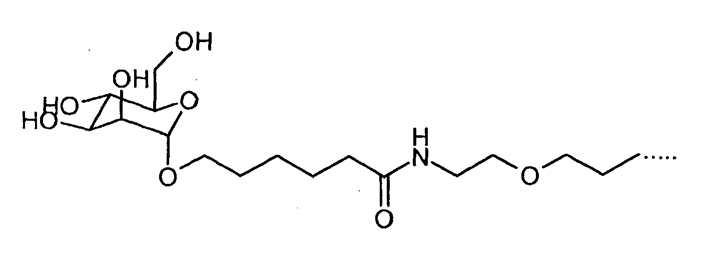

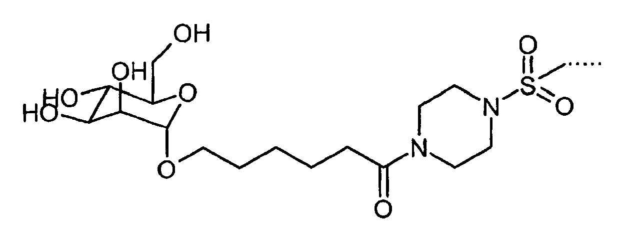

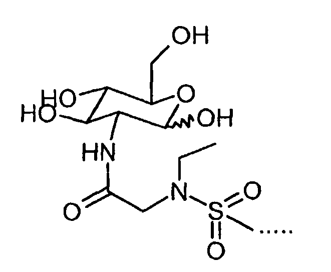

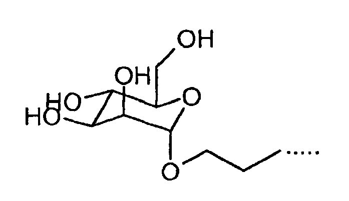

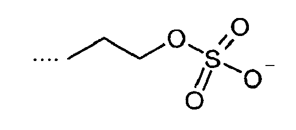

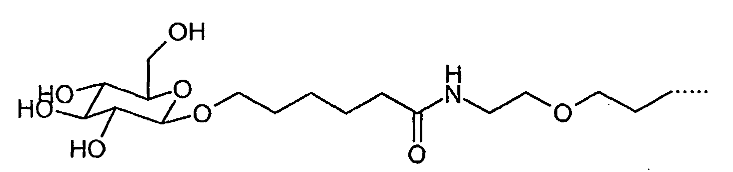

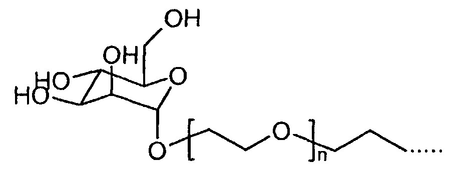

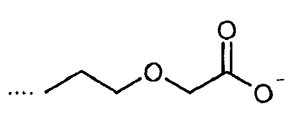

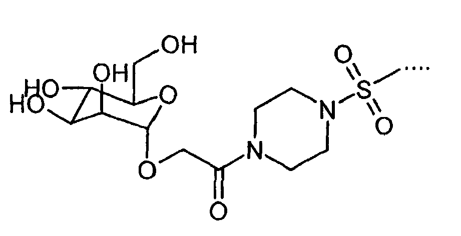

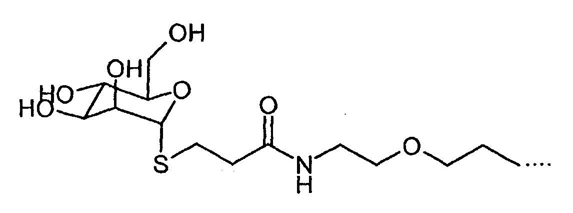

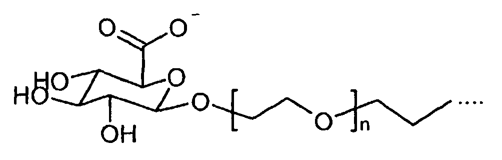

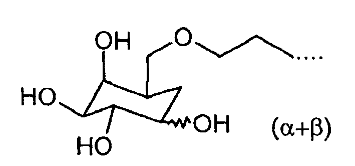

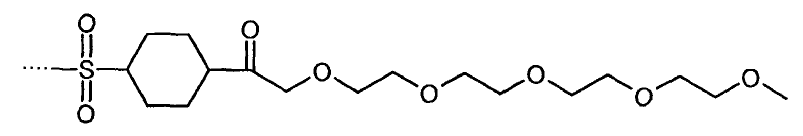

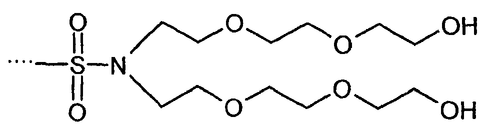

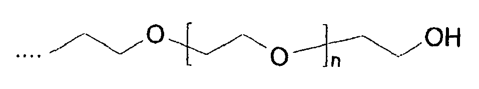

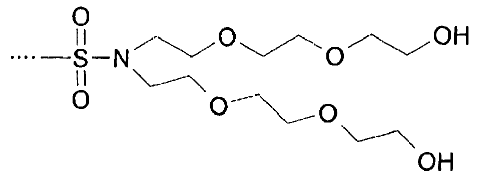

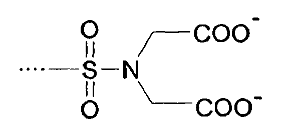

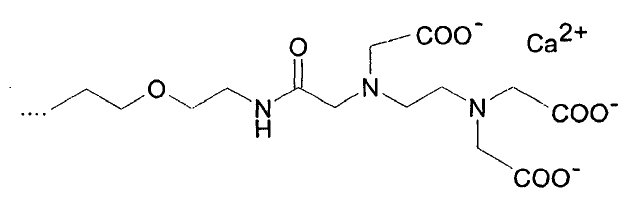

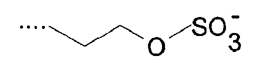

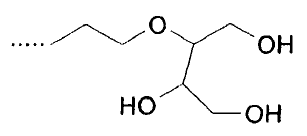

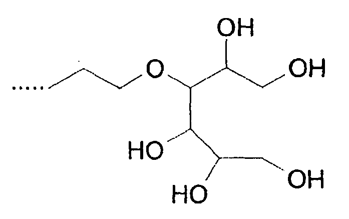

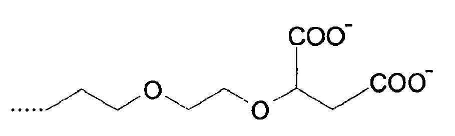

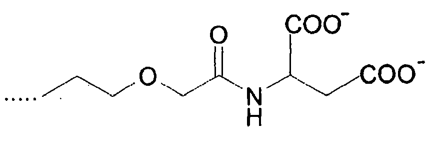

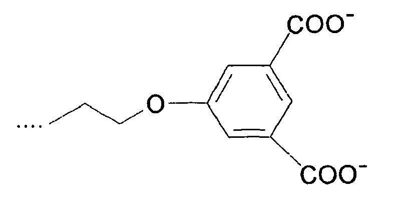

28. Formulierung gemäß Anspruch 1, **dadurch gekennzeichnet, daß** die hydrophile Gruppe B² ein Konjugat aus α, β oder γ-Cyclodextrin und einem Adamantan-, Biphenyl- oder Anthracenmolekül ist.

29. Verfahren zur Herstellung von galenischen Formulierungen gemäß Anspruch 1, **dadurch gekennzeichnet, daß** die paramagnetischen und diamagnetischen perfluoralkylhaltigen Verbindungen in einem Lösungsmittel unter starkem Rühren gelöst werden.

30. Verfahren zur Herstellung von galenischen Formulierungen gemäß Anspruch 1, **dadurch gekennzeichnet, daß** die paramagnetischen und diamagnetischen perfluoralkylhaltigen Verbindungen in einem Lösungsmittel unter gleichzeitiger Behandlung mit Ultraschall gelöst werden.

31. Verfahren zur Herstellung von galenischen Formulierungen gemäß Anspruch 1, **dadurch gekennzeichnet, daß** die paramagnetischen und diamagnetischen perfluoralkylhaltigen Verbindungen in einem Lösungsmittel unter gleichzeitiger Behandlung mit Mikrowellen gelöst werden.

32. Verfahren zur Herstellung von galenischen Formulierungen gemäß Anspruch 1, **dadurch gekennzeichnet, daß** die paramagnetischen und diamagnetischen perfluoralkylhaltigen Verbindungen in zwei verschiedenen Lösungsmitteln gelöst werden, beide Lösungen zusammengegeben werden und eines der beiden Lösungsmittel abdestilliert wird.

33. Feste Formulierung gemäß Anspruch 1, **dadurch gekennzeichnet, daß** sie durch Gefriertrocknen einer Lösung hergestellt wird, welche paramagnetische und diamagnetische perfluoralkylhaltige Substanzen enthält.

34. Verwendung von galenischen Formulierungen gemäß Anspruch 1 zur Herstellung von Kontrastmitteln für die Kernspintomographie.

35. Verwendung von galenischen Formulierungen gemäß Anspruch 1 zur Herstellung von Kontrastmitteln zur Darstellung der Lymphknoten oder des blood-pools.

36. Konjugate bestehend aus α-, β- oder γ-Cyclodextrin und Verbindungen der allgemeinen Formel XVIII
A¹-L³-R^{F} (XVIII)
worin A¹ für ein Adamantan-, Biphenyl- oder Anthracenmolekül, L³ für einen Linker und R^{F} für einen geradkettigen oder verzweigten Perfluoralkylrest mit 4 bis 30 Kohlenstoffatomen steht; und wobei der Linker L³ eine geradkettige Kohlenwasserstoffkette mit 1 bis 20 Kohlenstoffatomen ist, welche durch ein oder mehrere Sauerstoffatome, ein oder mehrere CO-, SO₂-, CONH-, NHCO-, CONR-, NRCO-, NH-, NR-Gruppen oder ein Piperazin unterbrochen sein kann, wobei R ein C₁-C₅-Alkylrest ist.

## Claims

1. Galenical formulation, **characterized in that** it contains paramagnetic perfluoroalkyl-containing compounds of general formula I
R^{F}-A (I)
where R^{F} represents a straight-chain or branched perfluoroalkyl radical with 4 to 30 carbon atoms and A is a moiety containing 1-6 metal complexes, and diamagnetic perfluoroalkyl-containing compounds of general formula XVI
R^{F}-L¹-B² (XVI)
where R^{F} represents a straight-chain or branched perfluoroalkyl radical with 4 to 30 carbon atoms, L¹ stands for a linker and B² stands for a hydrophilic group.

2. Formulation according to Claim 1, **characterized in that** the ratio of the paramagnetic to the diamagnetic perfluoroalkyl-containing compounds is between 5:95 and 95:5.

3. Formulation according to Claim 1, **characterized in that** the paramagnetic and diamagnetic perfluoroalkyl-containing compounds are dissolved in an aqueous solvent.

4. Formulation according to Claim 1, **characterized in that** the moiety A stands for a group L-M, where L stands for a linker and M stands for a metal complex consisting of an open-chain or cyclic chelating agent, which contains an atom of atomic numbers 21-29, 39, 42, 44 or 57-83 as a central atom.

5. Formulation according to Claim 4, **characterized in that** the linker L is a direct bond, a methylene group, an -NHCO group, a group 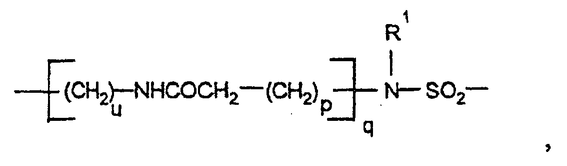 where p means the numbers 0 to 10, q and u, independently of one another, mean the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 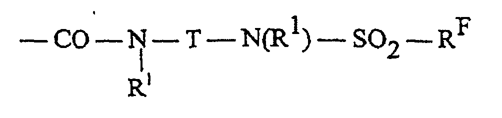 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
R^{F} represents a straight-chain or branched perfluoroalkyl radical with 4 to 30 carbon atoms, and
T means a C₂-C₁₀ chain, which optionally is interrupted by 1 to 2 oxygen atoms or 1 to 2 -NHCO groups.

6. Formulation according to Claim 4, **characterized in that** the metal complex M stands for a complex of general formula II 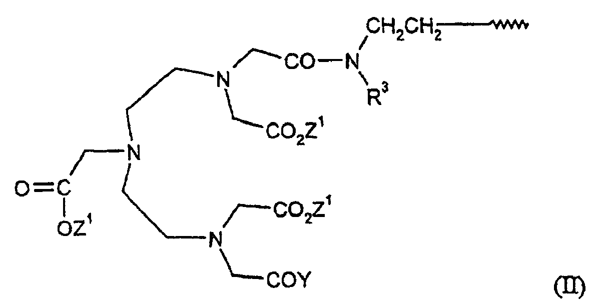 in which R³, Z¹ and Y are independent of one another and
R³ has the meaning of R¹ or -(CH₂)ₘ-L-R^{F}, where m is 0, 1 or 2, and
L is a direct bond, a methylene group, an -NHCO group, a group 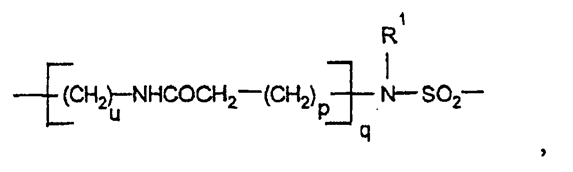 where p means the numbers 0 to 10, q and u, independently of one another, mean the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 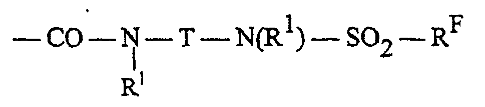 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
R^{F} represents a straight-chain or branched perfluoroalkyl radical with 4 to 30 carbon atoms,
T means a C₂-C₁₀ chain, which optionally is interrupted by 1 to 2 oxygen atoms or 1 to 2 -NHCO groups,
Z¹, independently of one another, mean a hydrogen atom or a metal ion equivalent of atomic numbers 21-29, 39, 42, 44 or 57-83,
Y means -OZ¹, or

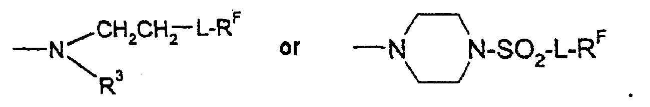

7. Formulation according to Claim 4, **characterized in that** the metal complex M stands for a complex of general formula III 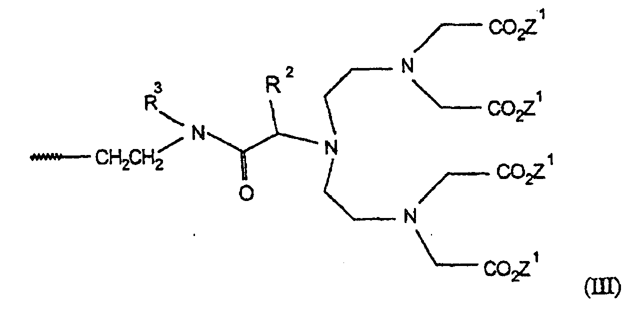 in which R³ and Z¹ are independent of one another and
R³ has the meaning of R¹ or - (CH₂)ₘ-L-R^{F}, where
m is 0, 1 or 2 and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
L is a direct bond, a methylene group, an -NHCO group, a group 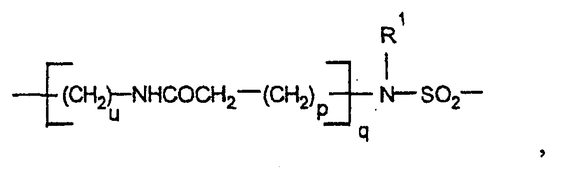 where p means the numbers 0 to 10, q and u, independently of one another, mean the numbers 0 or 1, and
R¹ means a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 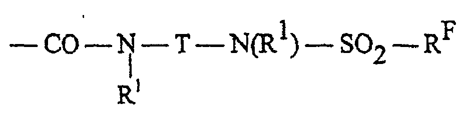 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
R^{F} represents a straight-chain or branched perfluoroalkyl radical with 4 to 30 carbon atoms,
T means a C₂-C₁₀ chain, which optionally is interrupted by 1 to 2 oxygen atoms or 1 to 2 -NHCO groups, and
Z¹, independently of one another, mean a hydrogen atom or a metal ion equivalent of atomic numbers 21-29, 39, 42, 44 or 57-83, and
R² has the meaning of R¹.

8. Formulation according to Claim 4, **characterized in that** the metal complex M stands for a metal complex of general formula IV 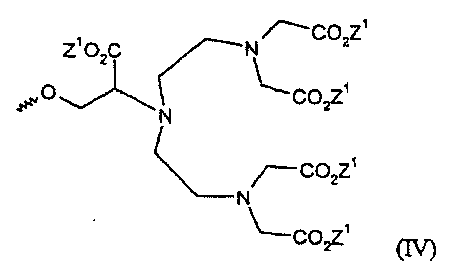 in which Z¹, independently of one another, means a hydrogen atom or a metal ion equivalent of atomic numbers 21-29, 39, 42, 44 or 57-83.

9. Formulation according to Claim 4, **characterized in that** the metal complex M stands for a metal complex of general formula V 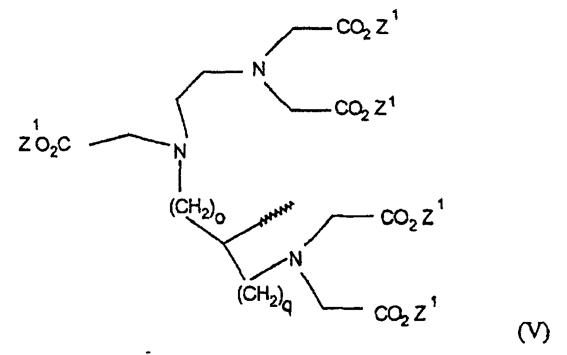 where Z¹, independently of one another, means a hydrogen atom or a metal ion equivalent of atomic numbers 21-29, 39, 42, 44 or 57-83 and o and q stand for the numbers 0 or 1 and the sum o + q = 1.

10. Formulation according to Claim 4, **characterized in that** the metal complex M stands for a metal complex of general formula VI 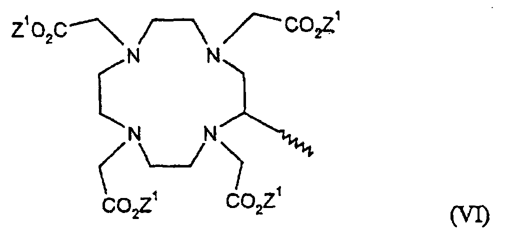 where Z¹, independently of one another, mean a hydrogen atom or a metal ion equivalent of atomic numbers 21-29, 39, 42, 44 or 57-83.

11. Formulation according to Claim 4, **characterized in that** the metal complex M stands for a metal complex of general formula VII 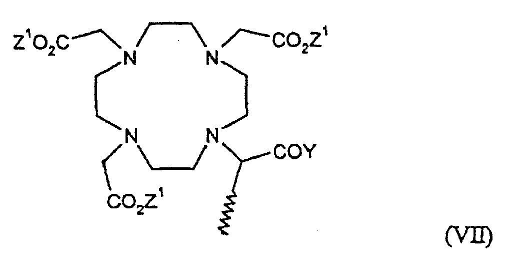 where Z¹, independently of one another, means a hydrogen atom or a metal ion equivalent of atomic numbers 21-29, 39, 42, 44 or 57-83 and
Y means -OZ¹ or 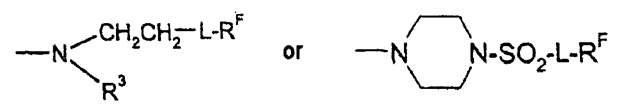 where
L means a direct bond, a methylene group, an -NHCO group, a group 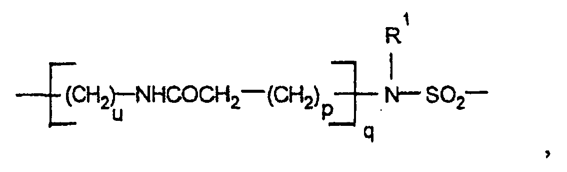 where p means the numbers 0 to 10, q and u, independently of one another, mean the numbers 0 or 1, and
R¹ means a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 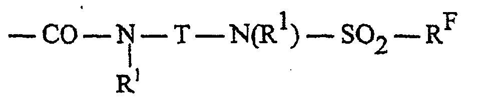 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
R^{F} represents a straight-chain or branched perfluoroalkyl radical with 4 to 30 carbon atoms,
T means a C₂-C₁₀ chain, which optionally is interrupted by 1 to 2 oxygen atoms or 1 to 2 -NHCO groups, and
R³ has the meaning of R¹ or -(CH₂)ₘ-L-R^{F}, where
m is 0, 1 or 2.

12. Formulation according to Claim 4, **characterized in that** the metal complex M is a complex of general formula VIII 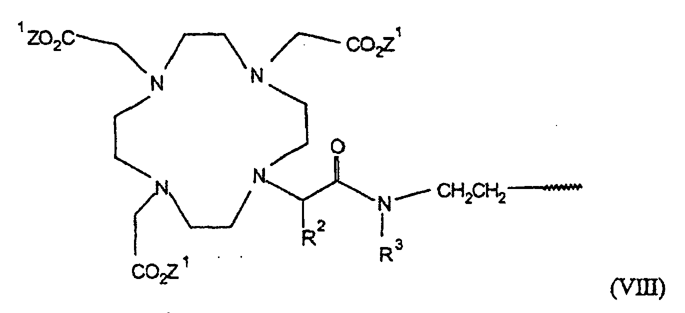 in which R³ and Z¹ are independent of one another and
R³ has the meaning of R¹ or -(CH₂)ₘ-L-R^{F}, where
m is 0, 1 or 2 and
L is a direct bond, a methylene group, an -NHCO group, a group 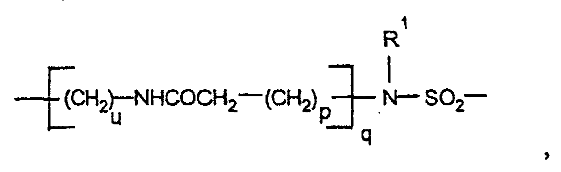 where p means the numbers 0 to 10, q and u, independently of one another, mean the numbers 0 or 1, and
R¹ means a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group; an -NR¹CO group, an -SO₂ group, an -NR¹-CO₂ group, 1 to 2 -CO groups, a group 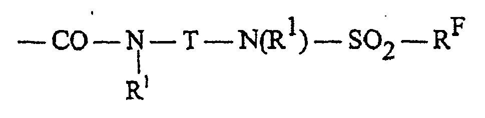 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
R^{F} represents a straight-chain or branched perfluoroalkyl radical with 4 to 30 carbon atoms,
T means a C₂-C₁₀ chain, which optionally is interrupted by 1 to 2 oxygen atoms or 1 to 2 -NHCO groups, and
Z¹, independently of one another, mean a hydrogen atom or a metal ion equivalent of atomic numbers 21-29, 39, 42, 44 or 57-83, and
R² has the meaning of R¹.

13. Formulation according to Claim 4, **characterized in that** the metal complex M is a complex of general formula IX 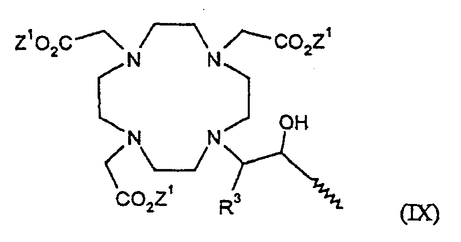 in which
Z¹, independently of one another, mean a hydrogen atom or a metal ion equivalent of atomic numbers 21-29, 39, 42, 44 or 57-83, and
R³ has the meaning of R¹ or -(CH₂)ₘ-L-R^{F}, where
m is 0, 1 or 2 and
L is a direct bond, a methylene group, an -NHCO group, a group 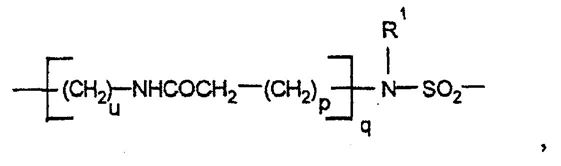 where p means the numbers 0 to 10, q and u, independently of one another, mean the numbers 0 or 1, and
R¹ means a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 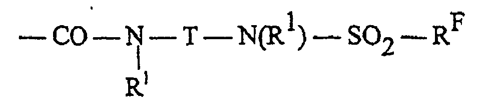 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
R^{F} represents a straight-chain or branched perfluoroalkyl radical with 4 to 30 carbon atoms, and
T means a C₂-C₁₀ chain, which optionally is interrupted by 1 to 2 oxygen atoms or 1 to 2 -NHCO groups.

14. Formulation according to Claim 4, **characterized in that** the metal complex M is a complex of general formula X 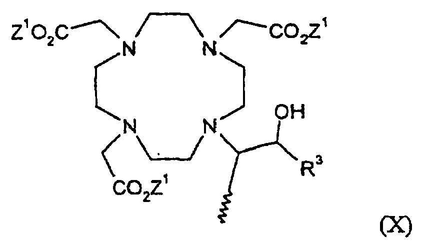 in which
Z¹, independently of one another, mean a hydrogen atom or a metal ion equivalent of atomic numbers 21-29, 39, 42, 44 or 57-83, and
R³ has the meaning of R¹ or -(CH₂)ₘ-L-R^{F}, where
m is 0, 1 or 2 and
L is a direct bond, a methylene group, an -NHCO group, a group 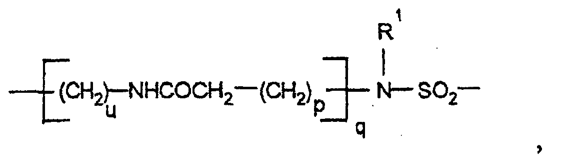 where p means the numbers 0 to 10, q and u, independently of one another, mean the numbers 0 or 1, and
R¹ means a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 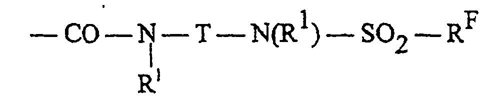 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1, and
R¹ is a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups, and
R^{F} represents a straight-chain or branched perfluoroalkyl radical with 4 to 30 carbon atoms, and
T means a C₂-C₁₀ chain, which optionally is interrupted by 1 to 2 oxygen atoms or 1 to 2 -NHCO groups.

15. Formulation according to Claim 4, **characterized in that** the metal complex M is a complex of general formula XI 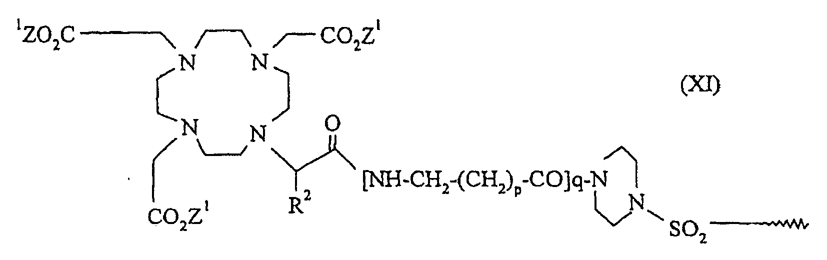 in which
Z¹, independently of one another, mean a hydrogen atom or a metal ion equivalent of atomic numbers 21-29, 39, 42, 44 or 57-83, and
p means the numbers 0 to 10, q means the numbers 0 or 1 and
R² means a hydrogen atom, a methyl group, a -CH₂-OH group, a CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups.

16. Formulation according to Claim 4, **characterized in that** the metal complex M is a complex of general formula XII 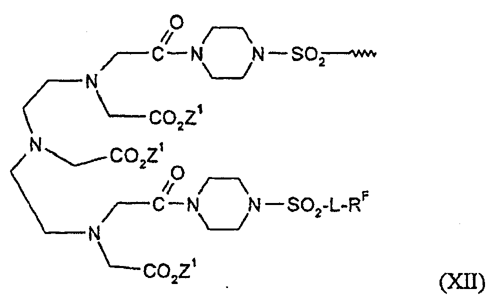 in which L is a direct bond, a methylene group, an
-NHCO group, a group 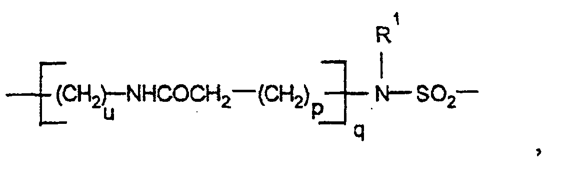 where p means the numbers 0 to 10, q and u, independently of one another, mean the numbers 0 or 1, and
R¹ means a hydrogen atom, a methyl group, a -CH₂-OH group, a -CH₂-CO₂H group or a C₂-C₁₅ chain, which optionally is interrupted by 1 to 3 oxygen atoms, 1 to 2 >CO groups or an optionally substituted aryl group and/or is substituted with 1 to 4 hydroxyl groups, 1 to 2 C₁-C₄ alkoxy groups, 1 to 2 carboxy groups,
or a straight-chain, branched, saturated or unsaturated C₂-C₃₀ carbon chain, which optionally contains 1 to 10 oxygen atoms, 1 to 3 -NR¹ groups, 1 to 2 sulphur atoms, a piperazine, a -CONR¹ group, an -NR¹CO group, an -SO₂ group, an -NR¹-CO₂ group, 1 to 2 -CO groups, a group 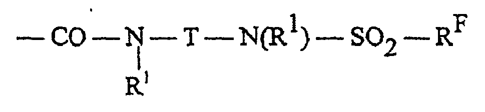 or 1 to 2 optionally substituted aryls and/or is interrupted by these groups and/or is optionally substituted with 1 to 3 -OR¹ groups, 1 to 2 oxo groups, 1 to 2 -NH-COR¹ groups, 1 to 2 -CONHR¹ groups, 1 to 2 -(CH₂)ₚ-CO₂H groups, 1 to 2 groups -(CH₂)ₚ-(O)_{q}-CH₂CH₂-R^{F},
where
p means the numbers 0 to 10, q means the numbers 0 or 1 and
R^{F} represents a straight-chain or branched perfluoroalkyl radical with 4 to 30 carbon atoms, and
Z¹, independently of one another, mean a hydrogen atom or a metal ion equivalent of atomic numbers 21-29, 39, 42, 44 or 57-83.

17. Formulation according to Claim 4, **characterized in that** the metal complex M is a complex of general formula XIII 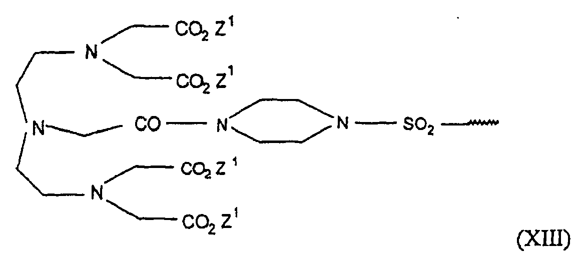 in which
Z¹, independently of one another, mean a hydrogen atom or a metal ion equivalent of atomic numbers 21-29, 39, 42, 44 or 57-83.

18. Formulation according to Claim 1, **characterized in that** the moiety A exhibits the following structure: 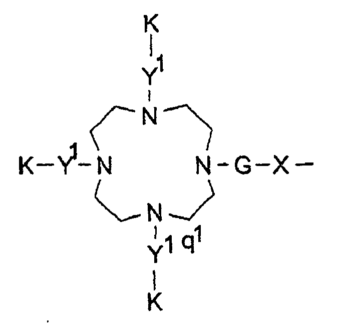 where
• q¹ is a number 0, 1, 2 or 3,
• K stands for a complexing agent or metal complex or salts thereof of organic and/or inorganic bases or amino acids or amino acid amides,
• X is a direct bond for the perfluoroalkyl group, a phenylene group or a C₁-C₁₀ alkylene chain, which optionally contains 1-15 oxygen atoms, 1-5 sulphur atoms,
1-10 carbonyl groups, 1-10 (NR) groups, 1-2 NRSO₂ groups, 1-10 CONR groups, 1 piperidine group, 1-3 SO₂ groups, 1-2 phenylene groups or optionally is substituted by 1-3 radicals R^{F}, in which R stands for a hydrogen atom, a phenyl, benzyl or a C₁-C₁₅ alkyl group, which optionally contains 1-2 NHCO groups, 1-2 CO groups,
1-5 oxygen atoms and optionally is substituted by 1-5 hydroxy, 1-5 methoxy, 1-3 carboxy, 1-3 R^{F} radicals,
• Y¹ is a direct bond or a chain of general formula II¹ or III¹: 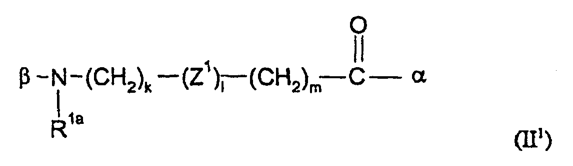 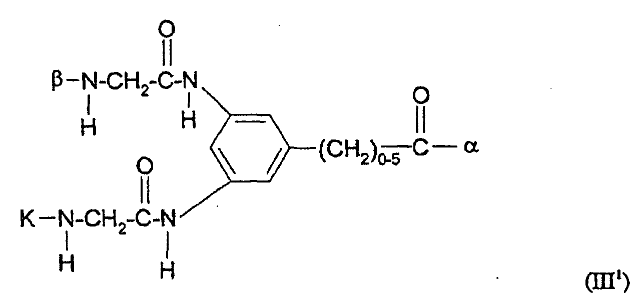 in which
■ R^{1a} is a hydrogen atom, a phenyl group, a benzyl group or a C₁-C₇ alkyl group, which optionally is substituted with a carboxy group, a methoxy group or a hydroxy group,
■ Z¹ is a direct bond, a polyglycol ether group with up to 5 glycol units or a moiety of general formula IV¹
-CH(R^{2a})- (IV¹)
in which R^{2a} is a C₁-C₇ carboxylic acid, a phenyl group, a benzyl group or a -(CH₂)₁₋₅-NH-K group,
■ α represents the binding to the nitrogen atom of the skeleton chain, β represents the binding to the complexing agent or metal complex K,
■ and in which variables k and m stand for natural numbers between 0 and 10, and 1 stands for 0 or 1,
and where
• G is a CO or SO₂ group.

19. Formulation according to Claim 4, wherein the linker L represents a moiety of general formula XIV 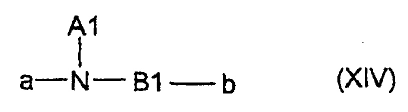 where
N represents a nitrogen atom,
A1 means a hydrogen atom, a straight-chain or branched C₁-C₃₀ alkyl group, which optionally is interrupted by 1-15 oxygen atoms and/or optionally is substituted with 1-10 hydroxy groups, 1-2 COOH groups, a phenyl group, a benzyl group and/or 1-5 -OR⁴ groups, with R⁴ in the meaning of a hydrogen atom or a C₁-C₇ alkyl radical, or -B1-R^{F},
B1 means a straight-chain or branched C₁-C₃₀ alkylene group that optionally is interrupted by 1-10 oxygen atoms, 1-5 -NH-CO groups, 1-5 -CO-NH groups, by a phenylene group (that is optionally substituted by a COOH group), 1-3 sulphur atoms, 1-2 -N(B2)-SO₂ groups, and/or 1-2 -SO₂-N(B2) groups with B2 in the meaning of A1, an NHCO group, a CONH group, an N(B2)-SO₂ group, or an -SO₂-N(B2) group and/or optionally is substituted with radical R^{F},
and in which a represents the binding to metal complex M, and b represents the binding to perfluoroalkyl group R^{F}.

20. Formulation according to Claim 4, **characterized in that** the metal complex M stands for a metal complex of general formula XV 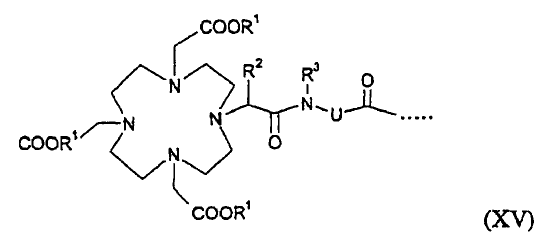 where
R¹ stands for a hydrogen atom or a metal ion equivalent of atomic numbers 21-29, 31, 32, 37-39, 42-44, 49 or 57-83,
R² and R³ stand for a hydrogen atom, a C₁-C₇ alkyl group, a benzyl group, a phenyl group, -CH₂OH or -CH₂-OCH₃,
U stands for radical L, where L and U, independently of one another, can be the same or different, however, and
the linker L stands for a moiety of general formula XIV 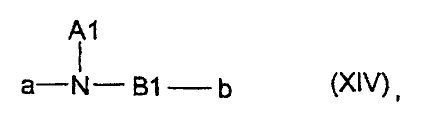 where
N represents a nitrogen atom,
A1 means a hydrogen atom, a straight-chain or branched C₁-C₃₀ alkyl group, which optionally is interrupted by 1-15 oxygen atoms and/or optionally is substituted with 1-10 hydroxy groups, 1-2 COOH groups, a phenyl group, a benzyl group and/or 1-5 -OR⁴ groups, with R⁴ in the meaning of a hydrogen atom or a C₁-C₇ alkyl radical, or -B1-R^{F},
B1 means a straight-chain or branched C₁-C₃₀ alkylene group that optionally is interrupted by 1-10 oxygen atoms, 1-5 -NH-CO groups, 1-5 -CO-NH groups, by a phenylene group (that is optionally substituted by a COOH group), 1-3 sulphur atoms, 1-2 -N(B2)-SO₂ groups, and/or 1-2 -SO₂-N(B2) groups with B2 in the meaning of A1, an NHCO group, a CONH group, an N(B2)-SO₂ group, or an -SO₂-N(B2) group and/or optionally is substituted with radical R^{F},
and in which a represents the binding to metal complex M, and b represents the binding to perfluoroalkyl group R^{F}.

21. Formulation according to any one of the preceding claims, **characterized in that** the central atom of the metal complex is a gadolinium atom (atomic number 64).

22. Formulation according to Claim 1, **characterized in that** the linker L¹ is a direct bond, an -SO₂ group or a straight-chain or branched carbon chain with up to 20 carbon atoms, which can be substituted with one or more -OH, -COO⁻, -SO₃ groups and/or optionally contains one or more -O-, -S-, -CO-, -CONH-, -NHCO-, -CONR-, -NRCO-, -SO₂-, -PO₄⁻-, -NH-, -NR groups, an aryl ring or a piperazine,
where R stands for a C₁ to C₂₀ alkyl radical, which in turn can contain one or more O atoms and/or can be substituted with -COO⁻ or SO₃ groups.

23. Formulation according to Claim 1, **characterized in that** the hydrophilic group B² is a mono- or disaccharide, one or more adjacent -COO⁻ or -SO₃⁻ groups, a dicarboxylic acid, an isophthalic acid, a picolinic acid, a benzenesulphonic acid, a tetrahydropyrandicarboxylic acid, a 2,6-pyridinedicarboxylic acid, a quaternary ammonium ion, an aminopolycarboxylic acid, an aminodipolyethylene glycol sulphonic acid, an aminopolyethylene glycol group, an SO₂-(CH₂)₂-OH group, a polyhydroxyalkyl chain with at least two hydroxyl groups or one or more polyethylene glycol chains with at least two glycol units, where the polyethylene glycol chains are terminated by an -OH or -OCH₃ group.

24. Formulation according to Claim 1, **characterized in that** the perfluoroalkyl chains of the perfluoroalkyl-containing metal complexes and of the other perfluoroalkyl-containing compounds contain 6 to 12 carbon atoms.

25. Formulation according to Claim 24, **characterized in that** the perfluoroalkyl chains each contain 8 carbon atoms.

26. Formulation according to Claim 1, **characterized in that** it has a metal concentration of 50 to 250 mmol/l.

27. Formulation according to Claim 1, **characterized in that** the hydrophilic group B² together with the linker L¹ is a radical selected from the group of the following radicals (in this case, n is a number between 1 and 10):

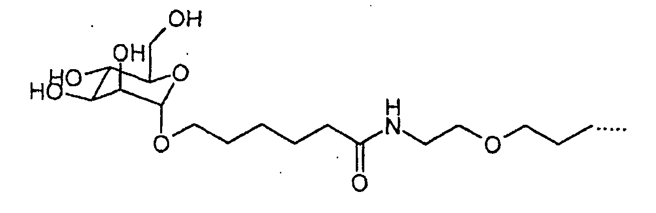

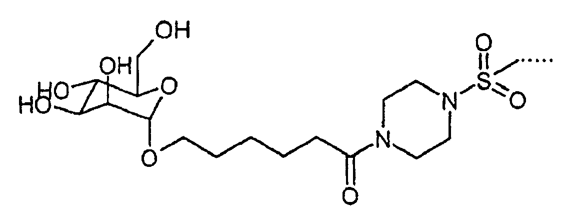

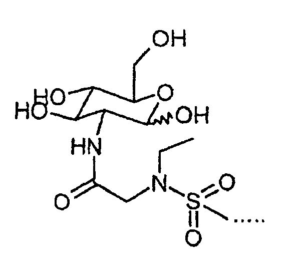

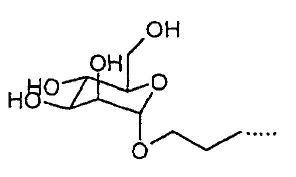

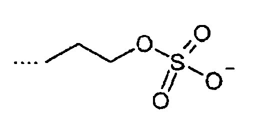

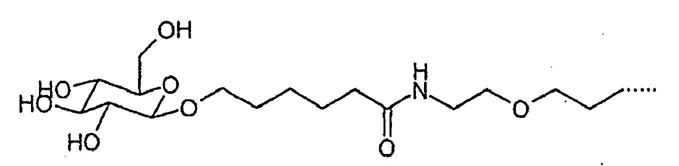

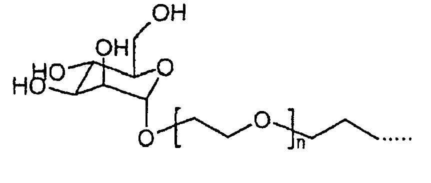

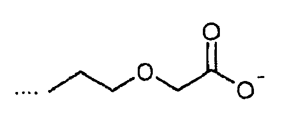

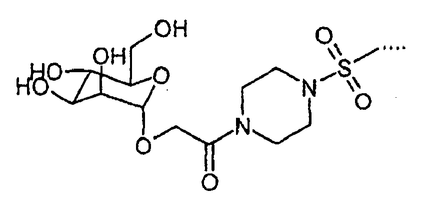

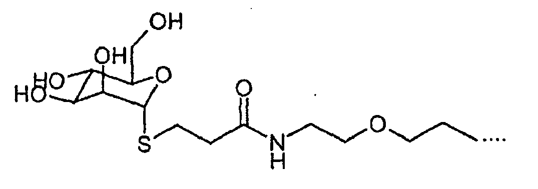

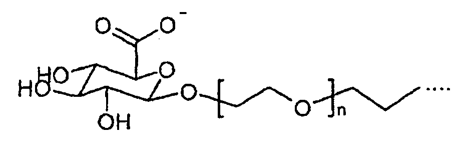

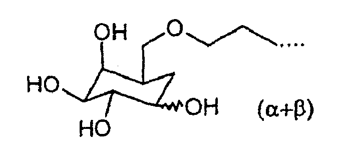

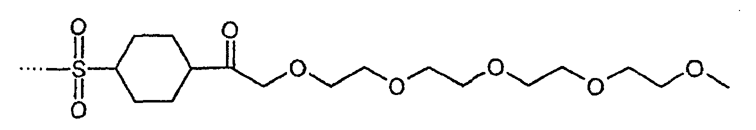

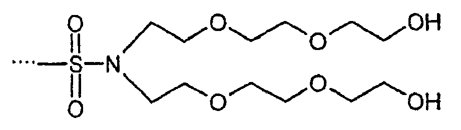

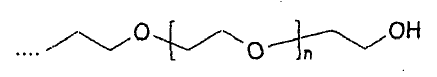

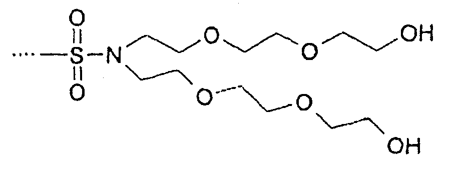

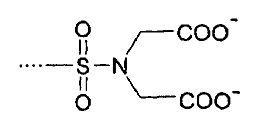

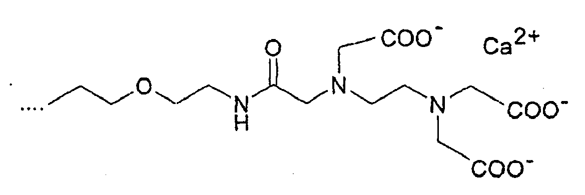

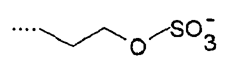

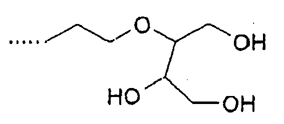

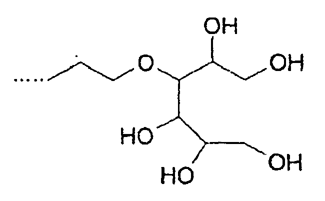

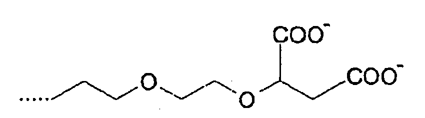

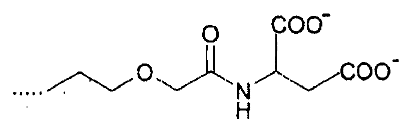

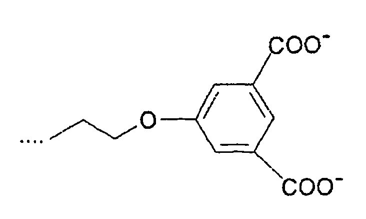

28. Formulation according to Claim 1, **characterized in that** the hydrophilic group B² is a conjugate of α-, β- or γ-cyclodextrin and an adamantane, biphenyl or anthracene molecule.

29. Process for preparing galenical formulations according to Claim 1, **characterized in that** the paramagnetic and diamagnetic perfluoroalkyl-containing compounds are dissolved in a solvent by vigorous stirring.

30. Process for preparing galenical formulations according to Claim 1, **characterized in that** the paramagnetic and diamagnetic perfluoroalkyl-containing compounds are dissolved in a solvent by concurrent treatment with ultrasound.

31. Process for preparing galenical formulations according to Claim 1, **characterized in that** the paramagnetic and diamagnetic perfluoroalkyl-containing compounds are dissolved in a solvent by concurrent treatment with microwaves.

32. Process for preparing galenical formulations according to Claim 1, **characterized in that** the paramagnetic and diamagnetic perfluoroalkyl-containing compounds are dissolved in two different solvents, the two solutions are combined and one of the two solvents is distilled off.

33. Solid formulation according to Claim 1, **characterized in that** it is prepared by freeze drying a solution containing paramagnetic and diamagnetic perfluoroalkyl-containing substances.

34. Use of galenical formulations according to Claim 1 for preparing contrast media for nuclear spin tomography.

35. Use of galenical formulations according to Claim 1 for preparing contrast media for visualizing the lymph nodes or the blood pool.

36. Conjugates consisting of α-, β- or γ-cyclodextrin and compounds of general formula XVIII
A¹-L³-R^{F} (XVIII)
where A¹ stands for an adamantane, biphenyl or anthracene molecule, L³ stands for a linker and R^{F} stands for a straight-chain or branched perfluoroalkyl radical with 4 to 30 carbon atoms; and where the linker R³ is a straight-chain hydrocarbon chain with 1 to 20 carbon atoms which can be interrupted by one or more oxygen atoms, one or more CO-, SO₂-, CONH-, NHCO-, CONR-, NRCO-, NH-, NR groups or a piperazine, where R is a C₁-C₅ alkyl radical.

## Revendications

1. Composition galénique, **caractérisée en ce qu'**elle contient des composés perfluoroalkylés paramagnétiques de formule générale I
R^{F}-A (I),
dans laquelle R^{F} représente un radical perfluoroalkyle à chaîne droite ou ramifiée, ayant de 4 à 30 atomes de carbone et A est un fragment qui contient 1-6 complexes métalliques,
et des composés perfluoroalkylés diamagnétiques de formule générale XVI
R^{F}-L¹-B² (XVI),
dans laquelle R^{F} représente un radical perfluoroalkyle à chaîne droite ou ramifiée, ayant de 4 à 30 atomes de carbone, L¹ représente un chaînon de liaison et B² un groupe hydrophile.

2. Composition selon la revendication 1, **caractérisée en ce que** le rapport des composés perfluoroalkylés paramagnétiques aux composés perfluoroalkylés diamagnétiques est compris entre 5:95 et 95:5.

3. Composition selon la revendication 1, **caractérisée en ce que** les composés perfluoroalkylés paramagnétiques et les composés perfluoroalkylés diamagnétiques sont présents en solution dans un solvant aqueux.

4. Composition selon la revendication 1, **caractérisée en ce que** la partie de la molécule A représente un groupe L-M, L représentant un chaînon de liaison et M représentant un complexe métallique consistant en un agent chélateur à chaîne ouverte ou cyclique, qui contient en tant qu'atome central un atome des nombres atomiques 21-29, 39, 42, 44 ou 57-83.

5. Composition selon la revendication 4, **caractérisée en ce que** L est une liaison directe, un groupe méthylène, un groupe -NHCO-, un groupe 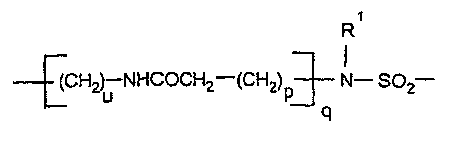 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹-CO₂, 1 ou 2 groupes -CO, un groupe 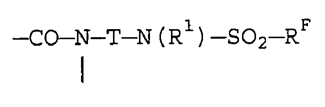 ou 1 ou 2 groupes aryle
éventuellement substitué(s) et/ou est interrompue par ces groupes,
et/ou est éventuellement substituée par 1 à 3 groupes -OR¹, 1 ou 2 groupes oxo, 1 ou 2 groupes -NH-COR¹, 1 ou 2 groupes -CONHR¹, ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R¹ étant un atome d'hydrogène, un groupe méthyle, un groupe -CH₂-OH, un groupe -CH₂CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
R^{F} représentant un radical perfluoroalkyle à chaîne droite ou ramifiée, ayant de 4 à 30 atomes de carbone, et
T représentant une chaîne en C₂-C₁₀ qui est éventuellement interrompue par 1 ou 2 atomes d'oxygène ou 1 ou 2 groupes -NHCO,

6. Composition selon la revendication 4, **caractérisée en ce que** le complexe métallique M représente un complexe de formule générale II 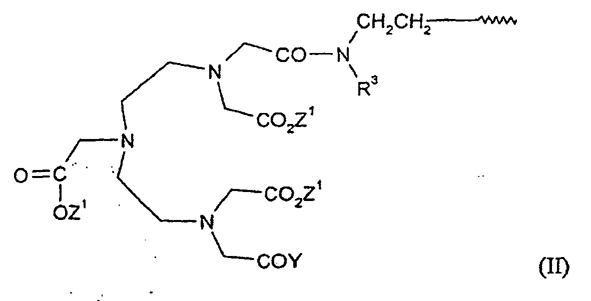 dans laquelle R³, Z¹ et Y sont indépendants les uns des autres et
R³ a la signification de R¹ ou représente un groupe -(CH₂)ₘ-L-R^{F}, où
m est 0, 1 ou 2, et
L est une liaison directe, un groupe méthylène, un groupe -NHCO-, un groupe 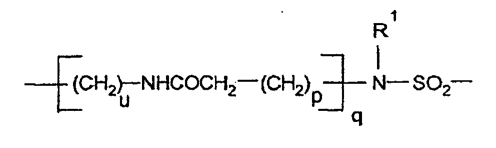 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹CO₂, 1 ou 2 groupes -CO, un groupe 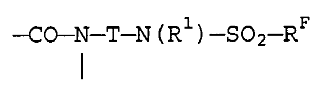 ou 1 ou 2 groupes aryle
éventuellement substitué(s) et/ou est interrompue par ces groupes,
et/ou est éventuellement substituée par 1 à 3 groupes -OR¹, 1 ou 2 groupes oxo, 1 ou 2 groupes -NH-COR¹, 1 ou 2 groupes -CONHR¹, ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R¹ étant un atome d'hydrogène, un groupe méthyle, un groupe -CH₂-OH, un groupe -CH₂CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
R^{F} représentant un radical perfluoroalkyle à chaîne droite ou ramifiée, ayant de 4 à 30 atomes de carbone, et
T représentant une chaîne en C₂-C₁₀ qui est éventuellement substitués par 1 ou 2 atomes d'oxygène ou 1 ou 2 groupes -NHCO,
Z¹ représentent chacun indépendamment un atome d'hydrogène ou un équivalent d'ion métallique des nombres atomiques 21-29, 39, 42, 44 ou 57-83,
Y représente -OZ¹ ou

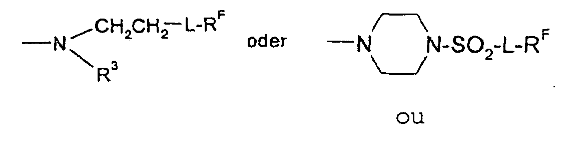

7. Composition selon la revendication 4, **caractérisée en ce que** le complexe métallique M représente un complexe de formule générale III 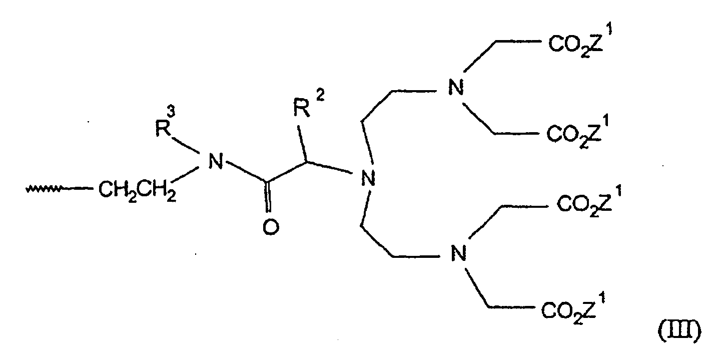 dans laquelle R³, Z¹ sont indépendants les uns des autres et
R³ a la signification de R¹ ou représente un groupe -(CH₂)ₘ-L-R^{F}, où
m est 0, 1 ou 2, et
R¹ est un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
L est une liaison directe, un groupe méthylène, un groupe -NHCO-, un groupe 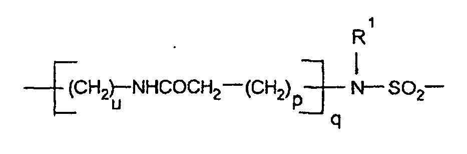 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹-CO₂, 1 ou 2 groupes -CO, un groupe 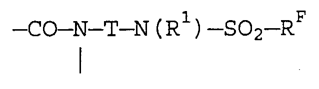 ou 1 ou 2 groupes aryle
éventuellement substitué(s) et/ou est interrompue par ces groupes,
et/ou est éventuellement substituée par 1 à 3 groupes -OR¹, 1 ou 2 groupes oxo, 1 ou 2 groupes -NH-COR¹, 1 ou 2 groupes -CONHR¹, 1 ou 2 groupes -(CH₂)ₚ-CO₂H, 1 ou 2 groupes -(CH₂)ₚ-(O)_{q}-CH₂CH₂-R^{F},
R^{F} représentant un radical perfluoroalkyle à chaîne droite ou ramifiée, ayant de 4 à 30 atomes de carbone,
T représentant une chaîne en C₂-C₁₀ qui est éventuellement interrompue par 1 ou 2 atomes d'oxygène ou 1 ou 2 groupes -NHCO,
Z¹ représentent chacun indépendamment un atome d'hydrogène ou un équivalent d'ion métallique des nombres atomiques 21-29, 39, 42, 44 ou 57-83,
et R² a la signification de R¹.

8. Composition selon la revendication 4, **caractérisée en ce que** le complexe métallique M représente un complexe métallique de formule générale IV 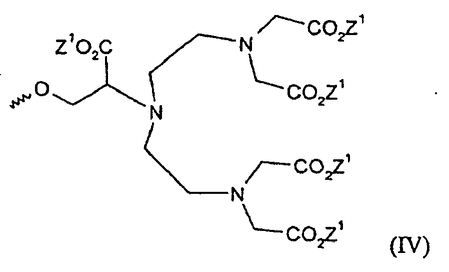 Z¹ représentant chacun indépendamment un atome d'hydrogène ou un équivalent d'ion métallique des nombres atomiques 21-29, 39, 42, 44 ou 57-83,

9. Composition selon la revendication 4, **caractérisée en ce que** le complexe métallique M représente un complexe métallique de formule générale V 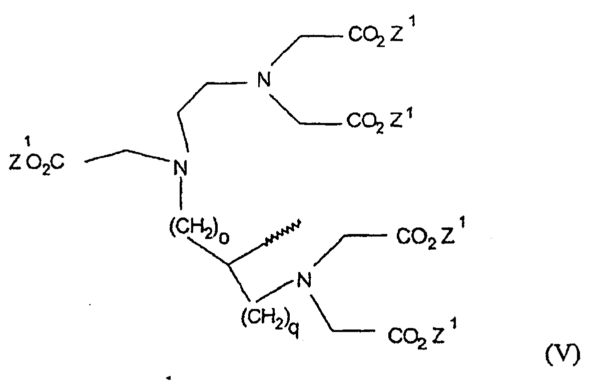 Z¹ représentant chacun indépendamment un atome d'hydrogène ou un équivalent d'ion métallique des nombres atomiques 21-29, 39, 42, 44 ou 57-83, et o et q représentant le chiffre 0 ou 1 et la somme o + q étant égale à 1.

10. Composition selon la revendication 4, **caractérisée en ce que** le complexe métallique M représente un complexe métallique de formule générale VI 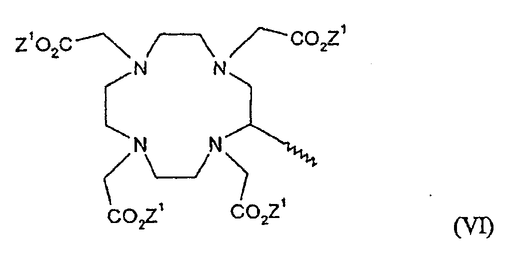 Z¹ représentant chacun indépendamment un atome d'hydrogène ou un équivalent d'ion métallique des nombres atomiques 21-29, 39, 42, 44 ou 57-83,

11. Composition selon la revendication 4, **caractérisée en ce que** le complexe métallique M représente un complexe métallique de formule générale VII 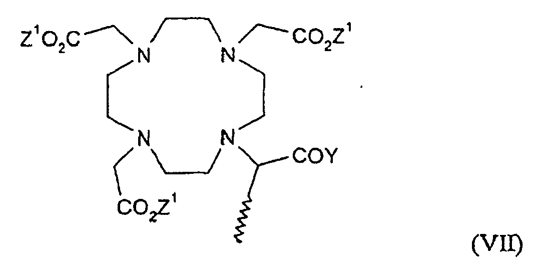 Z¹ représentant chacun indépendamment un atome d'hydrogène ou un équivalent d'ion métallique des nombres atomiques 21-29, 39, 42, 44 ou 57-83 et
Y représentant -OZ¹ ou 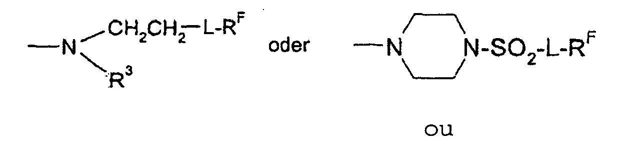 où
L est une liaison directe, un groupe méthylène, un groupe -NHCO-, un groupe 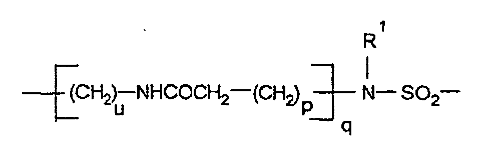 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹CO₂, 1 ou 2 groupes -CO, un groupe 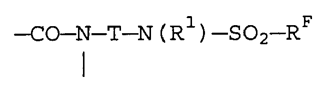 ou 1 ou 2 groupes aryle
éventuellement substitué(s) et/ou est interrompue par ces groupes, et/ou est éventuellement substituée par 1 à 3 groupes -OR¹, 1 ou 2 groupes oxo, 1 ou 2 groupes -NH-COR¹, 1 ou 2 groupes -CONHR¹, 1
ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
R^{F} représentant un radical perfluoroalkyle à chaîne droite ou ramifiée, ayant de 4 à 30 atomes de carbone,
T représentant une chaîne en C₂-C₁₀ qui est éventuellement interrompue par 1 ou 2 atomes d'oxygène ou 1 ou 2 groupes -NHCO, et
R³ a la signification de R¹ ou représente un groupe -(CH₂)ₘ-L-R^{F} où
m est 0, 1 ou 2.

12. Composition selon la revendication 4, **caractérisée en ce que** le complexe métallique M est un complexe de formule générale VIII 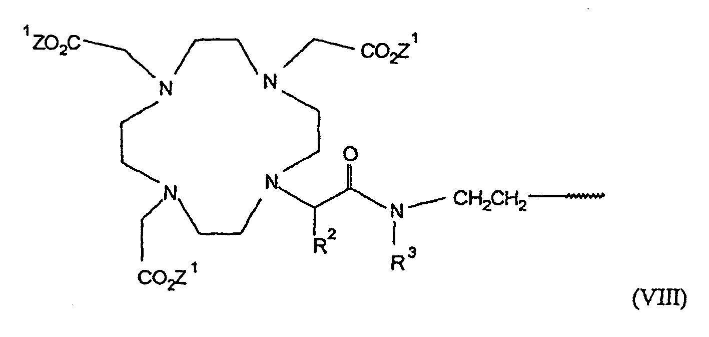 dans laquelle R³, Z¹ sont indépendants les uns des autres et
R³ a la signification de R¹ ou représente un groupe -(CH₂)ₘ-L-R^{F}, où
m est 0, 1 ou 2, et
L est une liaison directe, un groupe méthylène, un groupe -NHCO-, un groupe 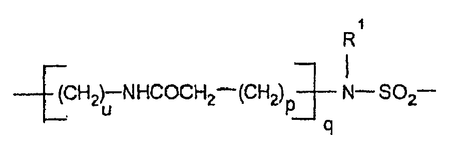 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹CO₂, 1 ou 2 groupes -CO, un groupe 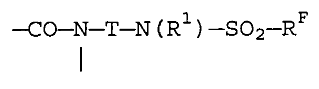 ou 1 ou 2 groupes aryle
éventuellement substitué(s) et/ou est interrompue par ces groupes, et/ou est éventuellement substituée par 1 à 3 groupes -OR¹, 1 ou 2 groupes oxo, 1 ou 2 groupes -NH-COR¹, 1 ou 2 groupes -CONHR¹, 1
ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
R^{F} représentant un radical perfluoroalkyle à chaîne droite ou ramifiée, ayant de 4 à 30 atomes de carbone,
T représentant une chaîne en C₂-C₁₀ qui est éventuellement interrompue par 1 ou 2 atomes d'oxygène ou 1 ou 2 groupes -NHCO,
Z¹ représentant chacun indépendamment un atome d'hydrogène ou un équivalent d'ion métallique des nombres atomiques 21-29, 39, 42, 44 ou 57-83,
et R² a la signification de R¹.

13. Composition selon la revendication 4, **caractérisée en ce que** le complexe métallique M est un complexe de formule générale IX 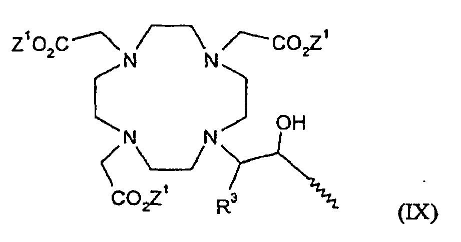 dans laquelle
Z¹ représentent chacun indépendamment un atome d'hydrogène ou un équivalent d'ion métallique des nombres atomiques 21-29, 39, 42, 44 ou 57-83,
R³ a la signification de R¹ ou représente un groupe -(CH₂)ₘ-L-R^{F}, où
m est 0, 1 ou 2, et
L est une liaison directe, un groupe méthylène, un groupe -NHCO-, un groupe 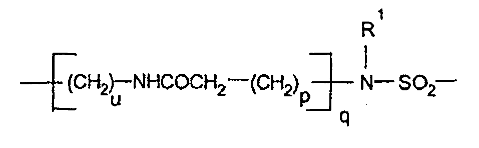 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹CO₂, 1 ou 2 groupes -CO, un groupe 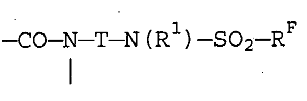 ou 1 ou 2 groupes aryle
éventuellement substitué(s) et/ou est interrompue par ces groupes,
et/ou est éventuellement substituée par 1 à 3 groupes -OR¹, 1 ou 2 groupes oxo, 1 ou 2 groupes -NH-COR¹, 1 ou 2 groupes -CONHR¹, 1 ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
R^{F} représentant un radical perfluoroalkyle à chaîne droite ou ramifiée, ayant de 4 à 30 atomes de carbone, et
T représentant une chaîne en C₂-C₁₀ qui est éventuellement interrompue par 1 ou 2 atomes d'oxygène ou 1 ou 2 groupes -NHCO.

14. Composition selon la revendication 4, **caractérisée en ce que** le complexe métallique M est un complexe de formule générale X 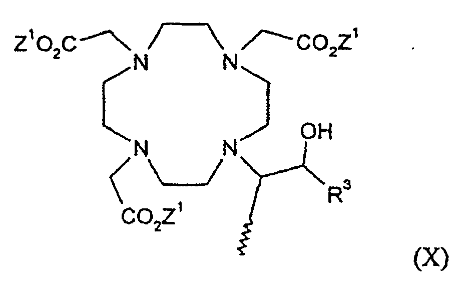 dans laquelle
Z¹ représentent chacun indépendamment un atome d'hydrogène ou un équivalent d'ion métallique des nombres atomiques 21-29, 39, 42, 44 ou 57-83,
R³ a la signification de R¹ ou représente un groupe -(CH₂)ₘ-L-R^{F}, où
m est 0, 1 ou 2, et
L est une liaison directe, un groupe méthylène, un groupe -NHCO-, un groupe 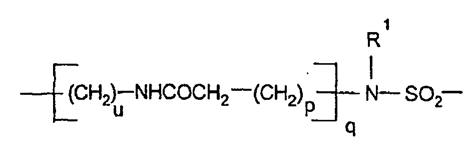 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹CO₂, 1 ou 2 groupes -CO, un groupe 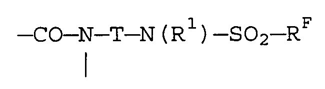 ou 1 ou 2 groupes aryle
éventuellement substitué(s) et/ou est interrompue par ces groupes,
et/ou est éventuellement substituée par 1 à 3 groupes -OR¹, 1 ou 2 groupes oxo, 1 ou 2 groupes -NH-COR¹, 1 ou 2 groupes -CONHR¹, 1
ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R¹ étant un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, et
R^{F} représentant un radical perfluoroalkyle à chaîne droite ou ramifiée, ayant de 4 à 30 atomes de carbone, et
T représentant une chaîne en C₂-C₁₀ qui est éventuellement interrompue par 1 ou 2 atomes d'oxygène ou 1 ou 2 groupes -NHCO,

15. Composition selon la revendication 4, **caractérisée en ce que** le complexe métallique M est un complexe de formule générale XI 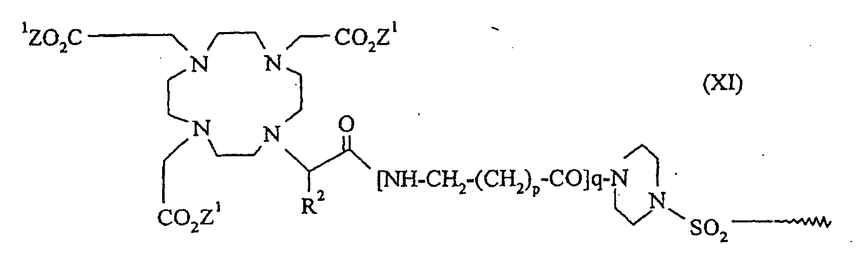 dans laquelle
Z¹ représentent chacun indépendamment un atome d'hydrogène ou un équivalent d'ion métallique des nombres atomiques 21-29, 39, 42, 44 ou 57-83, et
p représente les nombres 0 à 10, q représente le nombre 0 ou 1 et
R² est un atome d'hydrogène, un groupe méthyle, un groupe -CH₂-OH, un groupe -CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy.

16. Composition selon la revendication 4, **caractérisée en ce que** le complexe métallique M est un complexe de formule générale XII 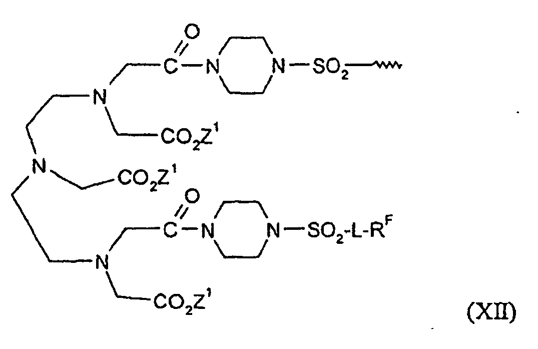 dans laquelle L est une liaison directe, un groupe méthylène, un groupe -NHCO-, un groupe 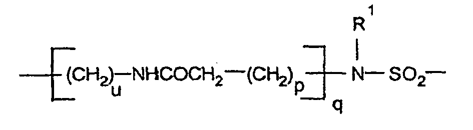 p représentant les nombres 0 à 10, q et u représentant, indépendamment l'un de l'autre, le nombre 0 ou 1 et
R¹ étant un atome d'hydrogène, un groupe méthyle, un groupe -CH₂-OH, un groupe-CH₂-CO₂H ou une chaîne en C₂-C₁₅ qui éventuellement est interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy,
ou est une chaîne carbonée en C₂-C₃₀ droite ou ramifiée, saturée ou insaturée, qui éventuellement contient 1 à 10 atomes d'oxygène, 1 à 3 groupes -NR¹, 1 ou 2 atomes de soufre, un groupe pipérazino, un groupe -CONR¹, un groupe -NR¹CO, un groupe -SO₂, un groupe -NR¹-CO₂, 1 ou 2 groupes-CO, un groupe 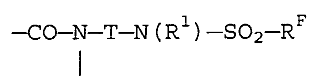 ou 1 ou 2 groupes aryle
éventuellement substitué(s) et/ou est interrompue par ces groupes,
et/ou est éventuellement substituée par 1 à 3 groupes -OR¹, 1 ou 2 groupes oxo, 1 ou 2 groupes -NH-COR¹, 1 ou 2 groupes -CONHR¹, 1 ou 2 groupes -(CH₂)ₚ-CO₂H, 1 ou 2 groupes -(CH₂)ₚ-(O)_{q}-CH₂CH₂-R^{F},
p représentant les nombres 0 à 10, q représentant le nombre 0 ou 1 et
R^{F} représentant un radical perfluoroalkyle à chaîne droite ou ramifiée, ayant de 4 à 30 atomes de carbone, et
Z¹ représentant chacun indépendamment un atome d'hydrogène ou un équivalent d'ion métallique des nombres atomiques 21-29, 39, 42, 44 ou 57-83,

17. Composition selon la revendication 4, **caractérisée en ce que** le complexe métallique M est un complexe de formule générale XIII 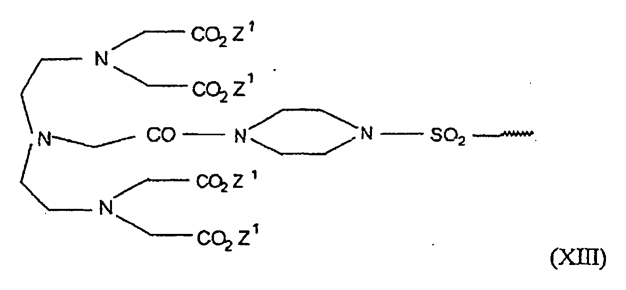 dans laquelle
Z¹ représentent chacun indépendamment un atome d'hydrogène ou un équivalent d'ion métallique des nombres atomiques 21-29, 39, 42, 44 ou 57-83,

18. Composition selon la revendication 1, **caractérisée en ce que** la partie A de la molécule a la structure suivante : 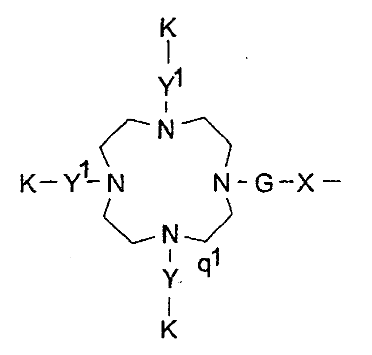 dans laquelle
• q¹ est le nombre 0, 1, 2 ou 3,
• K représente un complexant ou un complexe métallique ou leurs sels avec des bases organiques et/ou des bases minérales ou des acides aminés ou des amides d'acides aminés,
• X est une liaison directe avec le groupe perfluoroalkyle, un groupe phénylène ou une chaîne alkylène en C₁-C₁₀ qui contient éventuellement 1-15 atomes d'oxygène, 1-5 atomes de soufre,
1-10 groupes carbonyle, 1-10 groupes (NR), 1-2 groupes NRSO₂, 1-10 groupes CONR, 1 groupe pipéridino, 1-3 groupes SO₂, 1-2 groupes phénylène ou est éventuellement substituée par 1-3 radicaux R^{F}, R représentant un atome d'hydrogène, un groupe phényle, benzyle ou un groupe alkyle en C₁-C₁₅, qui comporte éventuellement 1-2 groupes NHCO-, 1-2 groupes CO-, 1,5 atomes d'oxygène et est éventuellement substitué par 1-5 radicaux hydroxy, 1-5 radicaux méthoxy, 1-3 radicaux carboxy, 1-3 radicaux R^{F},
• Y¹ est une liaison directe ou une chaîne de formule générale II¹ ou III¹ : 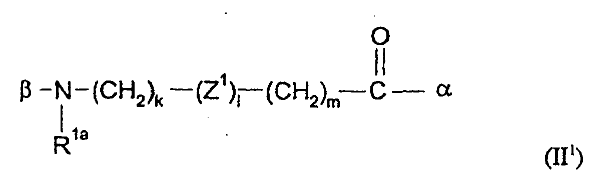 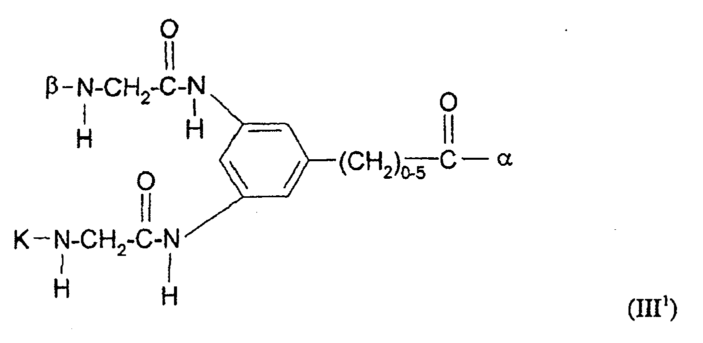 formules dans lesquelles
■ R^{1a} est un atome d'hydrogène, un groupe phényle, un groupe benzyle ou un groupe alkyle en C₁-C₇ qui est éventuellement substitué par un groupe carboxy, un groupe méthoxy ou un groupe hydroxy,
■ Z¹ est une liaison directe, un groupe polyglycoléther comportant jusqu'à 5 unités glycol ou
un fragment de formule générale IV¹
-CH(R^{2a})- (IV¹)
dans laquelle R^{2a} est un acide carboxylique en C₁-C₇, un groupe phényle, un groupe benzyle ou un groupe -(CH₂)₁₋₅NH-K,
■ α représente la liaison à l'atome d'azote de la chaîne du squelette, β représente la liaison au complexant ou au complexe métallique K,
et dans laquelle les variables k et m représentent des nombres naturels compris entre 0 et 10, et 1 représente 0 ou 1 ;
et dans laquelle
• G est un groupe CO ou SO₂.

19. Composition selon la revendication 4, dans laquelle le chaînon de liaison L représente un fragment de formule générale XIV 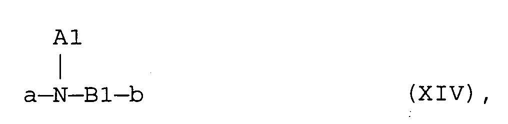 dans laquelle
N représente un atome d'azote,
A1 représente un atome d'hydrogène, un groupe alkyle en C₁-C₃₀ à chaîne droite ou ramifiée, qui est éventuellement interrompu par 1-15 atomes d'oxygène et/ou éventuellement substitué par 1-10 groupes hydroxy, 1-2 groupes COOH, un groupe phényle, un groupe benzyle et/ou 1-5 groupes -OR⁴, où R⁴ a la signification d'un atome d'hydrogène ou d'un radical alkyle en C₁-C₇, ou représente -B1-R^{F}, B1 représente un groupe alkylène en C₁-C₃₀ à chaîne droite ou ramifiée, qui est éventuellement interrompu par 1-10 atomes d'oxygène, 1-5 groupes -NH-CO, 1-5 groupes -CO-NH, par un groupe phénylène (éventuellement substitué par un groupe COOH), 1-3 atomes de soufre,
1-2 groupes -N(B2)-SO₂ et/ou 1-2 groupes -SO₂-N(B2), où B2 a la signification de A1,
un groupe NHCO, un groupe CONH, un groupe N(B2)-SO₂ ou un groupe -SO₂-N(B2) et/ou est éventuellement substitué par le radical R^{F},
et dans laquelle a représente la liaison au complexe métallique M et b représente la liaison au groupe perfluoroalkyle R^{F}.

20. Composition selon la revendication 4, **caractérisée en ce que** le complexe métallique M représente un complexe métallique de formule générale XV 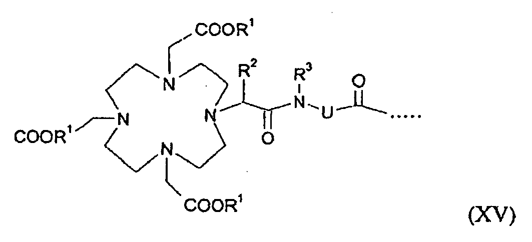 où
R¹ représente un atome d'hydrogène ou un équivalent d'ion métallique des nombres atomiques 21-29, 31, 32, 37-39, 42-44, 49 ou 57-83,
R² et R³ représentent un atome d'hydrogène, un groupe alkyle en C₁-C₇, un groupe benzyle, un groupe phényle, -CH₂OH ou -CH₂OCH₃,
U représente le reste L, L et U, indépendamment l'un de l'autre, pouvant toutefois être identiques ou différents, et
le chaînon de liaison L représente un fragment de formule générale XIV 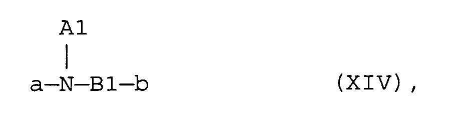 dans laquelle
N représente un atome d'azote,
A1 représente un atome d'hydrogène, un groupe alkyle en C₁-C₃₀ à chaîne droite ou ramifiée, qui est éventuellement interrompu par 1-15 atomes d'oxygène et/ou éventuellement substitué par 1-10 groupes hydroxy, 1-2 groupes COOH, un groupe phényle, un groupe benzyle et/ou 1-5 groupes -OR⁴, où R⁴ a la signification d'un atome d'hydrogène ou d'un radical alkyle en C₁-C₇, ou représente -B1-R^{F}, B1 représente un groupe alkylène en C₁-C₃₀ à chaîne droite ou ramifiée, qui est éventuellement interrompu par 1-10 atomes d'oxygène, 1-5 groupes -NH-CO, 1-5 groupes -CO-NH, par un groupe phénylène (éventuellement substitué par un groupe COOH), 1-3 atomes de soufre,
1-2 groupes -N(B2)-SO₂ et/ou 1-2 groupes -SO₂-N(B2), où B2 a la signification de A1,
un groupe NHCO, un groupe CONH, un groupe N(B2)-SO₂ ou un groupe -SO₂-N(B2) et/ou est éventuellement substitué par le radical R^{F},
et dans laquelle a représente la liaison au complexe métallique M et b représente la liaison au groupe perfluoroalkyle R^{F}.

21. Composition selon l'une quelconque des revendications précédentes, **caractérisée en ce que** l'atome central du complexe métallique est un atome de gadolinium (numéro atomique 64).

22. Composition selon la revendication 1, **caractérisée en ce que** le chaînon de liaison L¹ est une liaison directe, un groupe -SO₂ ou une chaîne carbonée droite ou ramifiée ayant jusqu'à 20 atomes de carbone, qui peut être substituée par un ou plusieurs groupes -OH, -COO⁻, -SO₃ et/ou comporte éventuellement un ou plusieurs groupes -O-, -S-, -CO-, -CONH-, -NHCO-, -CONR-, -NRCO-, -SO₂-, -PO₄⁻-, -NH-, -NR-, un noyau aryle ou un cycle pipérazine, R représentant un radical alkyle en C₁-C₂₀ qui à son tour peut comporter un ou plusieurs atomes d'oxygène et/ou peut être substitué par des groupes -COO⁻ ou SO₃.

23. Composition selon la revendication 1, **caractérisée en ce que** le groupe hydrophile B² est un mono- ou disaccharide, un ou plusieurs groupes -COO⁻ ou -SO₃⁻, un acide dicarboxylique, un acide isophtalique, un acide picolinique, un acide benzènesulfonique, un acide tétrahydropyrannedicarboxylique, un acide 2,6-pyridinedicarboxylique, un ion ammonium quaternaire, un acide aminopolycarboxylique, un acide aminodipolyéthylèneglycolsulfonique, un groupe aminopolyéthylèneglycol, un groupe SO₂-(CH₂)₂-OH, une chaîne polyhydroxyalkyle comportant au moins deux groupes hydroxy ou une ou plusieurs chaînes polyéthylèneglycol comportant au moins deux unités glycol, les chaînes polyéthylèneglycol étant terminées par un groupe -OH ou -OCH₃.

24. Composition selon la revendication 1, **caractérisée en ce que** les chaînes perfluoroalkyle des complexes métalliques perfluoroalkylés et des autres composés perfluoroalkylés contiennent de 6 à 12 atomes de carbone.

25. Composition selon la revendication 24, **caractérisée en ce que** les chaînes perfluoroalkyle contiennent chacune 8 atomes de carbone.

26. Composition selon la revendication 1, **caractérisée en ce qu'**elle présente une teneur en métal de 50 à 250 mmoles/l.

27. Composition selon la revendication 1, **caractérisée en ce que** le groupe hydrophile B² est conjointement avec le chaînon de liaison L¹ un radical choisi dans l'ensemble des radicaux suivants (dans ces formules n est un nombre compris entre 1 et 10) :

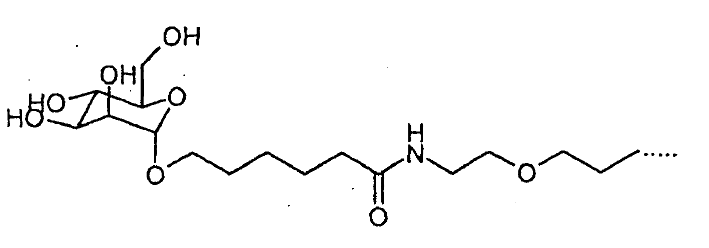

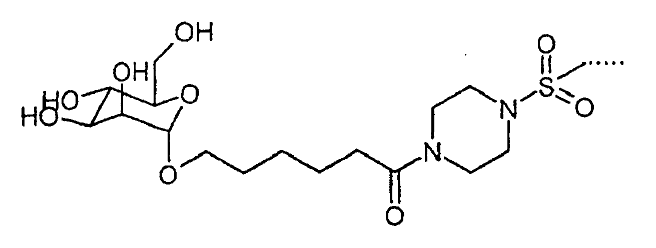

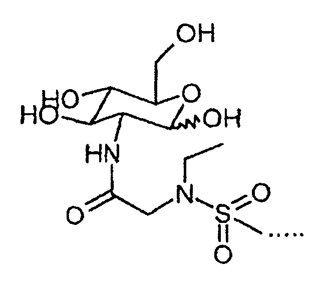

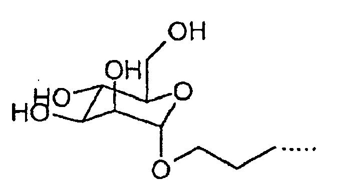

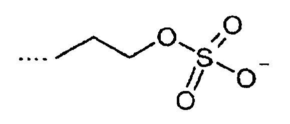

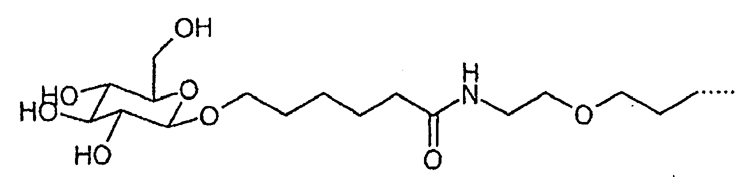

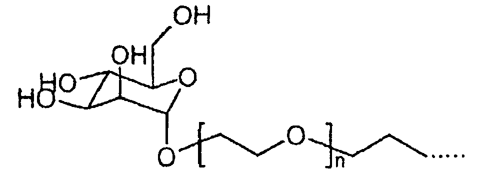

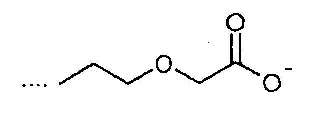

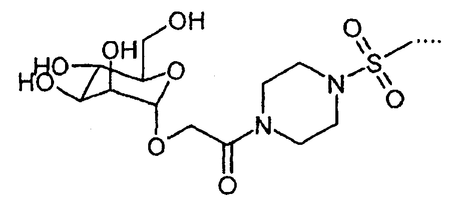

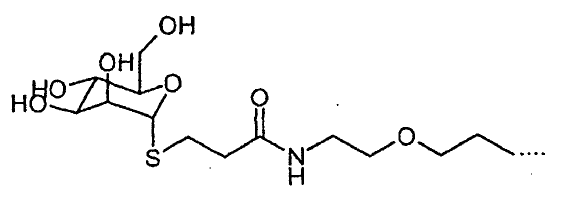

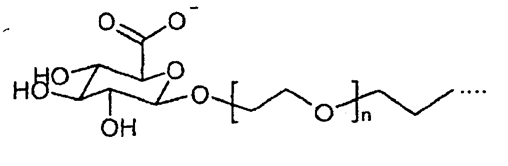

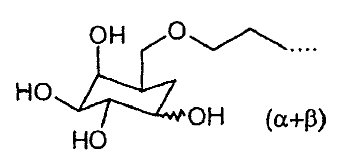

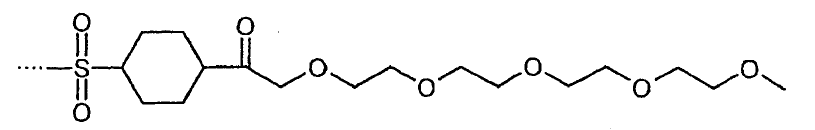

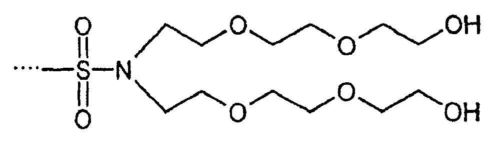

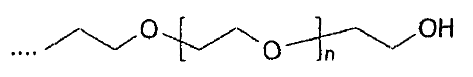

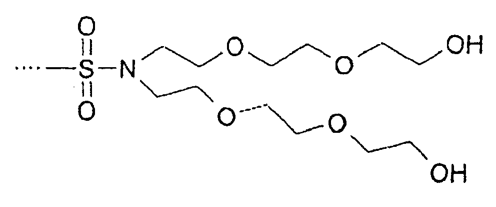

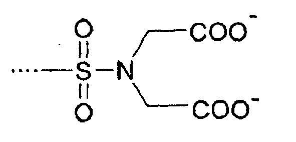

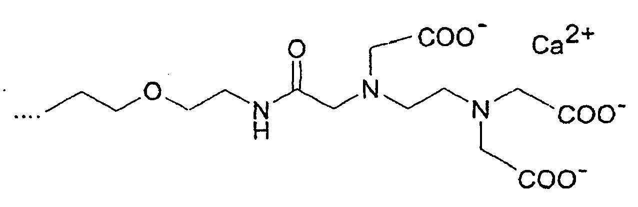

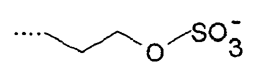

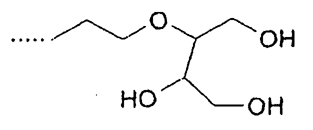

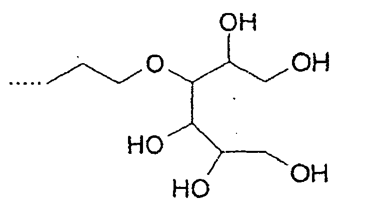

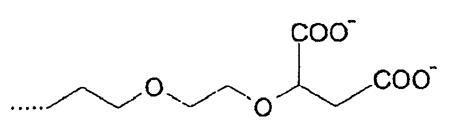

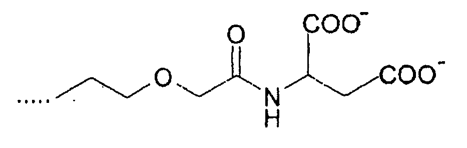

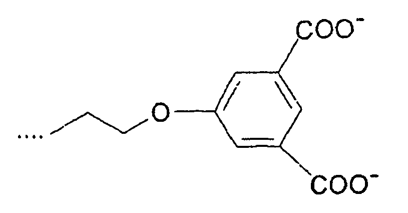

28. Composition selon la revendication 1, **caractérisée en ce que** le groupe hydrophile B² est un conjugué d'α-, β- ou γ-cyclodextrine et d'une molécule d'adamantane, de biphényle ou d'anthracène.

29. Procédé pour la préparation des compositions galéniques selon la revendication 1, **caractérisé en ce que** les composés perfluoroalkylés paramagnétiques et les composés perfluoroalkylés diamagnétiques sont dissous dans un solvant sous vigoureuse agitation.

30. Procédé pour la préparation des compositions galéniques selon la revendication 1, **caractérisé en ce que** les composés perfluoroalkylés paramagnétiques et les composés perfluoroalkylés diamagnétiques sont dissous dans un solvant avec traitement simultané par ultrasons.

31. Procédé pour la préparation des compositions galéniques selon la revendication 1, **caractérisé en ce que** les composés perfluoroalkylés paramagnétiques et les composés perfluoroalkylés diamagnétiques sont dissous dans un solvant avec traitement simultané par micro-ondes.

32. Procédé pour la préparation des compositions galéniques selon la revendication 1, **caractérisé en ce que** les composés perfluoroalkylés paramagnétiques et les composés perfluoroalkylés diamagnétiques sont dissous dans deux solvants différents, on réunit les deux solutions et on élimine par distillation l'un des deux solvants.

33. Composition solide selon la revendication 1, **caractérisée en ce qu'**on la prépare par lyophilisation d'une solution qui contient des substances perfluoroalkylées paramagnétiques et des substances perfluoroalkylées diamagnétiques.

34. Utilisation des compositions galéniques selon la revendication 1, pour la préparation de produits de contraste destinés à la tomographie par RMN.

35. Utilisation des compositions galéniques selon la revendication 1, pour la préparation de produits de contraste destinés à la représentation des ganglions lymphatiques et du volume sanguin.

36. Conjugués consistant en α-, β- ou γ-cyclodextrine et en composés de formule générale XVIII
A¹-L³-R^{F} (XVIII)
dans laquelle A¹ représente une molécule d'adamantane, de biphényle ou d'anthracène, L³ représente un chaînon de liaison et R^{F} représente un radical perfluoroalkyle à chaîne droite ou ramifiée ayant de 4 à 30 atomes de carbone ; et le chaînon de liaison L³ étant une chaîne hydrocarbonée droite ayant de 1 à 20 atomes de carbone, qui peut être interrompue par un ou plusieurs atomes d'oxygène, par un ou plusieurs groupes CO, SO₂, CONH, NHCO, CONR, NRCO, NH, NR ou par un cycle pipérazine, R étant un radical alkyle en C₁-C₅.
